# FASCICULE DE BREVET EUROPEEN

(11) **EP 0 823 903 B1**
(45) Date de publication et mention de la délivrance du brevet: **18.07.2001**
(21) Numéro de dépôt: 97904484.9
(22) Date de dépôt: 04.02.1997
(51) Int. Cl.: C07D 307/80, C07D 307/79, C07D 307/93, A61K 31/34

(54) **COMPOSES BIARYLES HETEROCYCLIQUES, COMPOSITIONS PHARMACEUTIQUES ET COSMETIQUES LES CONTENANT ET UTILISATIONS**
HETEROCYCLISCHE DIARYLVERBINDUNGEN, SIE ENTHALTENDE PHARMAZEUTISCHE UND KOSMETISCHE ZUSAMMENSETZUNGEN, UND IHRE VERWENDUNG.
HETEROCYCLIC DIARYL COMPOUNDS, PHARMACEUTICAL AND COSMETIC COMPOSITIONS CONTAINING SAME, AND USES THEREOF

(30) Priorité: 06.02.1996 FR 9601424
(43) Date de publication de la demande: 18.02.1998
(73) Titulaire: CENTRE INTERNATIONAL DE RECHERCHES DERMATOLOGIQUES GALDERMA, ( CIRD GALDERMA), 06560 Valbonne (FR)
(72) Inventeur: DIAZ, Philippe, F-06200 Nice (FR); CHARPENTIER, Bruno, F-06410 Biot (FR)
(74) Mandataire: Tezier Herman, Béatrice
(86) Numéro de dépôt international: FR9700217
(87) Numéro de publication internationale: WO9729100

(56) Documents cités:
- EP-A- 0 322 004
- EP-A- 0 470 039
- EP-A- 0 526 951
- WO-A-95/09167
- WO-A-96/03396
- BE-A- 848 963
- DE-A- 2 628 189
- US-A- 3 917 654
- US-A- 4 704 462
- CHEMICAL ABSTRACTS, vol. 115, no. 1, 8 Juillet 1991 Columbus, Ohio, US; abstract no. 008229, TOMIOKA H ET AL: "Buttressing effect in carbene chemistry. Effect of 3-alkyl groups on the reactions of 2-alkoxydiphenylcarbenes" XP002017128 & J. CHEM. SOC., PERKIN TRANS. 1;91; (2); PP.471-7, MIE UNIV.;FAC. ENG.; TSU; 514; JAPAN (JP),
- CHEMICAL ABSTRACTS, vol. 113, no. 9, 27 Août 1990 Columbus, Ohio, US; abstract no. 077524, RIETJENS M ET AL: "Rearrangements and fragmentations of metastable [C13H9S]+, [C14H11]+, [C13H11]+ and [C8H7S]+ ions" XP002017129 & ORG. MASS SPECTROM.;90; VOL.25 (5); PP.285-8, UNIV. GRONINGEN;DEP. ORG. CHEM.; GRONINGEN; 9747 AG; NETH. (NL),
- CHEMICAL ABSTRACTS, vol. 110, no. 23, 5 Juin 1989 Columbus, Ohio, US; abstract no. 212530, CABIDDU S ET AL: "Metalation reactions. Part XI. A novel, one-step synthesis of benzo[b]thiophene derivatives" XP002017130 & SYNTHESIS;88; (11); PP.888-90, UNIV. CALGIARI;IST. CHIM. ORG.; CAGLIARI; I-09124; ITALY (IT),
- CHEMICAL ABSTRACTS, vol. 100, no. 6, 6 Février 1984 Columbus, Ohio, US; abstract no. 036002, GIERER J ET AL: "Comparative studies of the participation of different neighboring groups in the alkaline cleavage of.beta.-aryl ether bonds in lignins" XP002017132 & SVEN. PAPPERSTIDN.;83; VOL.86 (9); PP.R100-R106, SWED. FOR. PROD. RES. LAB.;STOCKHOLM; S-114 86; SWED. (SE),
- CHEMICAL ABSTRACTS, vol. 097, no. 3, 19 Juillet 1982 Columbus, Ohio, US; abstract no. 023566, CLARK P D ET AL: "Addition reactions of benzo[b]thiophene. Part 4. Reactions of some acetoxy- and hydroxybenzo[b]thiophenes with benzene or toluene" XP002017133 & J. CHEM. SOC., PERKIN TRANS. 1;82; (3); PP.815-21, UNIV. HULL;DEP. CHEM.; HULL; HU6 7RX; UK (GB),
- CHEMICAL ABSTRACTS, vol. 096, no. 21, 24 Mai 1982 Columbus, Ohio, US; abstract no. 181089, CLARK P D ET AL: "Addition reactions of benzo[b]thiophene. Part 3. Addition and ring-opening reactions with phenolic ethers" XP002017134 & J. CHEM. SOC., PERKIN TRANS. 1;82; (2); PP.615-21, UNIV. HULL;DEP. CHEM.; HULL; HU6 7RX; UK (GB),
- CHEMICAL ABSTRACTS, vol. 096, no. 11, 15 Mars 1982 Columbus, Ohio, US; abstract no. 085357, CLARK P D ET AL: "Addition reactions of benzo[b]thiophene. Part 2. Reactions of substituted benzo[b]thiophenes in benzene or toluene" XP002017135 & J. CHEM. RES., SYNOP.;81; (10); PP.307, UNIV. HULL;DEP. CHEM.; HULL; HU6 7RX; UK (GB),
- CHEMICAL ABSTRACTS, vol. 093, no. 19, 10 Novembre 1980 Columbus, Ohio, US; abstract no. 186068, CLARK P D ET AL: "Addition reactions of benzo[b]thiophene. Part 1. Self-addition and addition of simple aromatic hydrocarbons" XP002017136 & J. CHEM. SOC., PERKIN TRANS. 1;80; (3); PP.677-85, UNIV. HULL;DEP. CHEM.; HULL; HU6 7RX; ENGL.,
- CHEMICAL ABSTRACTS, vol. 092, no. 10, 10 Mars 1980 Columbus, Ohio, US; abstract no. 078366, GIERER J ET AL: "Studies on the condensation of lignins in alkaline media. Part III. The formation of stilbenes, arylcoumarans and diarylmethanes on treatment of spruce wood meal with alkali and white liquor in the presence of xylenols" XP002017137 & ACTA CHEM. SCAND., SER. B;79; VOL.B33 (8); PP.580-2, SWED. FOREST PROD. RES. LAB.;CHEM. DEP.; STOCKHOLM; S-114 86; SWED.,
- CHEMICAL ABSTRACTS, vol. 087, no. 23, 5 Décembre 1977 Columbus, Ohio, US; abstract no. 184135, GIERER J ET AL: "Studies on the condensation of lignins in alkaline media. Part II. The formation of stilbene and arylcoumaran structures through neighbouring group participation reactions" XP002017139 & CAN. J. CHEM.;77; VOL.55 (4); PP.593-9, SWED. FOREST PROD. RES. LAB.;CHEM. DEP.; STOCKHOLM; SWED.,
- CHEMICAL ABSTRACTS, vol. 081, no. 3, 22 Juillet 1974 Columbus, Ohio, US; abstract no. 013330, GOLDENBERG C ET AL: "Benzofuran series. LII. Synthesis of 5- or 6-hydroxy-, 2- or 3-p-hydroxyphenylbenzofurans" XP002017141 & CHIM. THER.;73; VOL.8 (4); PP.398-411, S. A. LABAZ N. V.;CENT. RECH.; BRUSSELS; BELG.,

## Description

L'invention concerne, à titre de produits industriels nouveaux et utiles, des composés biaryles hétérocycliques. Elle concerne également l'utilisation de ces nouveaux composés dans des compositions pharmaceutiques destinées à un usage en médecine humaine ou vétérinaire, ou bien encore dans des compositions cosmétiques.

Les composés selon l'invention ont une activité marquée dans les domaines de la différenciation et de la prolifération cellulaire, et trouvent des applications plus particulièrement dans le traitement topique et systémique des affections dermatologiques liées à un désordre de la kératinisation, des affections dermatologiques (ou autres) à composante inflammatoire et/ou immunoallergique, et des proliférations dermiques ou épidermiques qu'elles soient bénignes ou malignes. Ces composés peuvent en outre être utilisés dans le traitement des maladies de dégénérescence du tissu conjonctif, pour lutter contre le vieillissement de la peau, qu'il soit photoinduit ou chronologique, et traiter les troubles de la cicatrisation. Ils trouvent par ailleurs une application dans le domaine ophtalmologique, notamment dans le traitement des coméopathies.

On peut également utiliser les composés selon l'invention dans des compositions cosmétiques pour l'hygiène corporelle et capillaire.

L'abstract 8229 du Chemical Abstracts vol 115, 1991 décrit des composés de type phényldihydrofuranes dont le radical phényle n'est pas substitué. Aucune activité biologique de ces composés n'est décrite.

L'abstract 212530 du Chemical Abstracts vol 110, 1989 et l'abstract 23566 du Chemical Abstracts vol 97, 1982 décrivent des composés benzo[b]thiophènes qui comprennent donc un hétérocycle soufré insaturé. Aucune activité biologique de ces composés n'est décrite.

L'abstract 13330 du Chemical Abstracts vol 81, 1974 décrit des composés 3p-hydroxyphénylbenzofuranes qui comprennent donc un hétérocycle insaturé. Aucune activité biologique de ces composés n'est décrite.

Le document EP 526 951 décrit des composés 3-phényl-benzo[b]thiophènes qui comprennent donc un hétérocycle soufré insaturé. Ces composés sont décrits comme pesticides et aucune activité pharmaceutique de ces composés n'est décrite.

EP 470 039 décrit des composés 3-arylindoles et 3-arylindazoles. Ces composés, qui renferment un hétérocycle azoté, sont décrits comme des actifs pharmaceutiques dans le traitement de l'anxiété, des psychoses des dépressions et de la schizophrénie.

Le Document BE 848 963 et son correspondant allemand DE 26 28 189 décrivent des composés 3-phénylindolines. Ces composés, qui renferment un hétérocycle azoté, sont décrits comme des actifs pharmaceutiques dans le traitement des anesthésiques locaux.

Les composés biaryle hétérocycliques ci-dessus ne renferment pas de groupement aryle substitué et/ou d'hétérocycle oxygéné.

Au contraire, les composés selon l'invention peuvent être représentés par la formule générale (I) suivante : 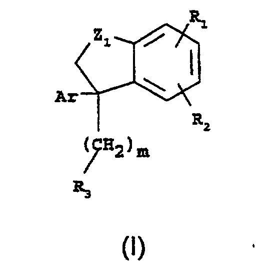 dans laquelle:
- Ar représente
   . soit le radical de formule (II) suivante : 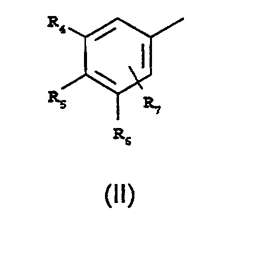
   . soit le radical de formule (III) suivante : 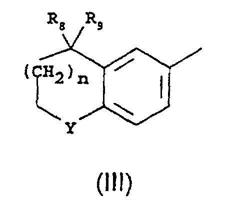
   . soit le radical de formule (IV) suivante : 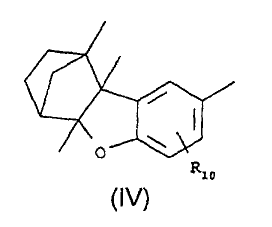
- R₁ représente un atome ou un radical choisi parmi
   (i) le radical -CH₃,
   (ii) le radical -(CH₂)ₚ-O-R₁₁,
   (iii) un radical -OR₁₁,
   (iv) un radical 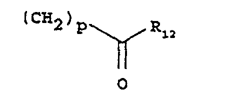
   (v) un radical -S(O)ₜR₁₃,
   R₁₁, R₁₂, R₁₃, p et t ayant les significations données ci-après,
- R₂ représente un atome d'hydrogène, un atome d'halogène, un radical alkyle ou le radical -OR₁₁,
   R₁₁ ayant la signification donnée ci-après,
- R₃ représente un atome ou un radical choisi parmi :
   (i) un atome ou un radical choisi parmi l'atome d'hydrogène, un radical alkyle, un radical alkényle, un radical alkynyle, un radical aryle, un radical monohydroxyalkyle ou polyhydroxyalkyle, un radical polyéther, un radical cyano ou un radical -O-R₁₁,
      R₁₁ ayant la signification donnée ci-après,
   (ii) un radical 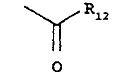 R₁₂ ayant la signification donnée ci-après,
   (iii) un radical 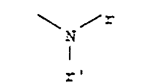 r et r' ayant la signification donnée ci-après
- Z₁ représente O,
- m est un nombre entier compris entre 0 et 10, étant entendu que dans tout ce qui précède :

R₄, R₅, R₆, R₇, identiques ou différents, sont choisis parmi:
   (i) un atome d'hydrogène,
   (ii) un radical alkyle présentant au moins 4 atomes de carbone, parmi lesquels le carbone attaché au radical phényl est au moins substitué par deux atomes de carbone,
   (iii) un radical cycloalkyle,
   (iv) un radical -(Z₂)ₙ-(CH₂)_{q}-CO-R₁₂,
   (v) un radical -Z₃-R₁₁,
      avec au moins un des radicaux R₄, R₅, R₆ et R₇ étant un radical alkyle tel que défini en (ii) ou un radical cycloalkyle (iii),
      Z₂, Z₃, R₁₁,R₁₂, n et q ayant les significations données ci-après,
R₈ et R₉ représentent des radicaux alkyles inférieur,
R₁₀ représente un radical alkyle inférieur, un radical -OR₁₁ ou un radical polyéther,
R₁₁, identique ou différent, représente un atome d'hydrogène, un radical alkyle inférieur, un radical aryle, un radical aralkyle, un radical monohydroxyalkyle ou polyhydroxyalkyle ou un radical polyéther, un radical acyle inférieur,
R₁₂, identique ou différent, représente:
   (a) un atome d' hydrogène, un radical alkynyle, un radical alkényle, un radical alkyle, un hétérocycle,
   (b) un radical 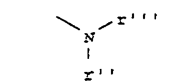 r" et r"' ayant la signification donnée ci-après
   (c) un radical -OR₁₃
R₁₃, identique ou différent, représente un atome d'hydrogène, un radical alkyle, un radical monohydroxyalkyle ou polyhydroxyalkyle, un radical aryle ou aralkyle éventuellement substitué(s) ou un reste de sucre, d'aminoacide ou de peptide
R', identique ou différent, représente un groupement protecteur de fonctions amines, un atome d'hydrogène, un radical alkyle inférieur, un radical polyéther ou un radical aryle éventuellement substitué ou un reste d'aminoacide, de peptide ou de sucre,
r et r', identiques ou différents, représentent des groupements protecteurs de fonctions amines, un atome d' hydrogène, un radical alkyle inférieur, un radical polyéther ou un radical aryle éventuellement substitué ou un reste d'aminoacide, de peptide ou de sucre, ou encore pris ensemble, forment un hétérocycle,
r" et r"', identiques ou différents, un atome d' hydrogène, un radical alkyle inférieur, un radical polyéther ou un radical aryle éventuellement substitué ou un reste d'aminoacide, de peptide ou de sucre, ou encore pris ensemble, forment un hétérocycle,
Y représente C(R₉)₂, O, S, Nr', CHOH, CO, SO, SO₂,
Z₂ représente O, S, NR',
Z₃ représente O ou S,
n, identique ou différent, est égal à 0 ou 1; p, identique ou différent, est égal à 0,1,2 ou 3; t est égal à 0,1, 2 ou 3; q est un entier compris entre 0 et 10,
et les isomères optiques et géométriques desdits composés de formule (I) ainsi que leurs sels .

Lorsque les composés selon l'invention se présentent sous forme de sels, par addition d'un acide, il s'agit de sels pharmaceutiquement ou cosmétiquement acceptables obtenus par addition d'un acide minéral ou organique, en particulier l'acide chlorhydrique, sulfurique, acétique, citrique, fumarique, hémisuccinique, maléique et mandélique. Lorsque les composés selon l'invention se présentent sous forme de sels par addition d'une base, il s'agit de sels d'un métal alcalin ou alcalino-terreux ou encore de zinc ou d'une amine organique.

Selon la présente invention, on entend par radical alkyle un radical linéaire ou ramifié éventuellement substitué par un ou plusieurs atomes d' halogène ayant de 1 à 20, de préférence de 1 à 12, atomes de carbone, avantageusement les radicaux méthyle, éthyle, isopropyle, butyle, tertiobutyle, hexyle, nonyle et dodécyle. Losqu'il est inférieur, le radical alkyl comprend généralement de 1 à 12 atomes de carbone, de préférence de 1 à 4 atomes de carbone.

Parmi les radicaux alkyle linéaire ayant de 1 à 20 atomes de carbone, on peut citer notamment les radicaux méthyle, éthyle, propyle, 2-éthyl-hexyle, octyle, dodécyle, hexadécyle et octadécyl.

Parmi les radicaux alkyle ramifié ayant de 1 à 20 atomes de carbone, on peut citer notamment les radicaux 2-méthylbutyle, 2-méthylpentyle, 1-méthylhexyle, 3-méthylheptyle.

Parmi les radicaux alkyle présentant au moins 4 atomes de carbone, où le carbone attaché au radical phényl est au moins substitué par deux atomes de carbone, on peut citer le radical tertiobutyle.

Par radical alkényle, on entend un radical ayant de 1 à 20 atomes de carbone linéaire ou ramifié comportant une pu plusieurs doubles liaisons.

Parmi les radicaux alkényle, on préfère un radical contenant de 1 à 5 atomes de carbone et présentant une ou plusieurs insaturations éthyléniques, tel que plus particulièrement le radical allyle.

Par radical alkynyle, on entend un radical ayant de 1 à 20 atomes de carbone linéaire ou ramifié comportant une à plusieurs triple liaisons.

Parmi les radicaux alkynyle, on préfère un radical ayant de 2 à 6 atomes de carbone, notamment un radical propargyle.

Par radical acyle inférieur, on entend un radical ayant de 1 à 6 atomes de carbone et de préférence, les radicaux acétyle, propionyle ou pivaloyle,

Par groupement protecteur de fonction amine, on entend les groupements correspondants décrits dans "Protecting groups in organic synthesis" by T.W Greene, Ed. by John Wiley and Sons (1981).

Par radical cycloalkyle, on entend un radical alcane cyclique ou polycyclique contenant de 1 à 10 atomes de carbone, éventuellement substitué par un ou des atomes d' halogène ou un ou des radicaux hydroxyle et de préférence les radicaux adamantyle ou 1-méthylcyclohéxyle.

Par radical polyéther, on entend un radical ayant de 1 à 6 atomes de carbone et de 1 à 3 atomes d' oxygène ou de soufre tel que les radicaux méthoxyméthyl éther, méthoxyéthoxyméthyl éther ou méthylthyométhyl éther.

Par radical monohydroxyalkyle ou polyhydroxyalkyle, on doit entendre un radical contenant de 1 à 6 atomes de carbone et de 1 à 5 groupes hydroxyles.

Parmi les radicaux polyhydroxyalkyle, on préfère un radical présentant de 3 à 6 atomes de carbone et de 2 à 5 groupes hydroxyles, tels que les radicaux 2,3-dihydroxypropyle, 2,3,4-trihydroxybutyle, 2,3,4,5-tétrahydroxypentyle ou le reste du pentaérythritol.

Parmi les radicaux aryle éventuellement substitués, on préfère un radical phényle éventuellement substitué par au moins un atome d'halogène, un radical hydroxyle, un radical alkyle, une fonction nitro, un groupe méthoxy ou une fonction amine éventuellement substituée.

Parmi les radicaux aralkyle, éventuellement substitués, on préfère le radical benzyle ou phénéthyle éventuellement substitué par au moins un atome d'halogène, un radical hydroxyle, une fonction nitro ou un groupe méthoxy.

Par reste de sucre, on entend un reste dérivant notamment de glucose, de galactose ou de mannose, ou bien encore de l'acide glucuronique.

Par reste d'aminoacide, on entend notamment un reste dérivant de l'un des acides aminés tels que la lysine, la glycine ou l'acide aspartique, et par reste de peptide on entend plus particulièrement un reste de dipeptide ou de tripeptide résultant de la combinaison d'acides aminés.

Par hétérocycle, on entend de préférence un radical pipéridino, morpholino, pyrrolidino ou pipérazino, éventuellement substitué en position 4 par un radical alkyle en C₁-C₆ ou polyhydroxyalkyle tels que définis ci-dessus.

Par radical aminoalkyle, on entend un radical contenant de préférence de 1 à 6 atomes de carbone, notamment les radicaux aminométhyle, 3-aminopropyle, 6-aminohexyle.

Parmi les radicaux cycloaliphatiques de C3 à C6 atomes de carbone, on peut citer plus particulièrement le radical cyclopropyle et cyclohexyle.

Parmi les atomes d'halogène, on préfère le fluor ou le chlore.

Lorsque les radicaux R₂ et R₉ représentent un atome d'halogène, celui-ci est de préférence un atome de fluor, de brome ou de chlore.

Parmi les composés de formule (I) ci-dessus rentrant dans le cadre de la présente invention, on peut notamment citer les suivants:
- Acide 3-[3-(1-adamantyl)-4-méthoxyphényl]-3-méthyl-2H-1-benzofuran - 6-carboxylique,
- 3-[3-(1-adamantyl)-4-méthoxyphényl]-3-méthyl-2H-1-benzofuran-6-carboxylate de méthyle,
- Acide 3-[3-(1- adamantyl)-4-méthoxyphényl)]-3-méthyl-2H-1-benzofuran-5-carboxylique,
- 3-[3-(1- adamantyl)-4-méthoxyphényl)]-3-méthyl-2H-1-benzofuran-5-carboxylate de méthyle,
- Acide 3-méthyl-3-(5,6,7,8-tétrahydro-5,5,8,8-tétraméthyl-2-naphtyl)-2H-1-benzofuran-6-carboxylique,
- 3-méthyl-3-(5,6,7,8-tétrahydro-5,5,8,8-tétraméthyl-2-naphtyl)-2H-1-benzofuran-6-carboxylate de méthyle,
- Acide 3-(propen-2-yl)-3-(5,6,7,8-tétrahydro-5,5,8,8-tétraméthyl-2-naphtyl)-2H-1-benzofuran-6-carboxylique,
- 3-(propen-2-yl)-3-(5,6,7,8-tétrahydro-5,5,8,8-tétraméthyl-2-naphtyl)-2H-1-benzofuran-6-carboxylate de méthyle,
- Acide 3-(propen-2-yl)-3-(5,6,7,8-tétrahydro-5,5,8,8-tétraméthyl-2-naphtyl)-2H-1-benzofuran-5-carboxylique,
- 3-(propen-2-yl)-3-(5,6,7,8-tétrahydro-5,5,8,8-tétraméthyl-2-naphtyl)-2H-1-benzofuran-5-carboxylate de méthyle,
- Acide 3-méthyl-3-(5,6,7,8-tétrahydro-5,5,8,8-tétraméthyl-2-naphtyl)-2H-1-benzofuran-5-carboxylique,
- 3-méthyl-3-(5,6,7,8-tétrahydro-5,5,8,8-tétraméthyl-2-naphtyl)-2H-1-benzofuran-5-carboxylate de méthyle,
- Acide 3-méthyl-3-(1,2,3,4-tétrahydro-1,4a,9b-triméthyl-1,4-méthanodibenzofuran-8-yl)-2H-1-benzofuran-6-carboxylique,
- 3-méthyl-3-(1,2,3,4-tétrahydro-1,4a,9b-triméthyl-1,4-méthanodibenzofuran-8-yl)-2H-1-benzofuran-6-carboxylate de méthyle,
- Acide 3-méthyl-3-(1,2,3,4-tétrahydro-1,4a,9b-triméthyl-1,4-méthanodibenzofuran-8-yl)-2H-1-benzofuran-5-carboxylique,
- 3-méthyl-3-(1,2,3,4-tétrahydro-1,4a,9b-triméthyl-1,4-méthanodibenzofuran-8-yl)-2H-1-benzofuran-5-carboxylate de méthyle,
- Acide 3-allyl-3-(5,6,7,8-tétrahydro-5,5,8,8-tétraméthyl-2-naphtyl)-2H-1-benzofuran-6-carboxylique,
- 3-allyl-3-(5,6,7,8-tétrahydro-5,5,8,8-tétraméthyl-2-naphtyl)-2H-1-benzofuran-6-carboxylate de méthyle,
- [3-(5,6,7,8-tétrahydro-5,5,8,8-tétraméthyl-2-naphtyl)-3-méthyl-*2H-*1-benzofuran-5-oyl]-morpholine,
- N-4-hydroxyphényl-3-(5,6,7,8-tétrahydro-5,5,8,8-tétraméthyl-2-naphtyl)-3-méthyl-*2H*-1-benzofuran-5-carboxamide,
- N-butyl-3-méthyl-3-(5,5,8,8-tetramethyl-5,8,7,8-tetrahydro-naphthalen-2-yl)-2H-1-benzofuran-5-carboxamide,
- 3-[4-(1-adamantyl)-3-méthoxyphényl-3-méthyl-2H-1-benzofuran]-6-carboxylate de méthyle.
- acide 3-[4-(1-adamantyl)-3-méthoxyphényl-3-méthyl-2H-1-benzofuran]-6-carboxylique,
- 3-(3-méthyl-5,6,7,8-tétrahydro-5,5,8,8-tétraméthyl-2-naphtyl)-3-méthyl-2H-1-benzofuran-6-carboxylate de méthyle,
- acide 3-(3-méthyl-5,6,7,8-tétrahydro-5,5,8,8-tétraméthyl-2-naphtyl)-3-méthyl-*2H*-1-benzofuran-6-carboxylique,
- 3-(5,6,7,8-tétrahydro-5,5,8,8-tétraméthyl-2-naphtyl)-3-(3,5-di-*tert*-butyl-4-hydroxybenzyl)-*2H*-1-benzofuran-6-carboxylate de méthyle,
- acide 3-(5,6,7,8-tétrahydro-5,5,8,8-tétraméthyl-2-naphtyl)-3-(3,5-di*-tert*-butyl-4-hydroxybenzyl)-2H-1-benzofuran-6-carboxylique,
- 3-(5,6,7,8-tétrahydro-5,5,8,8-tétraméthyl-2-naphtyl)-3-méthyl-2H-1-benzofuran-5-méthanol,
- 3-(5,6,7,8-tétrahydro-5,5,8,8-tétraméthyl-2-naphtyl)-3-méthyl-2H-1-benzofuran-5-carbaldehyde,
- (-)-3-(5,6,7,8-tetrahydro-5,5,8,8-tetramethyl-2-naphtyl)-3-méthyl-*2H*-1-benzofuran-5-carboxylate de méthyle,
- (+)-3-(5,6,7,8-tetrahydro-5,5,8,8-tetramethyl-2-naphtyl)-3-méthyl-*2H*-1-benzofuran-5-carboxylate de méthyle,
- (-)-3-(5,6,7,8-tetrahydro-5,5,8,8-tetramethyl-2-naphtyl)-3-méthyl-2H-1-benzofuran-6-carboxylate de méthyle,
- (+)-3-(5,6,7,8-tetrahydro-5,5,8,8-tetramethyl-2-naphtyl)-3-méthyl-2H-1-benzofuran-6-carboxylate de méthyle,
- 3-(5,6,7,8-tétrahydro-5,5,8,8-tétraméthyl-2-naphtyl)-3-(2-hexényl)-2H-1-benzofuran-6-carboxylate de méthyle,
- acide 3-(5,6,7,8-tétrahydro-5,5,8,8-tétraméthyl-2-naphtyl)-3-(2-hexényl)-2H-1-benzofuran-6-carboxylique,
- acide 3-(5,6,7,8-tétrahydro-5,5,8,8-tétraméthyl-2-naphtyl)-3-hexyl-*2H*-1-benzofuran-6-carboxylique,
- 3-Méthoxycarbonylmethyl-3-(5,5,8,8-tetramethyl-5,6,7,8-tétrahydronaphtalen-2-yl)-2H-1-benzofuran-6-carboxylate de méthyle,
- acide 3-carboxyméthyl-3-(5,5,8,8-tetraméthyl-5,6,7,8-tétrahydronaphtalen-2-yl)-2H-1-benzofuran-6-carboxylique.

Selon la présente invention, les composés de formule (I) plus particulièrement préférés sont ceux pour lesquels l'une au moins, et de préférence toutes, les conditions ci-dessous sont respectées:
- R₁ est un radical -(CH₂)ₚ-CO-O-R₁₃
- R₂ est un hydrogène
- R₃ est un hydrogène ou un radical alkényle
- R₅ ou R₆ est un radical -OR₁₁
- R₇ est un radical cycloalkyle
- Z₁ est un atome d'oxygène
- Y est un radical C(R₉)₂
- m est égal à 1.

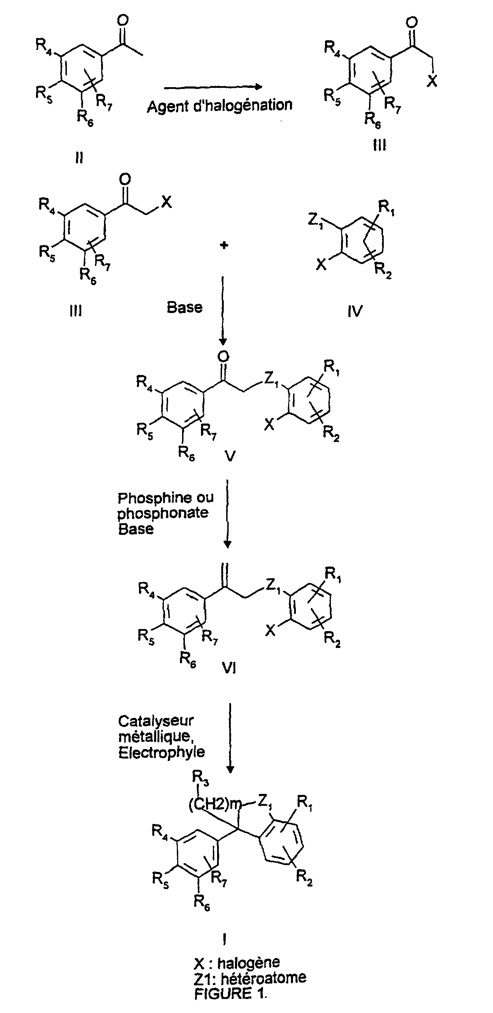

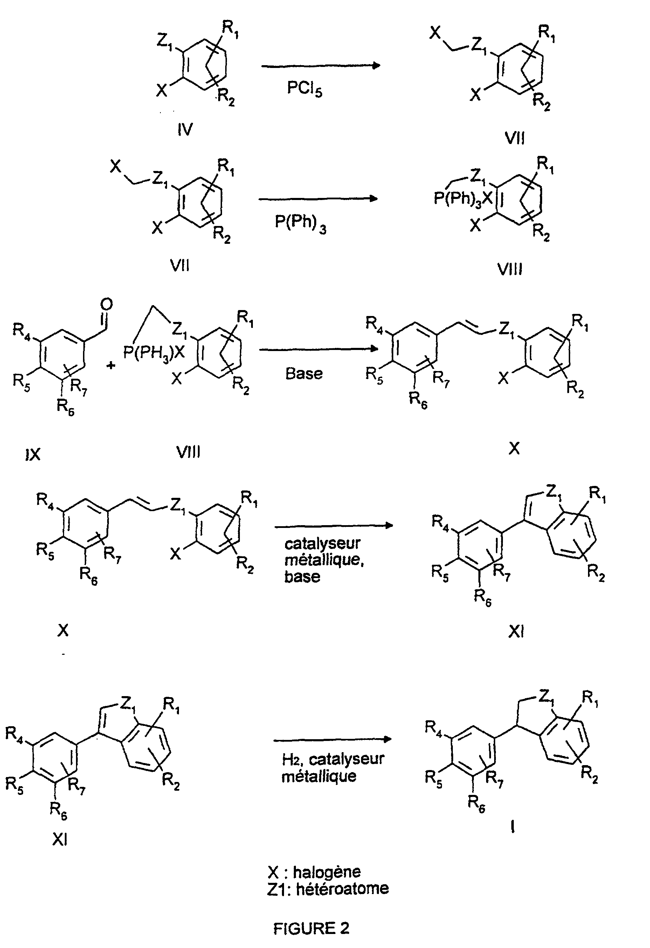

La présente invention a également pour objet les procédés de préparation des composés de formule (I), en particulier selon les schémas réactionnels donnés aux figures 1 et 2.

Ainsi les composés de formule générale (I) peuvent être obtenus (figure 1) à partir de la cétone II, par halogénation, par exemple au moyen d'un agent de bromation tel que le brome. Le composé III obtenu est ensuite couplé avec le composé IV, en présence de base telle que le carbonate de potassium ou l' hydrure de sodium. Le dérivé couplé V est soumis à l'action d'une phosphine ou d'un phosphonate en présence d'une base pour conduire au composé VI. Le composé VI est cyclisé par action d'un catalyseur métallique comme le diacétate de palladium, en présence d'un donneur d'hydrure comme l'acide formique ou d'un nuchéophile comme le vinyl tributyl étain ou l'acétate de lithium et si nécessaire d'une base.
L'adjonction de sels ou de zéolites d'argent comme Ag₃PO₄ et de phosphines chirales comme le Binap permet d'induire une cyclisation asymétrique.

Les composés de formule générale (I) peuvent aussi être obtenus selon le schéma de synthèse de la figure 2. Le composé VII est obtenu par halogénation du produit IV par le pentachlorure de phosphore par exemple. Il est converti en sel de phosphonium VIII par action de triphényl phosphine (selon le brevet EP 428423A2). Celui-ci est couplé à un aldéhyde ou une cétone IX, par action d'une base comme le méthanolate de sodium ou l'hydrure de sodium pour donner l'éther d'énol X. Il est cyclisé en présence d'un catalyseur métallique comme le diacétate de palladium, en présence d'une base comme la tributylamine pour donner le produit XI. Le composé de formule (I) est obtenu après hydrogenétion du produit XI,

Les produits de formule générale (I) ainsi obtenus peuvent servir de produits de départ pour la fabrication d'autres produits de formule générale (I). Ces dérivés seront obtenus selon les méthodes classiques de synthèse employées en chimie, telles que celles décrites dans "Advanced Organic Chemistry" de J. March; John Willey and Sons, 1985.

Par exemple, on peut procéder aux modifications fonctionnelles du groupe R₁ comme indiqué ci-dessous:

| | |
|---|---|
| acide carboxylique | -> ester |
| ester | -> acide carboxylique |
| acide | -> chlorure d' acide |
| chlorure d'acide | -> amide |
| acide | -> amide |
| acide | -> alcool |
| alcool | -> aldéhyde |
| amide | -> amine |
| thiol | -> thioéther |
| thioéther | -> sulfoxyde |
| thioéther | -> sulfone |
| acide sulfonique | -> ester sulfonique |
| acide sulfonique | -> sulfonamide |
| acide sulfinique | -> ester sulfinique |

Les composés selon l'invention présentent une activité dans le test de différenciation des cellules (F9) de tératocarcinome embryonnaire de souris (Cancer Research 43, p. 5268, 1983) et/ou dans le test d'inhibition de l'omithine décarboxylase après induction par le TPA chez la souris (Cancer Research 38, p. 793-801, 1978). Ces tests montrent les activités de ces composés respectivement dans les domaines de la différenciation et de la prolifération cellulaire. Dans le test de différenciation des cellules (F9), il est possible d'évaluer une activité agoniste, comme une activité antagoniste aux récepteurs de l'acide rétinoïque. En effet, un antagoniste est inactif lorsqu'il est seul dans ce test, mais inhibe partiellement ou totalement l'effet produit par un rétinoïde agoniste sur la morphologie et sur la sécrétion de l'activateur plasminogène. Certains de ces composés présentent donc également une activité dans un test qui consiste à identifier des molécules antagonistes des RARs, tel que décrit dans la demande de brevet français n° 95-07302 déposée le 19 juin 1995 par la Demanderesse. Ce test comprend les étapes suivantes : (i) on applique topiquement sur une partie de la peau d'un mammifère une quantité suffisante d'une molécule agoniste des RARs, (ii) on administre par voie systémique ou topique sur ce même mammifère ou sur cette même partie de la peau du mammifère, avant, pendant ou après l'étape (i), une molécule susceptible de présenter une activité antagoniste des RARs et (iii) on évalue la réponse sur la partie de la peau ainsi traitée du mammifère. Ainsi, la réponse à une application topique sur l'oreille d'un mammifère d'une molécule agoniste des RARs qui correspond à une augmentation de l'épaisseur de cette oreille peut être inhibée par l'administration par voie systémique ou topique d'une molécule antagoniste des RARs. En outre, certains de ces composés peuvent apporter une synergie à l'activité biologique de produits se liant aux récepteurs nucléaires.

La présente invention a également pour objet à titre de médicament les composés de formule (I) telle que définie ci-dessus.

Les composés selon l'invention conviennent particulièrement bien dans les domaines de traitement suivants :
1) pour traiter les affections dermatologiques liées à un désordre de la kératinisation portant sur la différenciation et sur la prolifération notamment pour traiter les acnées vulgaires, comédoniennes, polymorphes, rosacées, les acnées nodulokystiques, conglobata, les acnées séniles, les acnées secondaires telles que l'acnée solaire, médicamenteuse ou professionnelle,
2) pour traiter d'autres types de troubles de la kératinisation, notamment les ichtyoses, les états ichtyosiformes, la maladie de Darrier, les kératodermies palmoplantaires, les leucoplasies et les états leucoplasiformes, le lichen cutané ou muqueux (buccal),
3) pour traiter d'autres affections dermatologiques liées à un trouble de la kératinisation avec une composante inflammatoire et/ou immuno-allergique et notamment toutes les formes de psoriasis qu'il soit cutané, muqueux ou unguéal, et même le rhumatisme psoriatique, ou encore l'atopie cutanée, telle que l'eczéma ou l'atopie respiratoire ou encore l'hypertrophie gingivale ; les composés peuvent également être utilisés dans certaines affections inflammatoires ne présentant pas de trouble de la kératinisation,
4) pour traiter toutes les proliférations dermiques ou épidermiques qu'elles soient bénignes ou malignes, qu'elles soient ou non d'origine virale telles que verrues vulgaires, les verrues planes et l'épidermodysplasie verruciforme, les papillomatoses orales ou florides et les proliférations pouvant être induites par les ultra-violets notamment dans le cas des épithélioma baso et spinocellulaires,
5) pour traiter d'autres désordres dermatologiques tels que les dermatoses bulleuses et les maladies du collagène,
6) pour traiter certains troubles ophtalmologiques, notamment les cornéopathies,
7) pour réparer ou lutter contre le vieillissement de la peau, qu'il soit photo-induit ou chronologique ou pour réduire les pigmentations et les kératoses actiniques, ou toutes pathologies associées au vieillissement chronologique ou actinique,
8) pour prévenir ou guérir les stigmates de l'atrophie épidermique et/ou dermique induite par les corticostéroïdes locaux ou systémiques, ou toute autre forme d'atrophie cutanée,
9) pour prévenir ou traiter les troubles de la cicatrisation ou pour prévenir ou pour réparer les vergetures,
10) pour lutter contre les troubles de la fonction sébacée tels que l'hyperséborrhée de l'acné ou la séborrhée simple,
11) dans le traitement ou la prévention des états cancéreux ou précancéreux,
12) dans le traitement d'affections inflammatoires telles que l'arthrite,
13) dans le traitement de toute affection d'origine virale au niveau cutané, telle que le syndrome de Kaposis, ou général,
14) dans la prévention ou le traitement de l'alopécie,
15) dans le traitement d'affections dermatologiques ou générales à composante immunologique,
16) dans le traitement d'affections du système cardiovasculaire telles que l'artériosclérose.
17) dans le traitement de désordres cutanés dus à une exposition aux rayonnements U.V..

Dans les domaines thérapeutiques mentionnés ci-dessus, les composés selon l'invention peuvent être avantageusement employés en combinaison avec d'autres composés à activité de type rétinoïde, avec les vitamines D ou leurs dérivés, avec des corticostéroïdes, avec des anti-radicaux libres, des α-hydroxy ou α-céto acides ou leurs dérivés, ou bien encore avec des bloqueurs de canaux ioniques. Par vitamines D ou leurs dérivés, on entend par exemple les dérivés de la vitamine D₂ ou D₃ et en particulier la 1,25-dihydroxyvitamine D₃. Par anti-radicaux libres, on entend par exemple l'α-tocophérol, la Super Oxyde Dismutase, l'Ubiquinol ou certains chélatants de métaux. Par α-hydroxy ou α-céto acides ou leurs dérivés, on entend par exemple les acides lactique, malique, citrique, glycolique, mandélique, tartrique, glycérique ou ascorbique ou leurs sels, amides ou esters. Enfin, par bloqueurs de canaux ioniques, on entend par exemple le Minoxidil (2,4-diamino-6-pipéridino-pyrimidine-3-oxyde) et ses dérivés.

La présente invention a également pour objet des compositions médicamenteuses contenant au moins un composé de formule (I) telle que définie ci-dessus, l'un de ses isomères optiques ou géométriques ou un de ses sels.

La présente invention a donc ainsi pour objet une nouvelle composition médicamenteuse destinée notamment au traitement des affections susmentionnées, et qui est caractérisée par le fait qu'elle comprend, dans un support pharmaceutiquement acceptable et compatible avec le mode d'administration retenu pour cette dernière, au moins un composé de formule (I), l'un de ses isomères optiques ou géométriques ou un de ses sels.

L'administration des composés selon l'invention peut être effectuée par voie entérale, parentérale, topique ou oculaire.

Par voie entérale, les médicaments peuvent se présenter sous forme de comprimés, de gélules, de dragées, de sirops, de suspensions, de solutions, de poudres, de granulés, d'émulsions, de microsphères ou de nanosphères ou de vésicules lipidiques ou polymériques permettant une libération contrôlée. Par voie parentérale, les compositions peuvent se présenter sous forme de solutions ou de suspensions pour perfusion ou pour injection.

Les composés selon l'invention sont généralement administrés à une dose journalière d'environ 0,01 mg/kg à 100 mg/Kg en poids corporel, et ceci à raison de 1 à 3 prises.

Par voie topique, les compositions pharmaceutiques à base de composés selon l'invention sont plus particulièrement destinées au traitement de la peau et des muqueuses et peuvent alors se présenter sous forme d'onguents, de crêmes, de laits, de pommades, de poudres, de tampons imbibés, de solutions, de gels, de sprays, de lotions ou de suspensions. Elles peuvent également se présenter sous forme de microsphères ou nanosphères ou vésicules lipidiques ou polymériques ou de patches polymériques et d'hydrogels permettant une libération contrôlée. Ces compositions par voie topique peuvent par ailleurs se présenter soit sous forme anhydre, soit sous une forme aqueuse, selon l'indication clinique.

Par voie oculaire, ce sont principalement des collyres.

Ces compositions à usage topique ou oculaire contiennent au moins un composé de formule (I) telle que définie ci-dessus, ou l'un de ses isomères optiques ou géométriques ou encore l'un de ses sels, à une concentration de préférence comprise entre 0,001% et 5% en poids par rapport au poids total de la composition.

Les composés de formule (I) selon l'invention trouvent également une application dans le domaine cosmétique, en particulier dans l'hygiène corporelle et capillaire et notamment pour le traitement des peaux à tendance acnéique, pour la repousse des cheveux, l'anti-chute, pour lutter contre l'aspect gras de la peau ou des cheveux, dans la protection contre les aspects néfastes du soleil ou dans le traitement des peaux physiologiquement sèches, pour prévenir et/ou pour lutter contre le vieillissement photo-induit ou chronologique.

Dans le domaine cosmétique, les composés selon l'invention peuvent par ailleurs être avantageusement employés en combinaison avec d'autres composés à activité de type rétinoïde, avec les vitamines D ou leurs dérivés, avec des corticostéroïdes, avec des anti-radicaux libres, des α-hydroxy ou α-céto acides ou leurs dérivés, ou bien encore avec des bloqueurs de canaux ioniques, tous ces différents produits étant tels que définis ci-avant.

La présente invention vise donc également une composition cosmétique qui est caractérisée par le fait qu'elle comprend, dans un support cosmétiquement acceptable et convenant à une application topique, au moins un composé de formule (I) telle que définie ci-dessus ou l'un de ses isomères optiques ou géométriques ou l'un de ses sels, cette composition cosmétique pouvant notamment se présenter sous la forme d'une crème, d'un lait, d'une lotion, d'un gel, de microsphères ou nanosphères ou vésicules lipidiques ou polymériques, d'un savon ou d'un shampooing.

La concentration en composé de formule (I) dans les compositions cosmétiques selon l'invention est avantageusement comprise entre 0,001% et 3% en poids par rapport à l'ensemble de la composition.

Les compositions médicamenteuses et cosmétiques selon l'invention peuvent en outre contenir des additifs inertes ou même pharmacodynamiquement ou cosmétiquement actifs ou des combinaisons de ces additifs, et notamment : des agents mouillants; des agents dépigmentants tels que l'hydroquinone, l'acide azélaïque, l'acide caféïque ou l'acide kojique; des émollients; des agents hydratants comme le glycérol, le PEG 400, la thiamorpholinone, et ses dérivés ou bien encore l'urée; des agents antiséborrhéiques ou antiacnéiques, tels que la S-carboxyméthylcystéine, la S-benzyl-cystéamine, leurs sels ou leurs dérivés, ou le péroxyde de benzoyle; des antibiotiques comme l'érythromycine et ses esters, la néomycine, la clindamycine et ses esters, les tétracyclines; des agents antifongiques tels que le kétokonazole ou les polyméthylène-4,5 isothiazolidones-3; des agents favorisant la repousse des cheveux, comme le Minoxidil (2,4-diamino-6-pipéridino-pyrimidine-3-oxyde) et ses dérivés, le Diazoxide (7-chloro 3-méthyl 1,2,4-benzothiadiazine 1,1-dioxyde) et le Phénytoïn (5,4-diphényl-imidazolidine 2,4-dione); des agents anti-inflammatoires non stéroïdiens; des caroténoïdes et, notamment, le β-carotène; des agents anti-psoriatiques tels que l'anthraline et ses dérivés; et enfin les acides eicosa-5,8,11,14-tétraynoïque et eicosa-5,8,11-trynoïque, leurs esters et amides.

Les compositions selon l'invention peuvent également contenir des agents d'amélioration de la saveur, des agents conservateurs tels que les esters de l'acide parahydroxybenzoïque, les agents stabilisants, des agents régulateurs d'humidité, des agents régulateurs de pH, des agents modificateurs de pression osmotique, des agents émulsionnants, des filtres UV-A et UV-B, des antioxydants, tels que l'α-tocophérol, le butylhydroxyanisole ou le butylhydroxytoluène.

On va maintenant donner, à titre d'illustration et sans aucun caractère limitatif, plusieurs exemples d'obtention de composés actifs de formule (I) selon l'invention, ainsi que diverses formulations concrètes à base de tels composés. Dans les exemples : TTN signifie TétramethylTetrahydroNaphtyl

### A. EXEMPLES DE COMPOSES

### EXEMPLE 1

### Procédé de préparation du 3-[3-(1-adamantyl)-4-méthoxyphényl]-3-méthyl-2H-1-benzofuran-6-carboxylate de méthyle

### Acide 4-lodo-3-hydroxybenzoïque.

On additionne goutte à goutte une solution de perchlorate de sodium à 3,6% à un mélange d' acide 4-hydroxybenzoïque (2,55 g, 18,5 mmol), de soude (0,74 g, 18,5 mmol), d'iodure de sodium (2,77 g, 18,5 mmol) dans le méthanol (50 ml), à 0°C. On laisse agiter deux heures à 0°C. On additionne 20 ml d'une solution de thiosulfate de sodium à 10%. Après agitation, on acidifie par de l'acide chlorydrique jusqu'à pH 1. On extrait par 100 ml d'éther éthylique. La phase organique est lavée deux fois par 80 ml d' eau, séchée sur sulfate de magnésium et concentrée à l'évaporateur rotatif sous vide à 40°C.
Solide blanc. Masse:2,71 g, Rendement: quantitatif. Pf: 178-185°C
RMN ¹H (DMSO, 250 MHz): 7,13 (1H Ar, dd, J=1,08 Hz, J=7,55Hz), 7,41 (1H Ar, d, J=1,08 Hz), 7,76 (1H Ar, d, J=7,55 Hz), 10,71 (1H, s), 12,96 (1H, s).

### 4-lodo-3-hydroxybenzoate de méthyle.

On chauffe à reflux une solution d'acide 4-iodo 3-hydroxybenzoïque (2,71 g, 10 mmol) et d'acide sulfurique (0,7 ml) dans le méthanol (17 ml), pendant 6 h. On ajoute 20 ml d'eau et on alcalinise avec du bicarbonate de soude jusqu'à neutralité. On extrait par de l'éther éthylique (60 ml). La phase organique est lavée par deux fois 30 ml d'eau, séchée sur sulfate de magnésium, et concentrée à l'évaporateur rotatif sous vide à 40°C. Le produit est purifié par chromatographie flash sur colonne de silice (acétate d' éthyle 50 %, heptane 50 %).
Solide blanc. Masse: 2g. Rendement: 72%. Pf: 164°C
RMN ¹H (CDCl₃, 250 MHz): 3,91 (3H, s), 5,70 (1H,s), 7,33 (1H Ar, d, J=8,16Hz), 7,64 (1H Ar, s), 7,75 (1H Ar, d, J=8,16 Hz).

### 3-[(3-(1-adamantyl)-4-méthoxybenzoyl)méthyloxy]-4-iodobenzoate de méthyle.

Une solution de 3-(1-adamantyl)-4-méthoxybromoacétophénone (2,5g, 6,86 mmol), de 3-hydroxy-4-iodobenzoate de méthyle (1,9 g, 6,83 mmol) et de carbonate de potassium (0,95 g, 6,88 mmol) dans la méthyléthylcétone (50 ml), est chauffée à reflux pendant cinq heures. On filtre le milieu réactionnel, puis on ajoute 40 ml d'eau et 40 ml d'éther éthylique. Après agitation et décantation, la phase organique est lavée deux fois par 40 ml d' eau, séchée sur sulfate de magnésium et concentrée à l'évaporateur rotatif sous vide à 40 °C. Le produit est purifié par chromatographie flash sur colonne de silice (acétate d' éthyle 30 %, heptane 70 %).
Solide blanc. Masse: 2,52 g, Rendement: 66%. Pf: 156°C
RMN ¹H (CDCl₃, 250 MHz): 1,77 (6H, s), 2,10 (9H, s), 3,88 (3H, s), 3,92 (3H, s), 5,37 (2H, s), 6,92 (1H Ar, d, J=9,2 Hz), 7,37 à 7,41 (2H Ar, m), 7,87 à 7,92 (3H Ar, m).

### 4-Iodo-3-[2-[3-(1-adamantyl)-4-méthoxybenzyl]-1-propényloxy]benzoate de méthyle

On additionne en 8 heures, à un mélange de 3-[3-(1-adamantyl)-4-méthoxybenzoylméthyloxy]-4-iodobenzoate de méthyle (2,52 g, 4,49 mmol) et de bromure de méthyltriphénylphoshine (2,45 g, ) dans le THF (60 ml), du méthylate de sodium (0,94 ml).
On agite 12 heures à température ambiante. Le mélange est concentré à l'évaporateur rotatif sous vide à 40°C. On extrait par 40 ml d'éther éthylique et 40 ml d'eau. Après décantation la phase organique est lavée deux fois par 40 ml d'eau, séchée sur sulfate de magnésium anhydre et concentrée à l'évaporateur rotatif sous vide à 40°C. Le produit est purifié par chromatographie flash sur colonne de silice (CH₂Cl₂ 60 %, heptane 40 %).
Solide blanc. Masse: 1,16 g, Rendement: 46%. Pf: 73°C
RMN ¹H (CDCl₃, 250 MHz): 1,77 (6H, s), 2,06 (3H, s), 2,10 (6H, s), 3,85 (3H, s), 3,91 (3H, s), 4,98 (2H, s), 5,54 (2H, s), 6,86 (1H Ar, d, J=8,3 Hz), 7,28 à 7,40 (4H Ar, m), 7,51 (1H Ar, d, J=1,7 Hz), 7,86 (1H Ar, d, J=8,1 Hz).

### 3-[3-(1-adamantyl)-4-méthoxyphényl]-3-méthyl-2H-1-benzofuran-6-carboxylate de méthyle

On chauffe à 95°C pendant 7 jours un mélange de tributylamine (0,42 g, 2,26 mmol), de triphénylphosphine (0,012 g, 0,05mmol), de diacétate de palladium (0,005 g, 0,02 mmol), et de 4-lodo-3-[2-[3-(1-adamantyl)-4-méthoxybenzyl]-1-propényloxy]benzoate de méthyle (1,13 g, 2,02 mmol) dans l'acétonitrile (12 ml). On réapprovisionne les 5 premier jours en catalyseur et triphénylphosphine. Le milieu réactionnel est concentré à l'évaporateur rotatif sous vide à 40°C.
On rajoute 20 ml d' eau et 20 ml d'acétate d' éthyle. Après décantation, la phase organique est lavée deux fois par 20 ml d' eau, séchée sur sulfate de magnésium, et concentrée à l'évaporateur rotatif sous vide à 40°C.
Le produit est purifié par chromatographie flash sur colonne de silice (CH₂Cl₂ 50% heptane 50 %)
Solide blanc. Masse: 236 mg, Rendement: 27%. Pf: 91-92°C
RMN ¹H (CDCl₃, 250 MHz): 1,73 (9H, s), 2,03 (9H, s), 3,80 (3H, s), 3,90 (3H, s), 4,48 (1H, d, J=8,68 Hz), 4,61 (1H, d, J=8,68 Hz), 6,78 (1H Ar, d, J=8,52 Hz), 7,03 à 7,14 (3H Ar, m), 7,52 (1H Ar, d, J=1,14 Hz), 7,63 (1H Ar, dd, J=7,79 Hz, J=1,36 Hz).
RMN ¹³C (CDCl₃, 250 MHz): 29,57, 29,24, 37,25, 37,37, 40,65, 49,86, 52,32, 55,21, 86,83, 111,02, 11,53, 123,12, 124,06, 124,79, 124,95, 130,69, 137,08, 138,69, 141,81, 157,72, 159,92, 167,22.

### EXEMPLE 2

### Procédé de préparation de l'acide 3-[3-(1-adamantyl)-4-méthoxyphényl]-3-méthyl-2H-1-benzofuran-6-carboxylique

On chauffe 6 h à reflux un mélange de 3-[3-(1-adamantyl)-4-méthoxyphényl]-3-méthyl-2H-1-benzofuran-6-carboxylate de méthyle (62 mg, 0,14 mmol), de soude (0,012 g, 0,3 mmol), et d'hydroxyde de lithium (0,012 g, 0,28 mmol). On concentre à l'évaporateur rotatif sous vide à 40°C. On rajoute 10 ml d' eau et 10 ml d' acétate d' éthyle. On acidifie par une solution d'acide chlorhydrique concentrée jusqu' à un pH =1. Après décantation, la phase organique est lavée deux fois par 10 ml d' eau, séchée sur sulfate de magnésium, et concentré à l'évaporateur rotatif sous vide à 40°C. Le produit est purifié par chromatographie flash sur colonne de silice (CH₂Cl₂ 50% AcOEt 50 %)
Solide blanc. Masse: 43 mg, Rendement: 72%. Pf: 86°C
RMN ¹H (CDCl₃, 250 MHz): 1,75 (9H, s), 2,03 (9H, s), 3,81 (3H, s), 4,50 (1H, d, J=8,68 Hz), 4,63 (1H, d, J=8,68 Hz), 6,79 (1H Ar, d, J=8,50), 7,05 (1H Ar, dd, J=2,27 Hz, J=8,48 Hz), 7,09 à 7,15 (2H Ar, m), 7,59 (1H Ar, s), 7,70 (1H Ar, dd, J=1,25 Hz, 7,79 Hz).
RMN ¹³C (CDCl₃, 250 MHz): 25,95, 28,65, 36,66, 36,79, 40,07, 49,32, 54,63, 86,26, 110,95, 123,24, 123,59, 124,22, 124,34, 129, 24, 136,35, 138,13, 142,27, 157,17,

### EXEMPLE 3

### Procédé de préparation du 3-méthyl-3-(5,6,7,8-tétrahydro-5,5,8,8-tétraméthyl-2-naphtyl)-2H-1-benzofuran-6-carboxylate de méthyle

### 3-[(5,6,7,8-tétrahydro-5,5,8,8-tétraméthyl-2-naphtoyl) méthyloxy]-4-iodobenzoate de méthyle.

Une solution de 5,6,7,8-tétrahydro-5,5,8,8-tétraméthyl-2'-bromoacétonaphtone (1,26 g, 4,1 mmol), de 3-hydroxy-4-iodobenzoate de méthyle (1,15 g, 4,1 mmol),et de carbonate de potassium (0,62 g, 4,6 mmol) dans la méthyléthylcétone (25 ml), est chauffée à reflux pendant trois heures. On filtre puis on concentre à l'évaporateur rotatif, le milieu réactionnel. On ajoute 40 ml d'eau et 40 ml d'acétate d'éthyle. Après agitation et décantation, la phase organique est lavée deux fois par 40 ml d' eau, séchée sur sulfate de magnésium et concentrée à l'évaporateur rotatif sous vide à 40 °C. Le produit est purifié par chromatographie flash sur colonne de silice (acétate d' éthyle 10 %, heptane 90 %).
Solide blanc. Masse: 1,69 g, Rendement: 81%. Pf: 124°C
RMN ¹H (CDCL₃, 250 MHz): 1,31 (6H, s),1,32 (6H, s), 1,71 (4H, s), 3,88 (3H, s), 5,42 (2H, s), 7,35 à 7,41 (2H Ar, m), 7,43 (1H Ar, d, J=8,25 Hz), 7,74 (1H Ar, dd, J=8,25,J=2,5 Hz), 7,90 (1H Ar, d, J=7,5 Hz)7,98 (1H Ar, d, J=2,5 Hz).

### 4-lodo-3-[2-[5,6,7,8-tétrahydro-5,5,8,8-tétraméthyl-2-naphtyl]-1-propényloxy]benzoate de méthyle

On additionne en 8 heures, à un mélange de 3-[(5,6,7,8-tétrahydro-5,5,8,8-tétraméthyl-2-naphtoyl) methyloxy]-4-iodobenzoate de méthyle (1,66 g, 3,28 mmol) et de bromure de méthyltriphénylphoshine (1,63 g, 4,56 mmol) dans le THF (40 ml), du méthylate de sodium (0,62 ml, 3,28mmol).
On agite deux jours à température ambiante. Le mélange est concentré à l'évaporateur rotatif sous vide à 40°C.On extrait par 40 ml d'éther éthylique et 40 ml d'eau. Après décantation la phase organique est lavée deux fois par 40 ml d'eau, séchée sur sulfate de magnésium anhydre et concentrée à l'évaporateur rotatif sous vide à 40°C. Le produit est purifié par chromatographie flash sur colonne de silice (CH₂Cl₂ 60 %, heptane 40 %).
Solide blanc. Masse: 0,69 g, Rendement: 42%. Pf: 53°C
RMN ¹H (CDCl₃, 250 MHz): 1,29 (6H, s),1,30 (6H, s), 1,69 (4H, s), 3,91 (3H, s), 4,98 (2H, s), 5,58 (2H, s), 7,20 à 7,41 (4 H Ar, m), 7,50 (1H Ar, d, J=1,15 Hz), 7,87 (1H Ar, d, J=8,00).
RMN ¹³C (CDCl₃, 250 MHz): 31,34, 31,44, 33,71, 33,88, 34,55, 34,69, 51,86, 70,47, 92,84, 112,25, 113,66, 123,01, 123,06, 123,89, 131,08, 135,05, 139,12, 142,01, 144,53, 156,85, 166,05.

### 3-méthyl-3-(5,6,7,8-tétrahydro-5,5,8,8-tétraméthyl-2-naphtyl)-2H-1-benzofuran-6-carboxylate de méthyle

On chauffe à 60°C pendant 4 h un mélange de tributylamine (0,5 ml, 2,1 mmol), de diacétate de palladium (0,03 g, 0,2 mmol), d'acide formique (0,06 ml, 1,3 mmol))et de 4-lodo-3-[2-[5,6,7,8-tétrahydro-5,5,8,8-tétraméthyl-2-naphtyl]-1-propényloxy]benzoate de méthyle (0,65 g, 1,3 mmol) dans l'acétonitrile (10 ml). Le milieu réactionnel est concentré à l'évaporateur rotatif sous vide à 40°C. On ajoute 20 ml d'eau et 20 ml d'éther d'éthylique. Après décantation, la phase organique est lavée deux fois par 20 ml d'eau, séchée sur sulfate de magnésium, et concentrée à l'évaporateur rotatif sous vide à 40°C.
Le produit est purifié par chromatographie flash sur colonne de silice (CH₂Cl₂ 60% heptane 40 %)
Solide blanc. Masse: 330 mg, Rendement: 68%. Pf: 121°C
RMN ¹H (CDCl₃, 250 MHz): 1,20 (3H, s), 1,22 (3H, s), 1,25 (6H, s), 1,66 (4H, s), 1,73 (3H, s), 3,91 (3H, s), 4,48 (1H, d, J=8,75 Hz), 4,62 (1H, d, J=8,75 Hz), 7,00 (1H, dd, J=2 Hz, J=8,25 Hz), 7,09 (1H Ar, d, J=8 Hz), 7,18 à 7,24 (2 H Ar, m), 7,52 (1H Ar, s), 7,63 (1H Ar, d, J=8,00 Hz).
RMN ¹³C (CDCl₃, 250 MHz): 26,16, 31,77, 31,87, 33,96, 34,37, 35,01, 35,10, 49,79, 52,09, 86,48, 110,82, 122,84, 123,67, 123,93, 124,30, 126,60, 130,56, 141,35, 142,15, 143,32, 144,88, 159,80, 166,97.

### EXEMPLE 4

### Procédé de préparation de l'acide 3-méthyl-3-(5,6,7,8-tétrahydro-5,5,8,8-tétraméthyl-2-naphtyl)-2H-1-benzofuran-6-carboxylique

On agite à température ambiante pendant 24 h, un mélange de 3-méthyl-3-(5,6,7,8-tétrahydro-5,5,8,8-tétraméthyl-2-naphtyl)-2H-1-benzofuran-6-carboxylate de méthyle (290 mg, 0,77 mmol), de soude (0,06 g, 1,54 mmol), et d'hydroxyde de lithium (0,06 g, 1,54 mmol). On concentre à l'évaporateur rotatif sous vide à 40°C. On rajoute 10 ml d' eau et 10 ml d'acétate d'éthyle. On acidifie le mélange par une solution d'acide chlorhydrique concentré jusqu'à pH 1. Après décantation, la phase organique est lavée deux fois par 10 ml d'eau, séchée sur sulfate de magnésium, et concentrée à l'évaporateur rotatif sous vide à 40°C. Le produit est obtenu par recristallisation dans un mélange heptane, éther éthylique (3/1).
Solide blanc. Masse: 240 mg, Rendement: 86%. Pf: 206°C
RMN ¹H (DMSO, 250 MHz): 1,17 (6H, s), 1,20 (6H, s), 1,61 (4H, s), 1,70 (3H, s), 4,50 (1H, d, J=9 Hz), 4,65 (1H, d, J=9 Hz), 6,98 (1H, d, J=8,25 Hz), 7,19 à 7,27 (3 H Ar, m), 7,34 (1H Ar, s), 7,54 (1H Ar, d, J=7,75Hz).
RMN ¹³C (DMSO, 250 MHz): 25,81, 31,63, 31,73, 33,69, 34,09, 34,63, 34,71, 49,30, 85,43, 110,14, 122,62, 123,50, 123,79, 124,32, 126,56, 131,37, 140,89, 142,63, 142,72, 144,34, 159,37, 167,15.

### EXEMPLE 5

### Procédé de préparation du 3-méthyl-3-(5,6,7,8-tétrahydro-5,5,8,8-tétraméthyl-2-naphtyl)-2H-1-benzofuran-5-carboxylate de méthyle

### Acide 3-lodo-4-hydroxybenzoïque.

On additionne goutte à goutte une solution de perchlorate de sodium à 3,6% à un mélange d'acide 4-hydroxybenzoïque (12,75 g, 0,92 mol), de soude (3,7 g, 0,92 mol), d'iodure de sodium (13,85 g, 0,92 mol) dans le méthanol (350 ml), à 0°C. On laisse agiter deux heures à 0°C. On additionne 100 ml d'une solution de thiosulfate de sodium à 10%. Après agitation, on acidifie par de l'acide chlorydrique jusqu'à ph 1. On extrait par 600 ml d'éther éthylique. La phase organique est lavée deux fois par 400 ml d'eau, séchée sur sulfate de magnésium et concentrée à l'évaporateur rotatif sous vide à 40°C.
Solide blanc. Masse: 28,76 g. Rendement: quantitatif. Pf: 157°C
RMN ¹H (DMSO, 250 MHz): 6,74 (1H Ar, d, J=8,4 Hz), 7,71 (1H Ar, d, J=8,4 Hz), 8,13 (1H Ar, s), 10,16 (1H, s), 11,12 (1H, s).

### 3-Iodo-4-hydroxybenzoate de méthyle.

On chauffe à reflux une solution d'acide 3-iodo 4-hydroxybenzoïque (28,76 g, 0,11 mol) et d'acide sulfurique (6,6 ml) dans le méthanol (160 ml), pendant 6 h. On ajoute 300 ml d'eau et on alcalinise avec du bicarbonate de soude jusu'à neutralité. On extrait par de l'éther éthylique (600 ml). La phase organique est lavée par deux fois 400 ml d'eau, séchée sur sulfate de magnésium, et concentrée à l'évaporateur rotatif sous vide à 40°C. Le produit est purifié par chromatographie flash sur colonne de silice (acétate d' éthyle 10 %, CH₂Cl₂ 90 %).
Solide blanc. Masse: 19,1 g, Rendement: 63%. Pf: 153°C
RMN ¹H (CDCl₃, 250 MHz): 3,89 (3H, s), 7,01 (2H Ar, d, J=8,5 Hz), 7,94 (1 H Ar, dd, J=8,5 Hz, J=2 Hz), 8,37 (1H Ar d, J=2 Hz)

### 4-[(5,6,7,8-tétrahydro-5,5,8,8-tétraméthyl-2-naphtoyl) méthyloxy]3-iodobenzoate de méthyle.

Une solution de 5,6,7,8-tétrahydro-5,5,8,8-tétraméthyl-2'-bromoacétonaphtone (9,8 g, 0,032 mol), de 4-hydroxy-3-iodobenzoate de méthyle (8,8 g, 0,032 mol),et de carbonate de potassium (8,5 g, 0,062 mol) dans la méthyléthylcétone (450 ml), est chauffée à reflux pendant 1 jour. On filtre puis on concentre à l'évaporateur rotatif, le milieu réactionnel. On ajoute 500 ml d'eau et 500 ml d'éther éthylique. Après agitation et décantation, la phase organique est lavée deux fois par 500 ml d' eau, séchée sur sulfate de magnésium et concentrée à l'évaporateur rotatif sous vide à 40 °C. Le produit est purifié par chromatographie flash sur colonne de silice (acétate d' éthyle 10 %, heptane 90 %).
Solide blanc. Masse: 9,56 g. Rendement: 60%. Pf. 125°C.
RMN ¹H (CDCl₃, 250 MHz): 1,30 (6H, s),1,32 (6H, s), 1,71 (4H, s), 3,88 (3H, s), 5,40 (2H, s), 6,70 (1H Ar, d, J=8,7Hz), 7,43 (1H Ar, d, J=8,5Hz), 7,74 (1H Ar, dd, J=2Hz, J=8,5 Hz), 7,93 (1H Ar, dd, J=8,7,J=2,3 Hz), 7,98 (1H Ar, d, J=2 Hz), 8,48 (1H Ar, d, J=2,3 Hz).

### 3-lodo-4-[2-(5,6,7,8-tétrahydro-5,5,8,8-tétraméthyl-2-naphtyl)-1-propényloxy]benzoate de méthyle

On additionne en 8 heures, à un mélange de 4-[(5,6,7,8-tétrahydro-5,5,8,8-tétraméthyl-2-naphtoyl) méthyloxy]-3-iodobenzoate de méthyle (7,50 g, 14,8 mmol) et de bromure de méthyltriphénylphoshine (7,30 g, 20,42 mmol) dans le THF (80 ml), une solution de méthylate de sodium à 30 % (2,67 g, 14,83 mmol).
On agite 18 heures à température ambiante. Le mélange est concentré à l'évaporateur rotatif sous vide à 40°C. On extrait par 90 ml d'éther éthylique et 90 ml d'eau. Après décantation la phase organique est lavée deux fois par 90 ml d'eau, séchée sur sulfate de magnésium anhydre et concentrée à l'évaporateur rotatif sous vide à 40°C. Le produit est purifié par chromatographie flash sur colonne de silice (CH₂Cl₂ 70 %, heptane 30 %).
Solide blanc. Masse: 4,71 g, Rendement: 63%. Pf: 126°C
RMN ¹H (CDCl₃, 250 MHz): 1,29 (6H, s),1,30 (6H, s), 1,69 (4H, s), 3,89 (3H, s), 4,99 (2H, s), 5,55 (1H, s), 5,59 (1H, s), 6,87 (1H Ar, d, J=8,7Hz), 7,21 à 7,33 (2H Ar, m), 7,38 (1H Ar, d, J=1,8 Hz), 8,00 (1H Ar, dd, J=8,7,J=2 Hz), 8,48 (1H Ar, d, J=2 Hz).
RMN ¹³C (CDCl₃, 250 MHz): 31,79, 31,90, 34,16, 34,33, 34,96, 35,10, 52,09, 70,81, 85,85, 111,35, 112,73, 114,05, 123,33, 124,17, 124,46, 126,71, 129,67, 131,45, 131,74, 135,23, 141,06, 141,99, 145,05, 145,10, 160,67, 165,47.

### 3-méthyl-3-(5,6,7,8-tétrahydro-5,5,8,8-tétraméthyl-2-naphtyl)-2H-1-benzofuran-5-carboxylate de méthyle

On chauffe à 95°C pendant 4 h un mélange de tributylamine (2,28 ml, 9,6 mmol), de diacétate de palladium (0,06 g, 0,3 mmol), d'acide formique (0,29 ml, 7,4 mmol) et de 3-lodo-4-[2-[5,6,7,8-tétrahydro-5,5,8,8-tétraméthyl-2-naphtyl]-1-propényloxy]benzoate de méthyle (1,37 g, 2,72 mmol) dans l'acétonitrile (25 ml). Le milieu réactionnel est concentré à l'évaporateur rotatif sous vide à 40°C. On ajoute 40 ml d'eau et 40 ml d'éther d'éthylique. Après décantation, la phase organique est lavée deux fois par 20 ml d'eau, séchée sur sulfate de magnésium, et concentrée à l'évaporateur rotatif sous vide à;40°C.
Le produit est purifié par chromatographie flash sur colonne de silice (CH₂Cl₂ 50% heptane 50 %)
Solide blanc. Masse: 630 mg, Rendement: 61%. Pf: 74°C
RMN ¹H (CDCl₃, 250 MHz): 1,20 à 1,24 (12H, m), 1,65 (4H, s), 1,73 (3H, s), 3,83 (3H, s), 4,51 (1H, d, J=8,7 Hz), 4,66 (1H, d, J=8,7 Hz), 6,87 (1H Ar, d, J=8,3Hz), 6,96 (1H Ar, dd, J=8,3,J=2 Hz), 7,19 à 7,24 (2H Ar, m), 7,73 (1H Ar, d, J=1,8 Hz), 7,92 (1H Ar, dd, J=8,3,J=2 Hz).
RMN 13C (CDCl₃, 250 MHz): 26,63, 31,99, 32,08, 32,11, 34,18, 35,21, 35,32, 49,45, 52,04, 87,37, 109,82, 123,31, 123,98, 124,36, 126,46, 126,85, 131,41, 136,62, 142,56, 143,49, 145,04, 163,91, 167,19.

### EXEMPLE 6

### Procédé de préparation de l'acide 3-méthyl-3-(5,6,7,8-tétrahydro-5,5,8,8-tétraméthyl-2-naphtyl)-2H-1-benzofuran-5-carboxylique

On agite 5 jours à température ambiante, un mélange de 3-méthyl-3-(5,6,7,8-tétrahydro-5,5,8,8-tétraméthyl-2-naphtyl)-2H-1-benzofuran-5-carboxylate de méthyle (510 mg, 1,35 mmol), de soude (0,33 g, 7,9 mmol), d' hydroxyde de lithium (0,33 g, 7,9 mmol) et d'eau dans le THF. On concentre à l'évaporateur rotatif sous vide à 40°C. On rajoute 10 ml d' eau et 10 ml d'acétate d' éthyle. On acidifie le mélange par une solution d'acide chlorhydrique concentrée jusqu'à pH 1. Après décantation, la phase organique est lavée deux fois par 10 ml d'eau, séchée sur sulfate de magnésium, et concentrée à l'évaporateur rotatif sous vide à 40°C. Le solide obtenu est lavé à l'heptane.
Solide blanc. Masse: 400 mg, Rendement: 83%. Pf: 246°C
RMN ¹H (DMSO, 250 MHz): 1,20 à 1,23 (12H, m), 1,64 (4H, s), 1,74 (3H, s), 3,83 (3H, s), 4,44 (1H, d, J=8,7 Hz), 4,66 (1H, d, J=8,7 Hz), 6,85 (1H Ar, d, J=7,5Hz), 6,96 (1H Ar, dd, J=8,3,J=2 Hz), 7,19 à 7,24 (2H Ar, m), 7,73 (1H Ar, d, J=1,8 Hz), 7,92 (1H Ar, dd, J=8,3,J=2 Hz).
RMN ¹³C (DMSO, 250 MHz): 26,26, 31,63, 31,72, 31,75, 33,83, 34,25, 34,96, 35,06, 49,14, 87,01, 109,34, 123,36, 123,74, 124,03, 126,41, 126,52, 131,38, 136,25, 142,39, 143,02, 144,60, 163,59, 167,90.

### EXEMPLE 7

### Procédé de préparation du 3-méthyl-3-(1,2,3,4-tétrahydro-1,4a,9b-triméthyl-1,4-méthanodibenzofuran-8-yl)-2H-1-benzofuran-6-carboxylate de méthyle.

### 1,2,3,4-Tetrahydro-1,4a,9b-triméthyl-1,4-methanodibenzofuranne; (THTMDBF).

50 g (0,267 mmoles) de bromoanisole en solution dans 120 ml d'ether éthylique sont traités à 0°C par 200 ml de n-Butylithium (1,6 M dans l'hexane), puis le milieu est laissé sous agitation à température ambiante durant la nuit. On ajoute alors goutte à goutte 41,57 g (273 mmoles) de (+) Fenchone (Fluka) dans 100 ml d'éther éthylique et laisse sous agitation 6 h à température ambiante. Le milieu réactionnel est versé dans 200 ml d'une solution saturée de chlorure d'ammonium.
Après extraction par 600 ml d'éther éthylique, rinçage à l'eau, séchage sur sulfate de magnésium, filtration et évaporation, le résidu est chromatographié sur silice pour conduire à 61,26 g (88%) du 2-0-Anisyl-2-endo-fenchyl Alcool fondant à 62-64°C ; αD = + 78° (c = 1, éthanol).

A une solution de 55,24 g (0,21 mmoles) de 2-0-Anisyl-2-endo-fenchyl Alcool et de 4 g de carbonate de calcium dans 800 ml de chloroforme, on ajoute à -10°C, 57,5 g 0,276 mmoles de pentachlorure de phosphore.

Le mélange réactionnel est agité à température ambiante durant deux heures, puis on ajoute du carbonate de potassium (30 g) et on filtre. Le résidu solide est rincé au chloroforme puis chromatographié sur silice dans un mélange hexane/CH₂Cl₂ (9:1) pour conduire à 31,5 g (65%) du dérivé attendu fondant à 68°C ; αD = - 39,5° (c = 1, éthanol).

La même synthèse effectuée à partir de la (-) fenchone conduit à l'isomère dextrogyre du THTMDBF, fondant à 68°C ; αD = + 36,3° (c = 1, éthanol).

### 1,2,3,4-Tetrahydro-1,4a,9b-triméthyl-1,4-methanodibenzofuranne-8-yl, methylcétone

A une solution de 14,67 g de chlorure d'aluminium (0,11 mmoles) dans 150 ml de CH₂Cl₂, on ajoute goutte à goute une solution contenant 22,8 g (0.1 mmole) de (-) THTMDBF et 7,8 ml (0,11 mmoles) de chlorure d'acétyle dans 200 ml de dichlorométhane. Le milieu réactionnel est laissé sous agitation pendant 4 h puis est versé dans l'eau glacée et extrait par CH₂Cl₂. Après un traitement habituel de la phase organique, suivi d'une chromatographie sur silice dans le mélange hexanelether ethylique (85:15), on isole 17,26 g (64%) du dérivé atttendu fondant à 140-142°C ; αD =- 4,3° (c = 1, éthanol).

### 1,2,3,4-Tetrahydro-1,4a,9b-triméthyl-1,4-methanodibenzofuranne-8-yl, bromométhylcétone

18 g (66 mmoles) de la cétone obtenue à l'exemple 3 dans 100 ml de dioxanne et 100 ml d'éther sont traités par addition goutte à goutte d'une solution de 3,4 ml de brome dans 35 ml de CH₂Cl₂. On laisse sous agitation le milieu réactionnel 2 heures à température ambiante puis verse dans l'eau glacée et extrait par 800 ml d'éther éthylique. Après séchage et évaporation, le résidu est chromatographié sur silice dans le mélange Hexane/CH₂Cl₂ (50:50). On obtient 19,75 g (80%) du dérivé attendu sous forme d'huile orangée.

### 3-(1,2,3,4-tétrahydro-1,4a,9b-triméthyl-1,4-méthanodibenzofuran-8-oyl méthyloxy)-4-iodobenzoate de méthyle.

Une solution de 1,2,3,4-tétrahydro-1,4a,9b-triméthyl-1,4-méthanodibenzofuran-8-yl bromométhylcétone (2,5g, 6,86 mmol), de 3-hydroxy-4-iodobenzoate de méthyle (1,9 g, 6,83 mmol) et de carbonate de potassium (0,95 g, 6,88 mmol) dans la méthyléthylcétone (50 ml), est chauffée à reflux pendant cinq heures. On filtre le milieu réactionnel, puis on ajoute 40 ml d'eau et 40 ml d'éther éthylique. Après agitation et décantation, la phase organique est lavée deux fois par 40 ml d' eau, séchée sur sulfate de magnésium et concentrée à l'évaporateur rotatif sous vide à 40 °C. Le produit est purifié par chromatographie flash sur colonne de silice (acétate d' éthyle 30 %, heptane 70 %).
Huile incolore. Masse: 3,2 g, Rendement: 82%.
RMN ¹H (CDCl₃, 250 MHz): 0,85 à 1,67 (16H, m), 2,27 (1H, d, J=3,75 Hz), 3,88 (3H, s), 5,36 (2H, s), 6,79 (1H Ar, d, J=8,5 Hz), 7,38 à 7,40 (2H Ar, m), 7,71 (1H Ar, d, J=1,75 Hz), 7,86 à 7,90 (2H Ar, m).

### 4-iodo-3-(1,2,3,4-tétrahydro-1,4a,9b-triméthyl-1,4-méthanodibenzofuran-8yl)-1-propényloxybenzoate de méthyle.

On additionne, sous azote, à température ambiante, goutte à goutte en 8 h une solution de méthanolate de sodium 30 % dans le méthanol (1 g, 5,56 mmol) à une solution de 3-(1,2,3,4-tétrahydro-1,4a,9b-triméthyl-1,4-méthanodibenzofuran-8-oyl méthyloxy)-4-iodobenzoate de méthyle (3g, 5,50 mmol), de bromure de méthyl triphénylphosphine (2,71 g, 7,58 mmol) dans le THF (30 ml).
On laisse agiter 8 h à température ambiante. Le milieu réactionnel est concentré à l'évaporateur rotatif sous vide à 40°C. On rajoute 50 ml d'eau et 50 ml d'acétate d'éthyle. Après décantation la phase organique est lavée deux fois par 50 ml d'eau, séchée sur sulfate de magnésium, et concentrée à l'évaporateur rotatif sous vide à 40°C. Le produit est purifié par chromatographie flash sur colonne de silice (CH₂Cl₂ 90%, heptane 10%).
Huile incolore. Masse: 2,22 g, Rendement: 74%.
RMN ¹H (CDCl₃, 250 MHz): 0,87 à 1,66 (16H, m), 2,23 (1H, d, J=3,75 Hz), 3,91 (3H, s), 4,97 (2H, s), 5,51 (2H, s), 7,72 (1H Ar, d, J=8,3 Hz), 7,12 (1H Ar, s), 7,38 (1H Ar, d, J=8,1 Hz), 7,26 (1H Ar, dd, J=1,6 Hz), 7,51 (1H Ar, d, J=1,6 Hz), 7,86 (1H Ar, d, J=8,1 Hz).
RMN ¹³C (CDCl₃, 250 MHz): 17,83, 19,44, 21,75, 23,44, 33,97, 42,12, 49,00, 50,72, 52,17, 55,56, 71,02, 93,15, 97,99, 108,59, 112,53, 112,95, 121,44, 123,31, 126,20, 129,97, 131,34, 133,83, 139,41, 142,18, 157,14, 158,75, 166,39

### 3-méthyl-3-(1,2,3,4-tétrahydro-1,4a,9b-triméthyl-1,4-méthanodibenzofuran-8-yl)-2H-1-benzofuran-6-carboxylate de méthyle.

On chauffe 6h à 80°C une solution de 4-iodo-3-(1,2,3,4-tétrahydro-1,4a,9b-triméthyl-1,4-méthanodibenzofuran-8yl)-1-propényloxy benzoate de méthyle (0,71 g, 1,3 mmol) de diacétate de palladium (0,03 g, 0,13 mmol), d'acide formique (0,06 ml, 1,6 mmol), de tributylamine (0,6 ml, 2,6 mmol) dans 10 ml l'acétonitrile. Le milieu réactionnel est concentré à l'évaporateur rotatif sous vide à 40°C. On ajoute 20 ml d'eau et 20 ml d'éther d'éthylique. Après décantation, la phase organique est lavée deux fois par 20 ml d'eau, séchée sur sulfate de magnésium, et concentrée à l'évaporateur rotatif sous vide à;40°C. On obtient un mélange des deux diastéréoisomères avec une proportion de 50/50. Le mélange des deux diastéréoisomères est purifié par chromatographie flash sur colonne de silice (CH₂Cl₂ 80% heptane 20 %)
Huile incolore. Masse: 465 mg, Rendement: 85%.
RMN ¹H (CDCl₃, 250 MHz): 0,76 à 1,63 (16H, m), 1,71 (3H, s), 2,21 (1H, d, J=4,3 Hz), 3,91 (3H, s), 4,48 (1H, d, J=8,5 Hz), 4,58 (1H, d, J=8,5 Hz) et 4,56 (1H, d, J=8,5 Hz): 2 diastéréoisomères, 6,65 (1H Ar, d, J=8,3 Hz, 6,84 (1H Ar, d, J=2 Hz) et 6,87 (1H Ar, d, J=2 Hz): 2 diastéréoisomères, 6,99 à 7,05 (2H Ar, m), 7,51 (1H Ar, s), 7,62 (2H Ar, d, J=7,8 Hz).
RMN ¹³C (CDCl₃, 250 MHz): 17,87 et 17,98: 2 diastéréoisomères, 19,47, 21,92, 23,54, 26,49, 34,08 et 34,12: 2 diastéréoisomères, 42,12 et 42,15: 2 diastéréoisomères, 49,23, 49,57 et 49,65: 2 diastéréoisomères, 50,87, 52,11, 55,73 et 55,76: 2 diastéréoisomères, 86,82 et 86,86: 2 diastéréoisomères, 97,93, 108,53 et 108,61: 2 diastéréoisomères, 110,84, 121,41 et 121,65: 2 diastéréoisomères, 122,92, 123,69 et 123,73: 2 diastéréoisomères, 126,38 et 126,43: 2 diastéréoisomères, 130,52, 134,05, 136,63 et 136,71: 2 diastéréoisomères, 141,86 et 141,93: 2 diastéréoisomères, 157,57, 159,66 et 159,70: 2 diastéréoisomères, 166,98.

### EXEMPLE 8

### Procédé de préparation de l'acide 3-méthyl-3-(1,2,3,4-tétrahydro-1,4a,9b-triméthyl-1,4-méthanodibenzofuran-8-yl)-2H-1-benzofuran-6-carboxylique

On agite 48 h à température ambiante, un mélange de 3-méthyl-3-(1,2,3,4-tétrahydro-1,4a,9b-triméthyl-1,4-méthanodibenzofuran-8-yl)-2H-1-benzofuran-6-carboxylate de méthyle (0,41 g, 0,98 mmol), de soude (0,1 g, 2,5 mmol), d'hydroxyde de lithium (0,1 g, 2,5 mmol) de méthanol et d'eau dans le THF.
On concentre le milieu réactionnel à l'évaporateur rotatif sous vide à 40°C, puis on rajoute 10 ml d'eau. On acidifie la suspension par une solution d'acide chlorhydrique concentré jusqu'à un pH de 1. On filtre et le solide obtenu est recristalliisé dans un mélange heptane éther (1:5).
Solide blanc. Masse: 311 mg, Rendement: 78 %. Pf: 184°C
RMN ¹H (CDCl₃, 250 Mhz) : 0,78 à 1,63 (16H, m), 1,73 (3H, s), 2,21 (1H, d, J=3,8 Hz), 4,51 (1H, d, J=8,5 Hz), 4,59 (1H, d, J=8,5 Hz) et 4,61 (1H, d, J=8,5 Hz): 2 diastéréoisomères, 6,67 (1H Ar, d, J=8,3 Hz), 6,86 (1H Ar, d, J=2 Hz) et 6,89 (1H Ar, d, J=2 Hz): 2 diastéréoisomères, 7,02 à 7,08 (2H Ar, m), 7,59 (1H Ar, s), 7,70 (2H Ar, d, J=7,8 Hz).
RMN ¹³C (CDCl₃, 250 MHz): 17,90 et 18,02: 2 diastéréoisomères, 19,51, 21,94, 23,58, 26,48, 34,11, 42,15, 49,27, 49,65 et 49,73: 2 diastéréoisomères, 50,91, 55,77, 86,88, 98,00, 108,61 et 108,70: 2 diastéréoisomères, 111,40, 121,44 et 121,69: 2 diastéréoisomères, 123,68 et 123,85: 2 diastéréoisomères, 126,42 et 126,49: 2 diastéréoisomères, 129,67, 134,14, 136,53 et 136,60: 2 diastéréoisomères, 142,99 et 143,07: 2 diastéréoisomères, 157,64, 159,74 et 159,78: 2 diastéréoisomères, 171,94.

### EXEMPLE 9

### Procédé de préparation du 3-allyl-3-(5,6,7,8-tétrahydro-5,5,8,8-tétraméthyl-2-naphtyl)-2H-1-benzofuran-6-carboxylate de méthyle

On chauffe à 80°C pendant 3jours un mélange, de bis-(triphénylphosphine)-diacétate de palladium (0,15 g, 0,2 mmol), de tributyl vinyl étain (0,56 ml, 1,92 mmol)) et de 4-lodo-3-[2-[5,6,7,8-tétrahydro-5,5,8,8-tétraméthyl-2-naphtyl]-1-propényloxy]benzoate de méthyle (1 g, 1,99 mmol) dans l'acétonitrile (20 ml) en rajoutant du tributyl vinyl étain (0,28ml, 0,96 mmol) toutes les 24 h. Le milieu réactionnel est concentré à l'évaporateur rotatif sous vide à 40°C. On ajoute 40 ml d'eau et 40 ml d'éther d'éthylique. Après décantation, la phase organique est lavée deux fois par 40 ml d'eau, séchée sur sulfate de magnésium, et concentrée à l'évaporateur rotatif sous vide à;40°C.
Le produit est purifié par chromatographie flash sur colonne de silice (CH₂Cl₂ 80% heptane 20 %)
Huile incolore. Masse: 250 mg, Rendement: 32%.
RMN ¹H (CDCl₃, 250 MHz): 1,13 (6H, s), 1,16 (6H, s), 1,57 (4H, s), 2,77 (2H, t, J=7 Hz), 3,79 (3H, s), 4,49 (1H, d, J=9 Hz), 4,55 (1H, d, J=9 Hz), 4,91 (1H, s), 4,97 (1H, d, J=6,5 Hz), 5,49 (1H, m), 6,92 (1H, dd, J=2 Hz, J=8,25 Hz), 7,05 (1H Ar, d, J=8,00 Hz), 7,07 à 7,14 (2 H Ar, m), 7,41 (1H Ar, s), 7,54 (1H Ar, d, J=8,00 Hz).
RMN ¹³C (CDCl₃, 250 MHz): 31,64, 31,76, 33,82, 34,24, 34,88, 34,97, 43,26, 51,93, 53,30, 83,69, 110,63, 118,78, 122,27, 123,84, 124,53, 124,82, 126,58, 130,58, 133,46, 138,42, 140,85, 143,21, 144,79, 160,24, 166,78.

### EXEMPLE 10

### Procédé de préparation de l'acide 3-allyl-3-(5,6,7,8-tétrahydro-5,5,8,8-tétraméthyl-2-naphtyl)-2H-1-benzofuran-6-carboxylique

On agite. à température ambiante pendant 3jours, un mélange de 3-allyl-3-(5,6,7,8-tétrahydro-5,5,8,8-tétraméthyl-2-naphtyl)-2H-1-benzofuran-6-carboxylate de méthyle (240 mg, 0,61 mmol), de soude (0,12 g, 3 mmol), et d'hydroxyde de lithium (0,06 9, 3 mmol). On concentre à l'évaporateur rotatif sous vide à 40°C. On rajoute 10 ml d' eau et 10 ml d'acétate d'éthyle. On acidifie le mélange par une solution d'acide chlorhydrique concentré iusqu'à pH 1. Après décantation, la phase organique est lavée deux fois par 10 ml d' eau, séchée sur sulfate de magnésium, et concentrée à l'évaporateur rotatif sous vide à 40°C. Le produit est obtenu par recristallisation dans un mélange heptane, éther éthylique (3/1).
Solide blanc. Masse: 128 mg, Rendement: 54%. Pf: 167°C
RMN ¹H (CDCl₃, 250 MHz): 1,23 (6H, s), 1,26 (6H, s), 1,67 (4H, s), 2,89 (2H, t, J=7 Hz), 4,61 (1H, d, J=8,75 Hz), 4,67 (1H, d, J=8,75 Hz), 5,04 (1H, s), 5,08 (1H, d, J=4,5 Hz), 5,60 (1H, m), 7,01 (1H, dd, J=2 Hz, J=8,25 Hz), 7,17 à 77,22 (3H Ar, m), 7,57 (1H Ar, s), 7,71 (1H Ar, dd, J=1,3, J=8,00 Hz).
RMN ¹³C (CDCl₃, 250 MHz): 32,05, 32,18, 34,27, 34,69, 35,29, 35,38, 43,63, 53,78, 84,18, 111,57, 119,31, 123,41, 124,26, 124,95, 125,37, 127,05, 130,05, 133,79, 139,97, 141,11, 143,74, 145,30, 160,69, 171,87.

### EXEMPLE 11

### Procédé de préparation de [3-(5,6,7,8-tétrahydro-5,5,8,8-tétraméthyl-2-naphtyl)-3-méthyl-2H-1-benzofuran-5-oyl]-morpholine

Une solution de l'acide 3-(5,6,7,8-tetrahydro-5,5,8,8-tetramethyl-2-naphty)3-methyl-2H-1-benzofuran-5-carboxylique (100 mg, 0.275 mmol), de 1-hydroxybenzotriazole (74 mg, 0.55mmol), de 1,3-dicyclohexylcarbodiimide (112 mg, 0.55 mmol ) et de morpholine (0.024 ml, 0.255 mmol) dans 5 ml de THF et 2 ml de DMF, est agitée à température ambiante pendant 6 heures. On ajoute 30 mi d'eau et 30 ml d'acétate d'éthyle. Après agitation et décantation, la phase aqueuse est extraite avec 2x 30 ml d'acétate d'éthyle. Les phases organiques sont alors réunies et lavées deux fois avec 30 ml d'eau, puis séchées sur sulfate de magnésium et concentrées à l'évaporateur rotatif sous vide à 40°C. Le produit est alors purifié par chromatographie flash sur colonne de silice (heptane 50 % / acétate d'éthyle 50 %)
Solide blanc. Masse 80 mg. Rendement 70%. Pf 57°C.
RMN ¹H (CDCl₃, 250 MHz): 1,18 (3H, s) ; 1,22 (3H, s); 1,26 (6H, s ) ; 1,66 ( 4H, s) ; 1,73 ( 3H, s ) ; 3,64 ( 8H, m ) ; 4,52 ( 1H, d , J=9Hz ) ; 4,68 ( 1H , d , J=9Hz ) ; 6,87 ( 1H, d , J=8.25Hz ) ; 7,00 ( 1H, d , J=8.0Hz ) ; 7,11 à 7,29 (4H, m)
RMN ¹³C (CDCl₃, 250MHz): 26,03 ; 31,46 ; 31,57 ; 33,64 ; 34,03 ; 34,67 ; 34,77 ; 49,24 ; 66,56 ; 86,38 ; 109,37 ; 123,37 ; 123,64 ; 123,93 ; 126,30 ; 127,41 ; 128,05 ; 136,13 ; 142,01 ; 143,00 ; 144,48 ; 160,83 ; 170,37.

### EXEMPLE 12

### Procédé de préparation de N-4-hydroxyphényl-3-(5,6,7,8-tétrahydro-5,5,8,8-tétraméthyl-2-naphtyl)-3-méthyl-2H-1-benzofuran-5-carboxamide

Une solution de l'acide 3-(5,6,7,8-tetrahydro-5,5,8,8-tetramethyl-2-naphtyl)-3-methyl-2H-1-benzofuran-5-carboxylique (100 mg, 0.275 mmol), de 1-hydroxybenzotriazole (74 mg, 0.55mmol), de 1,3-dicyclohexylcarbodiimide (112 mg, 0.55 mmol) et de 4-aminophénol (30 mg, 0.255 mmol) dans 5 ml de THF et 2 ml de DMF, est agitée à température ambiante pendant 6 heures. On ajoute 30 mi d'eau et 30 ml d'acétate d'éthyle. Après agitation et décantation, la phase aqueuse est extraite avec 2x 30 ml d'acétate d'éthyle. Les phases organiques sont alors réunies et lavées deux fois avec 30 ml d'eau, puis séchées sur sulfate de magnésium et concentrées à l'évaporateur rotatif sous vide à 40°C. Le produit est alors purifié par chromatographie flash sur colone de silice (heptane 50 % / acétate d'éthyle 50 %)
Solide blanc. Masse 45 mg. Rendement 36%. Pf 50°C.
RMN ¹H (CDCl₃, 250mhz) : 1,21 (3H, s) ; 1,25 (9H, s) ; 1,66 (4H, s) ; 1,74 ( 3H, s) ; 4,52 (1H, d , J=9Hz ) ; 4,68 ( 1H, d, J=9Hz) ; 6,70 (2H, d , j=8,5Hz ) ; 6,90 (2H, d , J=8,5Hz ) ; 6,97 ( 1H, dd, J=8.75,2.0 ) ; 7,20 à 7,27 ( 2H, m ) ; 7,55 ( 1H, d, J=2,0Hz ) ; 7,72 ( 2H, m)
RMN ¹³C (CDCl₃, 250MHz): 26,2 ; 31,7 ; 31,9 ; 33,9 ; 34,3 ; 34,9 ; 35,0 ; 49,3 ; 87,0 ; 109,8 ; 115,9 ; 123,3; 123,6 ; 123,8 ; 126,7; 127,4; 128,4; 129,8 ; 136,7 ; 142,2 ; 143,3 ; 144,9 ; 153,7 ; 162,8 ; 166,2

### EXEMPLE 13

### Procédé de préparation du N-butyl-3-méthyl-3-(5,5,8,8-tetramethyl-5,6,7,8-tetrahydro-naphthalen-2-yl)-2H-1-benzofuran-5-carboxamide;

Une solution de l'acide 3-(5,6,7,8-tetrahydro-5,5,8,8-tetramethyl-2-naphtyl)-3-methyl-2H-1-benzofuran-5-carboxylique (100 mg, 0.275 mmol), de 1-hydroxybenzotriazole (74 mg, 0.55mmol), de 1,3-dicyclohexylcarbodiimide (112 mg, 0.55 mmol) et de butylamine (19 mg, 0.255 mmol) dans 5 ml de THF et 2 ml de DMF, est agitée à température ambiante pendant 6 heures. On ajoute 30 ml d'eau et 30 ml d'acétate d'éthyle. Après agitation et décantation, la phase aqueuse est extraite avec 2x 30 ml d'acétate d'éthyle. Les phases organiques sont alors réunies et lavées deux fois avec 30 ml d'eau, puis séchées sur sulfate de magnésium et concentrées à l'évaporateur rotatif sous vide à 40°C. Le produit est alors purifié par chromatographie flash sur colone de silice ( heptane 50 % I acétate d'éthyle 50 %)
Huile claire. Masse 80 mg. Rendement 69%.
RMN ¹H (CDCl₃, 250MHz): 0,93 ( 3H, t) ; 1,24 (3H, s) ; 1,25 (6H, s) ; 1,36 ( 2H, m ) ; 1,39 ( 2H, m ) ; 1,62 ( 4H, s ) ; 1,78 ( 3H, s ) ; 3,40 ( 2H, q, J=7,5Hz, 13,0Hz ); 4,48 ( 1H, d, J=9,0Hz ) ; 4,65 ( 1H, d, J= 9,0Hz ) ; 6,65 ( 1H, d , J=8,4Hz ) ; 6,97 (1H, dd , J= 8,75Hz, 2,0Hz ) ; 7,22 ( 2H, m ) ; 7,48 ( 1H, d, J=2,0Hz ) ; 7,62 (1H, dd, J=8,75Hz, 2,0Hz )
RMN ¹³C ( CDCl3, 250MHz ) : 13,7 ; 20,1 ; 26,2 ; 31,68 ; 31,74 ; 33,8 ; 34,3 ; 34,8 ; 34,9 ; 39,7 ; 49,3 ; 86,9 ; 109,4 ; 123,4 ; 123,8 ; 124,0 ; 126,6 ; 127,7 ; 136,5 ; 142,3 ; 143,2 ; 144,7 ; 162,2 ; 167,1

### EXEMPLE 14

### Procédé de préparation de 3-[4-(1-adamantyl)-3-méthoxyphényl-3-méthyl-2H-1-benzofuran]-6-carboxylate de méthyle

### 3-[4-(1-adamantyl)-3-méthoxybenzoylméthyloxy]-4-iodobenzoate de méthyle.

Une solution de 4-(1-adamantyl)-3-méthoxy-2'-bromoacétophénone (860 mg, 2,37 mmol), de 3-hydroxy-4-iodobenzoate de méthyle (660 mg, 2,37 mmol) et de carbonate de potassium (330 mg, 2,39 mmol) dans la méthyléthylcétone (20 ml), est chauffée à reflux pendant cinq heures. On filtre le milieu réactionnel, puis on ajoute 30 ml d'eau et 30 ml d'éther éthylique. Après agitation et décantation, la phase organique est lavée deux fois par 30 ml d' eau, séchée sur sulfate de magnésium et concentrée à l'évaporateur rotatif sous vide à 40 °C. Le produit est purifié par chromatographie flash sur colonne de silice (CH₂Cl₂).
Solide blanc. Masse: 900 mg, Rendement: 68%. Pf: 136°C
RMN ¹H (CDCl₃, 250 MHz): 1,78 (6H, s), 2,11 (9H, s), 3,89 (3H, s), 3,90 (3H, s), 5,42 (2H, s), 7,32 à 7,42 (3H Ar, m), 7,43 (1H Ar, s, J=1,5 Hz), 7,48 (1H Ar, dd, J=8 Hz, J=1,5Hz), 7,82 (1H Ar, d, J=8 Hz).
RMN ¹³C (CDCl₃, 250 MHz): 28,97 ; 37,03 ; 37,76 ; 40,24 ; 40,32 ; 52,42 ; 55,21 ; 71,62; 93,26 ; 110,35; 112,61; 120,91; 124,15 ; 126,91 ; 131,65 ; 132,98 ; 139,94; 145,59 ; 157,13; 159,34 ; 166,39 ; 192,69.

### 4-Iodo-3-[2-[4-(1-adamantyl)-3-méthoxyphényl]-1-propényloxy]benzoate de méthyle

On additionne en 8 heures, à un mélange de 3-[4-(1-adamantyl)-3-méthoxybenzoylméthyloxy)-4-iodobenzoate de méthyle (870 mg, 1,55 mmol) et de bromure de méthyltriphénylphosphine (790 mg, 2,21 mmol) dans le THF (10 ml), une solution de méthylate de sodium à 30 % dans le méthanol (0,3 ml, 1,6 mmol).
On agite 12 heures à température ambiante. Le mélange est concentré à l'évaporateur rotatif sous vide à 40°C.On extrait par 40 ml d'éther éthylique et 40 ml d'eau. Après décantation la phase organique est lavée deux fois par 40 ml d'eau, séchée sur sulfate de magnésium anhydre et concentré à l'évaporateur rotatif sous vide à 40°C. Le produit est purifié par chromatographie flash sur colonne de silice (CH₂Cl₂ 60 %, heptane 40 %).
Solide blanc. Masse: 535 mg, Rendement: 60 %, Pf: 109-111°C.
RMN ¹H (CDCl₃, 250 MHz): 1,77 (6H, s) ; 2,09 (3H, s) ; 2,10 (6H, s) ; 3,86 (3H, s) ; 3,91 (3H, s) ; 4,98 (2H, s), 5,62 (2H, s), 7,00 à 7,04 (2H Ar, m), 7,21 (1H Ar, d, J=7,25 Hz), 7,38 (1H Ar, dd, J=1,75 Hz, J=8,25 Hz), 7,50 (1H Ar, d, J=1,75Hz), 7,87 (1H Ar, d, J=8 Hz).
RMN ¹³C (CDCl₃, 250 MHz): 14,53 ; 23,11 ; 29,44 ; 29,51 ; 32,30 ; 37,35 ; 37,54 ; 41,00 ; 52,76 ; 55,55 ; 71,29 ; 93,70 ; 110,22 ; 113,12 ; 114,87 ; 118,66 ; 123,98 ; 126,97 ; 131,98 ; 137,35 ; 139,13 ; 140,03 ; 142,52 ; 157,69 ; 159,28 ; 166,93

### 3-[4-(1-adamantyl)-3-méthoxyphényl-3-méthyl-2H-1-benzofuran]-6-carboxylate de méthyle

On chauffe à 60°C pendant 3 h un mélange de tributylamine (0,44 ml, 1,84 mmol), de formiate de sodium (70 mg, 1,01 mmol), de diacétate de palladium (20 mg, 0,092 mmol), de 15 crown 5 (100 µl, 5 µmol) et de 4-Iodo-3-[2-[4-(1-adamantyl)-3-méthoxyphényl]-1-propényloxy]benzoate de méthyle (515 mg, 0,92 mmol) dans l'acétonitrile (10 ml). On ajoute de l'acide formique (0,035 ml, 0,92 mmol) après 3 h de chauffe. Le milieu réactionnel est concentré à l'évaporateur rotatif sous vide à 40°C.
On rajoute 5 ml d'eau et 5 ml d'acétate d' éthyle. Après décantation, la phase organique est lavée deux fois par 5 ml d' eau, séchée sur sulfate de magnésium, et concentrée à l'évaporateur rotatif sous vide à 40°C.
Le produit est purifié par chromatographie flash sur colonne de silice (CH₂Cl₂ 70% heptane 30 %)
Solide blanc. Masse: 362 mg, Rendement: 91%. Pf: 108-111°C
RMN ¹H (CDCl₃, 250 MHz): 1,67 (9H, s), 1,98 (9H, s), 3,65 (3H, s), 3,83 (3H, s), 4,41 (1H, d, J=8,75 Hz), 4,57 (1H, d, J=8,75 Hz), 6,62 (1H Ar, d, J=1,75 Hz), 6,75
(1H Ar, dd, J=8,25 Hz, J=1,75 Hz), 7,02 (1H Ar, d, J=7,75 Hz), 7,07 (1H Ar, d, J=7,75 Hz), 7,44 (1H Ar, s), 7,55 (1H Ar, d, J=8 Hz).
RMN ¹³C (CDCl₃, 250 MHz): 26,04, 29,10, 36,76, 37,15, 40,62, 49,79, 52,15, 54,98, 86,49, 110,17, 110,92, 118,24, 122,93, 123,96, 126,50, 130,72, 137,20, 141,20, 144,07, 158,88, 159,82, 166,97.

### EXEMPLE 15

### Procédé de préparation de l'acide 3-[4-(1-adamantyl)-3-méthoxyphényl-3-méthyl-2H-1-benzofuran]-6-arboxylique

On agite a température ambiante 3 jours un mélange de 3-[4-(1-adamantyl)-3-méthoxyphényl-3-méthyl-2H-1-benzofuran]-6-carboxylate de méthyle (320 mg, 0,74 mmol), de soude (160 mg; 4 mmol), d'hydroxyde de lithium (160 mg, 4 mmol) de méthanol (1 ml) et d'eau (1 ml) dans le THF (6 ml). On concentre à l'évaporateur rotatif sous vide à 40°C. On rajoute 10 ml d'eau et 10 ml d'éther éthylique.On acidifie par une solution d'acide chlorhydrique concentrée jusqu'à un pH =1. Après décantation, la phase organique est lavée deux fois par 10 ml d' eau, séchée sur sulfate de magnésium, et concentrée à l'évaporateur rotatif sous vide à 40°C.
Solide blanc. Masse: 282 mg, Rendement: 91%. Pf: 217-222°C
RMN ¹H (CDCl₃, 250 MHz): 1,76 (9H, s), 2,06 (9H, s), 3,74 (3H, s), 4,51 (1H, d, J=8,75 Hz), 4,67 (1H, d, J=8,75 Hz), 6,71 (1H Ar, s), 6,85 (1H Ar, d, J=8 Hz), 7,09 à 7,17 (2H Ar, m), 7,59 (1H Ar, s), 7,71 (1H Ar, d, J=7,75 Hz).
RMN ¹³C (CDCl₃, 250 MHz): 25,86, 28,85, 36,50, 36,92, 40,39, 49,54, 54,79, 86,16, 109,94, 110,88, 118,03, 122,92, 123,63, 126,24, 131,34, 136,85, 140,62, 143,99, 158,63, 159,52, 168,28.

### EXEMPLE 16

### Procédé de préparation de 3-(3-méthyl-5,6,7,8-tétrahydro-5,5,8,8-tétraméthyl-2-naphtyl)-3-méthyl-2H-1-benzofuran-6-carboxylate de méthyle 3-méthyl-5,6,7,8-tétrahydro-5,5,8,8-tétraméthyl-2'-bromoacétonaphtone

On aditionne goutte à goute une solution de brome (0,6 ml, 11,7 mmol) dans le dichlorométhane (5 ml) à une solution de 3-methyl-5,6,7,8-tétrahydro-5,5,8,8-tétraméthyl-2-acétonaphtone (2,59 g, 10,6 mmol) dans du dioxane (15 ml) et de l'éther éthylique (15 ml). La solution est agitée 1 h à température ambiante, puis est versée sur un mélange eau glace (25 g) et éther éthylique (25 ml). Après décantation la phase organique est lavée deux fois par de l'eau (25ml), séchée sur sulfate de magnésium anhydre et concentrée à l'évaporateur rotatif sous vide à 40°C. Le produit est purifié par chromatographie flash sur colonne de silice (CH₂Cl₂ 50 %, heptane 50 %) (Rf:0,3).
Solide blanc. Masse: 1,2 g, Rendement: 35%. Pf: huile
RMN ¹H (CDCl₃, 250 MHz): 1,29 (6H, s),1,30 (6H, s), 1,70 (4H, s), 2,49 (1H, s), 4,59 (3H, s), 7,18 (1 H Ar, s), 7,67 (1 H Ar, s).

### 3-[(3-méthyl-5,6,7,8-tétrahydro-5,6,8,8-tétraméthyl-2-naphtoyl) méthyloxy]-4-iodobenzoate de méthyle.

Une solution de 3- méthyl-5,6,7,8-tétrahydro-5,5,8,8-tétraméthyl-2'-bromoacétonaphtone (1,20 g, 3,7 mmol), de 3-hydroxy-4-iodobenzoate de méthyle (1,15 g, 4,1 mmol) et de carbonate de potassium (0,56 g, 4 mmol) dans la méthyléthylcétone (25 ml) est chauffée à reflux pendant trois heures. On filtre puis on concentre à l'évaporateur rotatif le milieu réactionnel. On ajoute 40 ml d'eau et 40 ml d'acétate d'éthyle. Après agitation et décantation, la phase organique est lavée deux fois par 40 ml d'eau, séchée sur sulfate de magnésium et concentrée à l'évaporateur rotatif sous vide à 40 °C. Le produit est purifié par chromatographie flash sur colonne de silice (CH₂Cl₂ 80 %, heptane 20 %), (Rf:0,4).
Solide blanc. Masse: 1,65 g, Rendement: 86%. Pf: 89-94°C
RMN ¹H (CDCl₃, 250 MHz) : 1,29 (6H, s),1,31 (6H, s), 1,70 (4H, s), 2,49 (3H, s), 3,88 (3H, s), 5,30 (2H, s), 7,19 (1H Ar, s), 7,30 (1H Ar, s), 7,37 (1H Ar, d, J=8 Hz), 7,63 (1H Ar, s), 7,87 (1H Ar, d, J=8 Hz).
RMN ¹³C (CDCl₃, 250 MHz): 20,94, 31,49, 31,87, 33,97, 34,46, 34,77, 34,83, 52,31, 72,54, 92,94, 112,36, 123,93, 126,99, 130,40, 131,49, 132,15, 139,83, 142,55, 149,94, 156,98, 166,27, 196,63.

### 4-lodo-3-[2-(3-méthyl-5,6,7,8-tétrahydro-5,5,8,8-tétraméthyl-2-naphtyl)-1-propényloxy]benzoate de méthyle

On additionne en 8 heures, à un mélange de 3-(3-méthyl-5,6,7,8-tétrahydro-5,5,8,8-tétraméthyl-2-naphtoylméthyloxy)-4-iodobenzoate de méthyle (1,6 g, 3,1 mmol) et de bromure de méthyltriphénylphoshine (1,51 g, 4,2 mmol) dans le THF (15 ml), du méthylate de sodium (0,62 ml, 3,17mmol).

On agite 3 jours à température ambiante. Le mélange est concentré à l'évaporateur rotatif sous vide à 40°C.On extrait par 30 ml d'éther éthylique et 30 ml d'eau. Après décantation la phase organique est lavée deux fois par 30 ml d'eau, séchée sur sulfate de magnésium anhydre et concentrée à l'évaporateur rotatif sous vide à 40°C. Le produit est purifié par chromatographie flash sur colonne de silice (CH₂Cl₂ 90 %, heptane 10 %) (Rf: 0,67).
Solide blanc. Masse: 0,54 g, Rendement: 34%. Pf: 83-86°C
RMN ¹H (CDCl₃, 250 Mhz) : 1,27 (6H, s),1,29 (6H, s), 1,68 (4H, s), 2,32 (3H, s), 3,89 (3H, s), 4,76 (2H, s), 5,22 (1H, s), 5,77 (1H, s), 7,13 (1 H Ar, d, J= 6,75 Hz), 7,36 à 7,43 (2H Ar, m), 7,87 (1H Ar, d, J=8,00).
RMN ¹³C (CDCl₃, 250 MHz) : 31,91, 31,99, 33,97, 34,06, 35,22, 52,37, 71,51, 93,14, 112, 58, 115,53, 123,50, 127,03, 128,26, 131,57, 132,49, 136,36, 139,62, 142,37, 143,71, 144,46, 157,19, 166,54.

### 3-(3-méthyl-5,6,7,8-tétrahydro-5,5,8,8-tétraméthyl-2-naphtyl)-3-méthyl-2H-1-benzofuran-6-carboxylate de méthyle

On chauffe à 45°C pendant 8 h un mélange de tributylamine (0,4 ml, 0,88 mmol), de diacétate de palladium (0,017 g, 0,083 mmol), d'acide formique (0,034 ml, 0,91 mmol)) de formiate de sodium (60 mg, 0,88 mmol) de 15 crown 5 et de 4-lodo-3-[2-(3-méthyl-5,6,7,8-tétrahydro-5,5,8,8-tétraméthyl-2-naphtyl)-1-propényloxy]benzoate de méthyle (429 mg, 0,83 mmol) dans l'acétonitrile (10 ml). Le milieu réactionnel est concentré à l'évaporateur rotatif sous vide à 40°C. On ajoute 20 ml d'eau et 20 ml d'éther d'éthylique. Après décantation, la phase organique est lavée deux fois par 20 ml d'eau, séchée sur sulfate de magnésium, et concentrée à l'évaporateur rotatif sous vide à;40°C.
Le produit est purifié par chromatographie flash sur colonne de silice (CH₂Cl₂ 90% heptane 10 %)(Rf: 0,38).
Solide blanc. Masse: 180 mg, Rendement: 55%. Pf: 174,6°C RMN ¹H (CDCl₃, 250 MHz): 1,21 (3H, s), 1,26 (9H, s), 1,67 (4H, s), 1,76 (3H, s), 1,97 (3H, s), 4,49 (1H, d, J=9 Hz), 4,78 (1H, d, J=9 Hz), 7,03 à 7,05 (2H Ar, m), 7,28 (1H Ar, s), 7,48 (1H Ar, s), 7,61 (1H Ar, d, J=7,75 Hz).
RMN ¹³C (CDCl₃, 250 MHz): 21,63 ; 29,52 ; 32,03 ; 32,13 ; 32,26 ; 32,38 ; 34,14 ; 34,48 ; 35,54 ; 35,61 ; 50,35 ; 52,51 ; 85,05 ; 110,95 ; 122,94 ; 124,27 ; 125,60 ; 130,87 ; 131,13 ; 133,71 ; 139,10 ; 142,00 ; 142,28 ; 144,06 ; 160,03 ; 167,44.

### EXEMPLE 17

### Procédé de préparation de l'acide 3-(3-méthyl-5,6,7,8-tétrahydro-5,5,8,8-tétraméthyl-2-naphtyl)-3-méthyl-2H-1-benzofuran-6-carboxylique

On agite à température ambiante pendant 24 h un mélange de 3-(3-méthyl-5,6,7,8-tétrahydro-5,5,8,8-tétraméthyl-2-naphtyl)-3-méthyl-2H-1-benzofuran-6-carboxylate de méthyle (140 mg, 0,36 mmol), de soude (0,06 g, 1,54 mmol), et d'hydroxyde de lithium (0,06 g, 1,54 mmol). On concentre à l'évaporateur rotatif sous vide à 40°C. On rajoute 10 ml d'eau et 10 ml d'éther éthylique. On acidifie le mélange par une solution d'acide chlorhydrique concentrée jusqu'à pH 1. Après décantation, la phase organique est lavée deux fois par 10 ml d' eau, séchée sur sulfate de magnésium, et concentrée à l'évaporateur rotatif sous vide à 40°C. Le produit est obtenu par recristallisation dans un mélange heptane, éther éthylique (3/1). (AcOEt 90, heptane 10) (Rf: 0,56).
Solide blanc. Masse: 118 mg, Rendement: 87%. Pf: 247°C
RMN ¹H (CDCl₃, 250 Mhz) : 1,20 (3H, s), 1,25 (9H, s), 1,66 (4H, s), 1,76 (3H, s), 1,98 (3H, s), 4,49 (1H, d, J=9 Hz), 4,77 (1H, d, J=9 Hz), 7,02 à 7,05 (2H Ar, m), 7,27 (1H Ar, s), 7,50 (1H Ar, s), 7,63 (1H Ar, d, J=7,15 Hz).
RMN ¹³C (CDCl₃, 250 MHz): 21,60 ; 29,48 ; 32,00 ; 32,10 ; 32,22 ; 32,35 ; 34,09 ; 34,43; 35,51 ; 35,57 ; 50,30 ; 84,94 ; 111,14; 123,16 ; 124,14 ; 125,57 ; 131,06 ; 131,56 ; 133,66 ; 139,17 ; 141,69 ; 142,20 ; 143,95 ; 159,92 ; 169,11.

### EXEMPLE 18

### Procédé de préparation de 3-(5,6,7,8-tétrahydro-5,5,8,8-tétraméthyl-2-naphtyl)-3-(3,5-di-tert-butyl-4-hydroxybenzyl)-2H-1-benzofuran-6-carboxylate de méthyle

### Tris[3,5-bis(1,1-diméthyléthyl)-4-(triméthylsilyloxy)phényl]boroxine.

Une solution de BuLi dans l'hexane 2,5 M (4,7 ml, 11,8 mmol) est additionée goutte à goutte à une suspension de 4-bromo-2,6-*tert*-butyl triméthylsilyloxyphényle (4g, 11,2 mmol), dans TMEDA/THF (5 ml/40 ml) à-78°C. L'agitation est poursuivie 1h à la même température, puis le triméthylborate est additioné goutte à goutte à -78°C. L'agitation est poursuivie 45 mn à -78°C et 50 ml d'une solution de NH₄Cl saturée sont additionés. Le mélange est laissé revenir à température ambiante. Le pH de la solution est ajusté à 6,5 au moyen d'une solution d'HCl concentré. Le mélange est extrait par du CH₂Cl₂, lavé 2 fois à l'eau, séché et concentré à l'évaporateur rotatif sous vide. Le produit est cristallisé dans l'heptane poudre jaune. Masse: 2,36 g, Rendement: 66%. F=230°C. (235-237°C)¹²⁹
RMN δ ppm:
¹H (250 MHz,CDCl₃) : 0,46 (9H, s), 1,47 (18H, s), 8,16 (2H, s).

### 3-(5,6,7,8-tétrahydro-5,5,8,8-tétraméthyl-2-naphtyl)-3-(3,5-di-tert-butyl-4-hydroxybenzyl))-2H-1-benzofuran-6-carboxylate de méthyle.

Un mélange de diacétate de palladium (9 mg, 0,04 mmol), de 4-Iodo-3-[2-(5,6,7,8-tétrahydro-5,5,8,8-tétraméthyl-2-naphtyl)-1-propényloxy]benzoate de méthyle (180 mg, 0,2 mmol), d'une solution de méthanolate de sodium à 30% dans le méthanol (70 mg, 0,4 mmol) et du produit Tris[3,5-bis(1,1-diméthyléthyl)-4-(triméthylsilyloxy)phényl]boroxine (100 mg, 0,2 mmol) dans le DME (3 ml) est chauffé à 60°C pendant 2h. Le milieu réactionnel est concentré à l'évaporateur rotatif sous vide à 40°C, et traité par 10 ml d'eau et d'éther éthylique. Après décantation, la phase organique est lavée deux fois par 10 ml d'eau, séchée sur sulfate de magnésium, et concentrée à l'évaporateur rotatif sous vide à;40°C.
Le produit est purifié par chromatographie flash sur colonne de silice (CH₂Cl₂ 40% heptane 60 %)
Produit amorphe jaune. Masse: 22 mg, Rendement: 19%.
RMN δ ppm:
¹H (250 MHz,CDCl₃): 1,20 à 1,27 (30H, m, CH3 TTN et tBu), 1,67 (4H, s, CH₂ TTN), 3,10 (1H, d, J=13 Hz, H du CH₂ du BHT), 3,40 (1H, d, J=13 Hz, H du CH₂ du BHT), 3,90 (3H, s, O-CH₃), 4,60 (1H, d, J=9 Hz, H du O-CH₂ ), 4,92 (1H, d, J=9 Hz, H du O-CH₂), 5,03 (1H, s, OH), 6,39 (2H Ar, s), 6,74 (1H Ar, d, J=8 Hz), 7,04 (1H Ar, dd, J=2 Hz, J=8,3 Hz), 7,23 à 7,27 (2H Ar, m), 7,42 (1H Ar, d, J=1,5 Hz), 7,54 (1H Ar, dd, J=1,5 Hz, J=8 Hz).
¹³C (250 MHz,CDCl₃): 30,19 (CH₃ tBu), 31,78 (CH₃ TTN), 31,86 (CH₃ TTN), 32,05 (CH₃ TTN), 33,96 (C tBu), 34,04 (C TTN), 34,39 (C TTN), 35,02 (CH₂ TTN), 35,10 (CH2 TTN), 46,72 (CH₂), 52,10 (C), 55,39 (OCH₃), 83,49 (CH₂O), 111,77 (CH Ar), 121,53 (CH Ar), 124,32 (CH Ar), 124,96 (CH Ar), 126,51 (CH Ar), 126,60 (CH Ar), 126,94 (CH Ar), 127,08 (C Ar), 130,51 (C Ar), 134,86 (C Ar), 137,13 (C Ar), 140,47 (C Ar), 143,21 (C Ar), 145,02 (C Ar), 152,46 (C-O Ar), 160,53 (C-O Ar), 167,01 (COO).

### EXEMPLE 19

### Procédé de préparation de l'acide 3-(5,6,7,8-tétrahydro-5,5,8,8-tétraméthyl-2-naphtyl)-3-(3,5-di-tert-butyl-4-hydroxybenzyl)-2H-1-benzofuran-6-carboxylique

Une solution du produit précédent, de méthanethiolate de sodium (4 équivalents) dans le DMF est chauffée à 100°C pendant 4h. De l'eau et de l'éther éthylique sont additionnés. Le mélange est acidifié à pH=1, par une solution d'acide chlorhydrique concentré. La phase organique est lavée deux fois à l'eau, séchée sur sulfate de magnésium anhydre et concentrée à l'évaporateur rotatif sous vide à 40°C.
poudre blanche. Masse: 71 mg, Rendement: 83%. F=211-213°C.
C₃₈H₄₈O₄ Mw: 568,80
Analyse élémentaire: Calculé: C: 80,24, H: 8,51. Trouvé: C: 80,00, H: 8,62.
SM m/z: 567(M+).
IR (cm⁻¹): 1293 =C-O, 1436 C=C, 1688 C=O, 2959 C-H.
RMN δ ppm:
¹H (250 MHZ,CDCl₃): 1,21 à 1,27 (30H, m, CH₃ TTN et tBu), 1,68 (4H, s, CH₂ TTN), 3,12 (1H, d, J=13 Hz, H du CH₂ du BHT), 3,42 (1H, d, J=13 Hz, H du CH₂ du BHT), 4,61 (1H, d, J=9 Hz, H du O-CH₂), 4,77 (1H, d, J=9 Hz, H du O-CH₂), 5,01 (1H, s, OH), 6,39 (2H Ar, s), 6,76 (1H Ar, d, J=8 Hz), 7,06 (1H Ar, d, J=8 Hz), 7,24 à 7,28 (2H Ar, m), 7,49 (1H Ar, s), 7,61 (1H Ar, d, J=8 Hz), 12,85 (1H, COOH).
13C (250 MHz,CDCl₃): 30,24 (CH₃ tBu), 31,86 (CH3 TTN), 31,92 (CH₃ TTN), 32,10 (CH3 TTN), 34,02 (C tBu), 34,10 (C TTN), 34,46 (C TTN), 35,08 (CH2 TTN), 35,16 (CH2 TTN), 46,72 (CH₂), 55,52 (C), 83,57 (CH₂O), 111,33 (CH Ar), 122,22 (CH Ar), 124,35 (CH Ar), 125,00 (CH Ar), 126,69 (CH Ar), 126,96 (CH Ar), 127,07 (C Ar), 129,55 (C Ar), 134,97 (C Ar), 138,27 (C Ar), 140,39 (C Ar), 143,34 (C Ar), 145,14 (C Ar), 152,55 (C-O Ar), 160,66 (C-O Ar), 171,08 (COO).

### EXEMPLE 20

### Procédé de préparation du 3-(5,6,7,8-tétrahydro-5,5,8,8-tétraméthyl-2-naphtyl)-3-méthyl-2H-1-benzofuran-5-méthanol

Une solution de 1M de borane dans le THF (7,7 ml, 7,7 mmol) est additionnée goutte à goutte à 0°C à une solution de l'acide 3-méthyl-3-(5,6,7,8-tétrahydro-5,5,8,8-tétraméthyl-2-naphtyl)-2H-1-benzofuran-5-carboxylique (1,7g, 4,7mol) dans le THF (10 ml). Le mélange est agité 4h à température ambiante puis 2 m d'une solution de THF et d'eau (1:1) sont additionnés. Après concentration à l'évaporateur rotatif sous vide à 40°C. On extrait à l'acétate d'éthyle. La phase organique est lavée à l'eau, séchée sur sulfate de magnésium anhydre, concentrée à l'évaporateur rotatif sous vide à 40°C. Le produit est purifié par chromatographie flash sur colonne de silice.
Solide amorphe jaune. Masse: 1,7 g, Rendement: quantitatif.
C₂₄H₃₀O₂ Mw: 350,50
Calculé: C: 82,24, H: 8,63. Trouvé: C: 82,40, H: 8,50.
SM m/z: 349(M+).
RMN δ ppm:
¹H (250 MHz,CDCl₃): 1,20 à 1,25 (12H, m), 1,66 (4H, s), 1,72 (3H, s), 3,47 (1H, s), 4,44 (1H, d, J=8,8 Hz), 4,59 (2H, s), 4,60 (1H, d, J=8,8 Hz), 6,84 (1H Ar, d, J=8Hz), 7,01 (1H Ar, dd, J=8,3,J=2,3 Hz), 7,05 (1H Ar, d, J=1,8Hz), 7,17 à 7,22 (3H Ar, m).
¹³C (250 MHz,CDCl₃): 25,86 (CH₃), 31,39 (CH₃ TTN), 31,48 (CH₃ TTN), 31,51 (CH₃ TTN), 33,55 (C TTN), 33,97 (C TTN), 34,65 (CH₂ TTN), 34,75 (CH2 TTN), 49,25 (C), 65,05 (CH₂OH), 86,02 (CH₂O), 109,29 (CH Ar), 123,07 (CH Ar), 123,45 (CH Ar), 123,78 (CH Ar), 126,11 (CH Ar), 127,26 (CH Ar), 133,18 (C Ar), 135,93 (C Ar), 142,38 (C Ar), 142,65 (C Ar), 144,27 (C Ar), 159,00 (C-O Ar).

### EXEMPLE 21

### Procédé de préparation de 3-(5,6,7,8-tétrahydro-5,5,8,8-tétraméthyl-2-naphtyl)-3-méthyl-2H-1-benzofuran-5-carbaldehyde

Un mélange de l'alcool précédemment obtenu (1g, 2,86 mmol), de dichromate de pyridinium (2,15g, 5,7 mmol) dans le dichlorométhane est agité à température ambiante 3h. Après filtration et concentration à l'évaporateur rotatif sous vide à 40°C, le produit est purifié par chromatographie flash sur colonne de silice.
Huile. Masse: 0,98 g, Rendement: 98%.
C₂₄H₂₈O₂ Mw: 348,48.
Analyse élémentaire: Calculé: C: 82,72, H: 8,10. Trouvé: C: 82,50, H: 8,10. SM m/z: 349 (M+).
RMN δ ppm:
1H (250 MHz,CDCl₃): 1,20 à 1,26 (12H, m), 1,67 (4H, s), 1,76 (3H, s), 4,57 (d,1H, J=8,9), 4,73 (d,1H, J=8,9), 6.96 (1H Ar, s), 7,00 (1H Ar, s), 7,20 à 7,25 (2H Ar, m), 7,59 (1H Ar, d, J=1,5Hz), 7,74 (1H Ar, dd, J=8,3 Hz, J=1,8Hz), 9.83 (1H, s). ¹³C (250 MHz,CDCl₃): 26,44 (CH₃), 31,77 (CH₃ TTN), 31,87 (CH₃ TTN), 31,91 (CH₃ TTN), 33,99 (C TTN), 34,39 (C TTN), 34,98 (CH₂ TTN), 35,09 (CH₂ TTN), 49,04 (C), 87,45 (CH₂O), 110,26 (CH Ar), 123,74 (CH Ar), 124,12 (CH Ar), 125,52 (CH Ar), 126,75 (CH Ar), 130,90 (C Ar), 132,95 (CH Ar), 137,68 (C Ar), 141,99 (C Ar), 143,48 (C Ar), 144,94 (C Ar), 155,10 (C-O Ar), 190,67 (CHO).

### EXEMPLE 22

### Procédé de préparation de (-)-3-(5,6,7,8-tetrahydro-5,5,8,8-tetramethyl-2-naphtyl)-3-méthyl-2H-1-benzofuran-5-carboxylate de méthyle.

Un mélange de carbonate de calcium (100 mg, 1 mmol), de diacétate de palladium (10 mg, 0,05 mmol), de formiate de sodium (68 mg, 1 mmol), de 3-Iodo-4-[2-(5,6,7,8-tétrahydro-5,5,8,8-tétraméthyl-2-naphtyl)-1-propényloxy] benzoate de méthyle (250 mg, 05 mmol), de (R)-(+)-2,2'- Bis(diphénylphosphino)-1,l'-binaphtyl (65 mg, 0,1 mmol), et de zeolite d'argent (Aldrich 36,660-9) dans l'acétonitrile (7 ml) est chauffé à 60°C pendant 2 jours. Le milieu réactionnel est filtré sur célite, concentré à l'évaporateur rotatif sous vide à 40°C. 10 ml d'eau et 10 ml d'éther d'éthylique sont additionnés. Après décantation, la phase organique est lavée deux fois par 10 ml d'eau, séchée sur sulfate de magnésium, et concentrée à l'évaporateur rotatif sous vide à 40°C. Le produit est purifié par chromatographie flash sur colonne de silice.
Solide blanc. Masse: 105 mg, Rendement: 56%. [a]_{d}²⁰(CHCl₃): -151°. ee: 68,4%

### EXEMPLE 23

### Procédé de préparation du (+)-3-(5,6,7,8-tetrahydro-5,5,8,8-tetramethyl-2-naphtyl)-3-méthyl-2H-1-benzofuran-5-carboxylate de méthyle.

Même protocole que précédemment avec (S)-(-)-2,2'- Bis(diphénylphosphino)-1,1'-binaphthyl.
Solide blanc. Masse: 75 mg, Rendement: 40%. [α]_{d}²⁰(CHCl₃): +116°. ee: 58,8%.

### EXEMPLE 24

### Procédé de préparation du (-)-3-(5,6,7,8-tetrahydro-5,5,8,8-tetramethyl-2-naphtyl)-3-méthyl-2H-1-benzofuran-6-carboxylate de méthyle.

Le protocole expérimental est analogue à celui suivi pour l'obtention de l'exemple 22 appliqué au 4-Iodo-3-[2-(5,6,7,8-tétrahydro-5,5,8,8-tétraméthyl-2-naphtyl)-1-propényloxy]benzoate de méthyle.
Solide blanc. Masse: 75 mg, Rendement: 40%. [α]_{d}²⁰(CHCl₃): -29,8°. ee: 79,6%.

### EXEMPLE 25

### Procédé de préparation du (+)-3-(5,6,7,8-tetrahydro-5,5,8,8-tetramethyl-2-naphtyl)-3-méthyl-2H-1-benzofuran-6-carboxylate de méthyle.

Le protocole expérimental est analogue à celui suivi pour l'obtention de l'exemple 23 appliqué au 4-lodo-3-[2-(5,6,7,8-tétrahydro-5,5,8,8-tétraméthyl-2-naphtyl)-1-propényloxy]benzoate de méthyle.
Solide blanc. Masse: 75 mg, Rendement: 40%. [α]_{d}²⁰(CHCl₃): +28,5°. ee: 81%.

### EXEMPLE 26

### Procédé de préparation du 3-(5,6,7,8-tétrahydro-5,5,8,8-tétraméthyl-2-naphtyl)-3-(2-hexényl)-2H-1-benzofuran-6-carboxylate de méthyle.

### 4-Iodo-3-[2-(5,6,7,8-tétrahydro-5,5,8,8-tétraméthyl-2-naphtyl)2-octényloxy]benzoate de méthyle.

Le protocole expérimental est analogue à celui suivi pour l'obtention du 4-Iodo-3-[2-(5,6,7,8-tétrahydro-5,5,8,8-tétraméthyl-2-naphtyl)-1-propényloxy]benzoate de
méthyle appliqué au 3-[(5,6,7,8-tétrahydro-5,5,8,8-tétraméthyl-2-naphtoyl)
méthyloxy]-4-iodobenzoate de méthyle et au bromure d'hexyltriphénylphosphonium.
Solide blanc. Masse: 653 mg, Rendement: 21%. F=62°C
C₃₀H₃₉IO₃ Mw= 574,54
Analyse élémentaire: Calculé: C: 62,72, H: 6,84. Trouvé: C: 62,41, H: 6,72.
SM m/z: 574 (M+).
IR (cm⁻¹): 1210 =C-O, 1490 C=C, 1718 C=O, 2956-2962 C-H.
RMN δ ppm:
¹H (250 MHz, CDCl₃): 0,82 à 0,88 (2 fois 3H, m), 1,29 à 1,42 (18H, m, CH₃ TTN et CH₂ 4,5 et 6 ), 1,67 (4H, s, CH₂ TTN), 2,03 (H n°3 pour le produit majoritaire, d, J=7,5Hz), 2,09 (H n°3' pour le produit majoritaire, d, J=7,5Hz), 2,28 (H n°3 pour le produit minoritaire, d, J=7,3Hz), 2,33 (H n°3' pour le produit minoritaire, d, J=7,5Hz), 3,89 (3H, s, pour le produit majoritaire), 3,91 (3H, s, pour le produit minoritaire), 4,78 (2H, s, pour le produit majoritaire ), 4,96 (2H, s, pour le produit minoritaire), 5,96 (1H, t, J=7,5Hz, pour le produit majoritaire), 6,06 (1H, t, J=7,5Hz, pour le produit majoritaire ), 7,05 (1H Ar, d, J=8Hz), 7,15 (1H Ar, s), 7,22 à 7,37 (2 H Ar, m), 7,44 (1H Ar, s, pour le produit majoritaire), 7,52 (1H Ar, s, pour le produit minoritaire), 7,83 (1H Ar, d, J=8).
13C (CDCl₃): 14,45 (CH₃), 22,63 (), 28,76 (), 29,58 (), 31,54 (), 31,65 (), 31,96 (CH₃ TTN), 31,99 (CH3 TTN), 34,18 (C TTN), 34,31 (C TTN), 35,25 (CH₂ TTN), 52,36 (OCH₃), 73,83 (CH₂O), 93,51 (C-1), 112,98 (CH), 123,31 (CH), 125,77 (CH Ar), 126,36 (CH Ar), 127,19 (CH Ar), 131,48 (CH), 135,19 (C), 139,51 (CH), 143,74 (C), 144,59 (C), 157,45 (C-O Ar), 166,65 (COO).

### 3-(5,6,7,8-tétrahydro-5,5,8,8-tétraméthyl-2-naphtyl)-3-(2-hexényl)-2H-1-benzofuran-6-carboxylate de méthyle.

Un mélange de tributylamine (0,28 ml, 1,16 mmol), de diacétate de palladium (24 mg, 0,1 mmol) et de 4-lodo-3-[2-(5,6,7,8-tétrahydro-5,5,8,8-tétraméthyl-2-naphtyl)2-octényloxy]benzoate de méthyle (610 mg, 1,06 mmol) dans l'acétonitrile (15 ml) est chauffe à 80°C pendant 24 h. Le milieu réactionnel est concentré à l'évaporateur rotatif sous vide à 40°C puis traité par 20 ml d'eau et d'éther d'éthylique. Après décantation, la phase organique est lavée deux fois par 20 ml d'eau, séchée sur sulfate de magnésium, et concentrée à l'évaporateur rotatif sous vide à;40°C.
Le produit est purifié par chromatographie flash sur colonne de silice (CH₂Cl₂ 80% heptane 20 %). Amorphe jaune. Masse: 305 mg, Rendement: 64%.
C₃₀H₃₈O₃ Mw: 446,63
Analyse élémentaire:
Calculé: C: 80,68, H: 8,58. Trouvé: C: 80,52, H: 8,38.
SM m/z: 446(M+).
RMN δ ppm:
¹H (250 MHz,CDCl₃): 0,78 à 0,91 (2 fois CH3 des chaines hexyle des deux produits, m), 1,21 à 1,31 (16H, m, 4 CH₃ TTN et 1 CH₂), 1,55 à 1,73 (6H, m, 2 CH₂ TTN et 1 CH₂), 1,83 à 2,12 (2H, m, CH2 allylique), 3,90 (O-CH₃, s), 4,54-4,65 (CH₂O, m), 5,14 à 5,47 (2H éthyléniques, m), 7,00 (2 fois 1H, d, J=8,12 Hz), 7,01 à 7,23 (2 fois 3H Ar, m), 7,47 (2 fois 1H Ar, m), 7,61 (2 fois 1H Ar,m).

### EXEMPLE 27

### Procédé de préparation de l'acide 3-(5,6,7,8-tétrahydro-5,5,8,8-tétraméthyl-2-naphtyl)-3-(2-hexényl)-2H-1-benzofuran-6-carboxylique.

Le protocole expérimental est analogue à celui suivi pour l'obtention de l'exemple 2 appliqué à l'exemple 26.
Amorphe jaune. Masse: 285 mg, Rendement: 80%.
C₂₉H₃₆O₃ Mw: 432,60
SM m/z: 431(M+).

### EXEMPLE 28

### Procédé de préparation de l'acide 3-(5,6,7,8-tétrahydro-5,5,8,8-tétraméthyl-2-naphtyl)-3-hexyl-2H-1-benzofuran-6-carboxylique

Une solution du produit de l'exemple 27 dans l'acétate d'éthyle, en présence de palladium sur charbon sous une pression de 6 bar d'hydrogène est agitée à température ambiante 48 h. Le mélange est filtré sur célite puis concentré à l'évaporateur rotatif sous vide à 40°C.
Le produit est purifié par cristallisation dans CH₃CN.
solide blanc. Masse: 125 mg, Rendement: 45%. F=137°C.
C₂₉H₃₈O₃ Mw: 434,62
Analyse élémentaire: Calculé: C: 80,14, H: 8,81, O: 11,04. Trouvé: C: 79,72, H: 8,71, O: 10,67.
SM m/z: 433 (M+).
IR (cm⁻¹): 1292 =C-O, 1441 C=C, 1687 C=O, 2931-2963 C-H.
RMN δ ppm:
¹H (250 MHz,CDCl₃): 0,85 (3H, t, J=8,7 Hz), 1,20 à 1,26 (20H, m), 1,67 (4H, s), 2,09 (2H, t, J=7 Hz), 4,60 (2H, s), 7,00 (1H, d, J=8,3 Hz), 7,15 à 7,25 (3H Ar, m), 7,56 (1H Ar, s), 7,71 (1H Ar, d J=7,8 Hz),
¹³C (250 Mhz, CDCl₃): 13,88 (CH₃), 22,47(CH₂), 24,42 (CH₂), 29,63 (CH₂), 31,52 (CH₂), 31,66 (CH₃ TTN), 31,78 (CH₃ TTN), 33,85 (C TTN), 34,26 (C TTN), 34,93 (CH₂ TTN), 35,01 (CH₂ TTN), 38,87 (CH₂), 53,79 (C), 84,57 (CH₂O), 111,16 (CH Ar), 122,99 (CH Ar), 123,80 (CH Ar), 124,47 (CH Ar), 126,55 (CH Ar), 129,49 (C Ar), 140,25 (C Ar), 143,12 (C Ar), 144,78 (C Ar), 160,29 (C-O Ar), 171,64 (COO).

### EXEMPLE 29

### Procédé de préparation de 3-Méthoxycarbonylmethyl-3-(5,5,8,8-tetramethyl-5,6,7,8-tétrahydronaphtalen-2-yl)-2H-1-benzofuran-6-carboxylate de méthyle.

### 4-Iodo-3-[3-methoxycarbonyl-2-(5,5,8,8-tetramethyl-5,6,7,8-tetrahydronaphtalen-2-yl)-allyloxy]-benzoate de méthyle.

Le protocole expérimental est analogue à celui suivi pour l'obtention du 4-lodo-3-[2-(5,6,7,8-tétrahydro-5,5,8,8-tétraméthyl-2-naphtyl)-1-propényloxy]benzoate de méthyle appliqué au produit 3-[(5,6,7,8-tétrahydro-5,5,8,8-tétraméthyl-2-naphtoyl) méthyloxy]-4-iodobenzoate de méthyle et au triméthylphosphonoacétate.
Solide blanc. Masse: 225 mg, Rendement: 12%. F=115°C C₂₇H₃₁IO₅ Mw:561,9.
Analyse élémentaire: Calculé: C: 57,66, H: 5,56. Trouvé: C: 57,25, H: 5,68.
RMN δ ppm:
¹H (250 Mhz, CDCl₃): 1,28 (6H, s, CH3 TTN),1,30 (6H, s, CH3 TTN), 1,70 (4H, s, CH2 TTN), 3,59 (3H, s, O-CH3), 3,91 (3H, s, O-CH3), 4,83 (2H, s), 6,50 (1H ,s), 7,07 (1H Ar, d, J=8,2 Hz), 7,22 (1H Ar, s), 7,33 (1H Ar, d, J=8Hz), 7,40 à 7,43 (2H Ar, m), 7,89 (1H Ar, d, J=8,2).
13C (250 MHz, CDCl₃): 32,21 (CH3 TTN), 34,59 (C TTN), 34,66 (C TTN), 35,41 (CH₂ TTN), 51,61 (OCH₃), 52,71 (OCH₃), 71,97 (CH₂O), 93,99 (C-1), 112,81 (CH), 117,30 (CH), 124,28 (CH), 124,84 (CH), 126,70 (CH), 132,01 (C), 133,32 (C), 140,07 (CH Ar), 145,09 (C), 145,76 (C), 151,91 (C), 155,94 (C Ar), 166,65 (COO), 166,74 (COO).

### 3-Méthoxycarbonylmethyl-3-(5,5,8,8-tetramethyl-5,6,7,8-tétrahydronaphtalen-2-yl)-2H-1-benzofuran-6-carboxylate de méthyle.

Le protocole expérimental est analogue à celui suivi pour l'obtention de l'exemple 3 appliqué au produit précedent. Le produit est purifié par chromatographie flash sur colonne de silice (CH₂Cl₂ 80% heptane 20 %). Produit amorphe blanc. Masse: 88 mg, Rendement: 59%.
C₂₇H₃₂O₅ Mw:436,55.
Analyse élémentaire: Calculé: C: 74,29, H: 7,39. Trouvé: C: 74,27, H: 7,28.
SM m/z: 436 (M+).
RMN δ ppm:
¹H (CDCI3): 1,19 (6H, s, CH₃ TTN), 1,22 (6H, s, CH3 TTN), 1,64 (4H, s, CH2 TTN), 3,03 (1H, d, J=15 Hz), 3,31 (1H, d, J=15 Hz), 3,58 (3H, s, O-CH₃), 3,90 (3H, s, O-CH₃), 4,81 (1H, d, J=17 Hz), 4,92 (1H, d, J=17 Hz), 6,96 (1H Ar, d, J=7,5 Hz), 7,14 à 7,21 (3H Ar, m), 7,50 (1H Ar, s), 7,60 (1H Ar, d, J=8,00 Hz).
13C (CDCl₃): 32,06 (CH₃ TTN), 32,16 (CH₃ TTN), 32,20 (CH3 TTN), 34,30 (C TTN), 34,72 (C TTN), 35,33 (CH₂ TTN), 35,40 (CH₂ TTN), 43,24 (CH₂), 52,00 (C), 52,08 (OCH₃), 52,48 (OCH₃), 84,55 (CH₂O), 111,40 (CH Ar), 122,98 (CH Ar), 123,59 (CH Ar), 123,98 (CH Ar), 124,97 (CH Ar), 127,27 (CH Ar), 131,42 (C Ar), 138,78 (C Ar), 140,75 (C Ar), 143,96 (C Ar), 145,35 (C Ar), 160,07 (C-O Ar), 167,19 (COO), 171,36 (COO).

### EXEMPLE 30

### Procédé de préparation de l'acide 3-carboxyméthyl-3-(5,5,8,8-tetraméthyl-5,6,7,8-tétrahydronaphtalen-2-yl)-2H-1-benzofuran-6-carboxylique.

Le protocole expérimental est analogue à celui suivi pour l'obtention de l'exemple 2 appliqué au produit de l'exemple 29.
poudre blanche. Masse: 51 mg, Rendement: 71%. F=268°C.
C₂₅H₂₈O₅ Mw: 408,50
Analyse élémentaire: Calculé: C: 73,51, H: 6,91. Trouvé: C: 72,37, H: 6,88.
SM m/z: 407(M+). IR (cm⁻¹): 1258-1280 =C-O, 1411-1435 C=C, 1699 C=O, 2925 C-H.
RMN δ ppm:
¹H (DMSO): 1,19 (12H, s, CH3 TTN), 1,21 (6H, s, CH3 TTN), 1,52 (4H, s, CH₂ TTN), 3,06 (1H, d, J=16 Hz), 3,54 (1H, d, J=16 Hz), 4,83 (1H, d, J=10 Hz), 5,01 (1H, d, J=10 Hz), 7,04 (1H Ar, d, J=8,3 Hz), 7,21 (1H Ar, d, J=8,3Hz), 7,29 à 7,31 (2H Ar, m), 7,36 (1H Ar, d, J=7,8 Hz), 7,47 (1H Ar, d, J=7,8Hz).
13C (DMSO): 31,52 (CH3 TTN), 31,60 (CH₃ TTN), 33,61 (C TTN), 34,07 (C TTN), 34,61 (CH2 TTN), 42,31 (CH₂), 50,63 (C), 83,44 (CH₂O), 110,06 (CH Ar), 122,28 (CH Ar), 122,86 (CH Ar), 123,13 (CH Ar), 124,59 (CH Ar), 126,57 (CH Ar), 131,40 (C Ar), 139,45 (C Ar), 141,45 (C Ar), 142,63 (C Ar), 144,31 (C Ar), 158,77 (C-O Ar), 167,03 (COO), 172,09 (COO).

### B. EXEMPLES DE FORMULATION

1) VOlE ORALE
(a) On prépare la composition suivante sous la forme d'un comprimé de 0,8 g

| | |
|---|---|
| Composé de l'exemple 1 | 0,005 g |
| Amidon prégélatinisé | 0,265 g |
| Cellulose microcristalline | 0,300 g |
| Lactose | 0,200 g |
| Stéarate de magnésium | 0,030 g |

Pour le traitement de l'acné, on administre à un individu adulte 1 à 3 comprimés par jour pendant 3 à 6 mois selon la gravité du cas traité.
(b) On prépare une suspension buvable, destinée à être conditionnée en ampoules de 5 ml

| | |
|---|---|
| Composé de l'exemple 2 | 0,050 g |
| Glycérine | 0,500 g |
| Sorbitol à 70 % | 0,500 g |
| Saccharinate de sodium | 0,010 g |
| Parahydroxybenzoate de méthyle | 0,040 g |
| Arôme q.s. | |
| Eau purifiée q.s.p. | 5 ml |

Pour le traitement de l'acné, on administre à un individu adulte 1 ampoule par jour pendant 3 mois selon la gravité du cas traité.
(c) On prépare la formulation suivante destinée à être conditionnée en gélules:

| | |
|---|---|
| Composé de l'exemple 3 | 0,025 g |
| Amidon de maïs | 0,060 g |
| Lactose q.s.p. | 0,300 g |

Les gélules utilisées sont constituées de gélatine, d'oxyde de titane et d'un conservateur.
Dans le traitement du psoriasis, on administre à un individu adulte, 1 gélule par jour pendant 30 jours.
2) VOIE TOPIQUE
(a) On prépare la crème Eau-dans l'Huile non ionique suivante :

| | |
|---|---|
| Composé de l'exemple 4 | 0,100 g |
| Mélange d'alcools de lanoline émulsifs, de cires et d'huiles raffinés, vendu par la Société BDF sous la dénomination "Eucérine anhydre" | 39,900 g |
| Parahydroxybenzoate de méthyle | 0,075 g |
| Parahydroxybenzoate de propyle | 0,075 g |
| Eau déminéralisée stérile q.s.p. | 100,000 g |

Cette crème est appliquée sur une peau psoriatique 1 à 2 fois par jour pendant 30 jours.
(b) On prépare un gel en réalisant la formulation suivante:

| | |
|---|---|
| Composé de l'exemple 5 | 0,050 g |
| Erythromycine base | 4,000 g |
| Butylhydroxytoluène | 0,050 g |
| Hydroxypropylcellulose vendue par la société Hercules sous le nom de "KLUCEL HF" | 2,000 g |
| Ethanol (à 95°) q.s.p. | 100,000 g |

Ce gel est appliqué sur une peau atteinte de dermatose ou une peau acnéique 1 à 3 fois par jour pendant 6 à 12 semaines selon la gravité du cas traité.
(c) On prépare une lotion antiséborrhéique en procédant au mélange des ingrédients suivants:

| | |
|---|---|
| Composé de l'exemple 6 | 0,030 g |
| Propylène glycol | 5,000 g |
| Butylhydroxytoluène | 0,100 g |
| Ethanol (à 95°) q.s.p. | 100,000 g |

Cette lotion est appliquée deux fois par jour sur un cuir chevelu séborrhéique et on constate une amélioration significative dans un délai compris entre 2 et 6 semaines.
(d) On prépare une composition cosmétique contre les effets néfastes du soleil en procédant au mélange des ingrédients suivants:

| | |
|---|---|
| Composé de l'exemple 7 | 1,000 g |
| Benzylidène camphre | 4,000 g |
| Triglycérides d'acides gras | 31,000 g |
| Monostéarate de glycérol | 6,000 g |
| Acide stéarique | 2,000 g |
| Alcool cétylique | 1,200 g |
| Lanoline | 4,044 g |
| Conservateurs | 0,300 g |
| Propylène glycol | 2,000 g |
| Triéthanolamine | 0,500 g |
| Parfum | 0,400 g |
| Eau déminéralisée q.s.p | 100,000 g |

Cette composition est appliquée quotidiennement, elle permet de lutter contre le vieillissement photoinduit.
(e) On prépare la crème Huile dans l'Eau non ionique suivante:

| | |
|---|---|
| Composé de l'exemple 8 | 0,500 g |
| Vitamine D3 | 0,020 g |
| Alcool cétylique | 4,000 g |
| Monostéarate de glycérol | 2,500 g |
| Stéarate de PEG 50 | 2,500 g |
| Beurre de Karité | 9,200 g |
| Propylène glycol | 2,000 g |
| Parahydroxybenzoate de méthyle | 0,075 g |
| Parahydroxybenzoate de propyle | 0,075 g |
| Eau déminéralisée stérile q.s.p. | 100,000 g |

Cette crème est appliquée sur une peau psoriatique 1 à 2 fois par jour pendant 30 Jours.
(f) On prépare un gel topique en procédant au mélange des ingrédients suivants:

| | |
|---|---|
| Composé de l'exemple 9 | 0,050 g |
| Ethanol | 43,000 g |
| a -tocophérol | 0,050 g |
| Polymère carboxyvinylique vendu sous la dénomination "Carbopol 941" par la société "Goodrich" | 0,500 g |
| Triéthanolamine en solution aqueuse à 20 % en poids | 3,800 g |
| Eau | 9,300 g |
| Propylène glycol qsp. | 100,000 g |

Ce gel est appliqué dans le traitement de l'acné 1 à 3 fois par jour pendant 6 à 12 semaines selon la gravité du cas traité.
(g) On prépare une lotion capillaire anti-chute et pour la repousse des cheveux en procédant au mélange des ingrédients suivants:

| | |
|---|---|
| Composé de l'exemple 10 | 0,05 g |
| Composé vendu sous la dénomination "Minoxidil" | 1,00 g |
| Propylène glycol | 20,00g |
| Ethanol | 34,92 g |
| Polyéthylèneglycol (masse moléculaire = 400) | 40,00 g |
| Butylhydroxyanisole | 0,01 g |
| Butylhydroxytoluène | 0,02 g |
| Eau qsp | 100,00 g |

On applique cette lotion 2 fois par jour pendant 3 mois sur un cuir chevelu ayant subi une chute de cheveu importante.
(h) On prépare une crème anti-acnéique en procédant au mélange des ingrédients suivants:

| | |
|---|---|
| Composé de l'exemple 6 | 0,050 g |
| Acide rétinoïque | 0,010 g |
| Mélange de stéarates de glycérol et de polyéthylène glycol (75 moles) vendu sous le nom de "Gelot 64" .. par la société "GATTEFOSSE" | 15,000 g |
| Huile de noyau polyoxyéthylénée à 6 moles d'oxyde d'éthylène vendue sous le nom de "Labrafil M2130 CS" par la société "GATTEFOSSE" | 8,000 g |
| Perhydrosqualène | 10,000 g |
| Conservateurs | qs |
| Polyéthylèneglycol (masse moléculaire = 400) | 8,000 g |
| Sel disodique de l'acide éthylène-diamine tétracétique | 0,050 g |
| Eau purifiée qsp | 100,000g |

Cette crème est appliquée sur une peau atteinte de dermatose ou une peau acnéique 1 à 3 fois par jour pendant 6 à 12 semaines.
(i) On prépare une crème huile dans l'eau en réalisant la formulation suivante:

| | |
|---|---|
| Composé de l'exemple 5 | 0,020 g |
| 17-valérate de bétamethasone | 0,050 g |
| S-carboxyméthyl cystéine | 3, 000 g |
| Stéarate de polyoxyéthylène (40 moles d'oxyde d'éthylène) vendu sous le nom de "Myrj 52" par la société "ATLAS" | 4,000 g |
| Monolaurate de sorbitan, polyoxyéthylène à 20 moles d'oxyde d'éthylène vendu sous le nom de "Tween 20" par la société "ATLAS" | 1,800 g |
| Mélange de mono et distéarate de glycérol vendu sous la | |
| dénomination de "Géléol" par la société "GATTEFOSSE" | 4,200 g |
| Propylène glycol | 10,000 g |
| Butylhydroxyanisole | 0,010 g |
| Butylhydroxytoluène | 0,020 g |
| Alcool cétostéarylique | 6,200 g |
| Conservateurs | q.s. |
| Perhydrosqualène | 18,000 g |
| Mélange de triglycérides caprylique-caprique vendu sous la dénomination de "Miglyol 812" par la société "DYNAMIT NOBEL" | 4,000 g |
| Triéthanolamine (99 % en poids) | 2,500 g |
| Eau q.s.p | 100,000g |

Cette crème est appliquée 2 fois par jour sur une peau atteinte de dermatose pendant 30 jours.
(j) On prépare la crème de type huile dans l'eau suivante:

| | |
|---|---|
| Acide lactique | 5,000 g |
| Composé de l'exemple 2 | 0,020 g |
| Stéarate de polyoxyéthylène (40 moles d'oxyde d'éthylène) vendu sous le nom de "Myrj 52" par la société "ATLAS" | 4,000 g |
| Monolaurate de sorbitan, polyoxyéthylène à 20 moles d'oxyde d'éthylène vendu sous le nom de Tween 20" par la société "ATLAS" | 1,800 g |
| Mélange de mono et distéarate de glycérol vendu sous la dénomination de "Geleol" par la société "GATTEFOSSE" | 4,200 g |
| Propylène glycol | 10,000 g |
| Butylhydroxyanisole | 0,010 g |
| Butylhydroxytoluène | 0,020 g |
| Alcool cétostéarylique | 6,200 g |
| Conservateurs | q.s. |
| Perhydrosqualène | 18,000 g |
| Mélange de triglycérides caprylique-caprique vendu sous la dénomination de "Miglyol 812" ..par la société "DYNAMIT NOBEL" | 4,000 g |
| Eau q.s.p. | 100,000 g |

Cette crème est appliquée 1 fois par jour, elle aide à lutter contre le vieillissement qu'il soit photoinduit ou chronofogique.

## Revendications

1. Composés biaryles hétérocycliques, caractérisés par le fait qu'ils répondent à la formule générale (I) suivante: 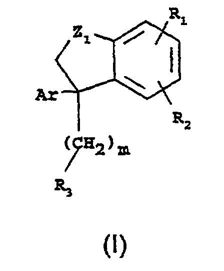 dans laquelle:
- Ar représente
. soit le radical de formule (II) suivante : 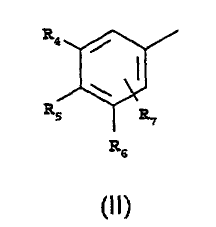
. soit le radical de formule (III) suivante: 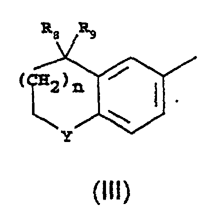
. soit le radical de formule (IV) suivante: 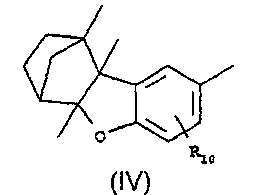
- R₁ représente un atome ou un radical choisi parmi
(i) le radical -CH₃,
(ii) le radical -(CH₂)ₚ-O-R₁₁,
(iii) un radical -OR₁₁,
(iv) un radical 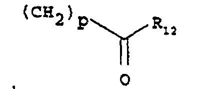
(v) un radical -S(O)ₜR₁₃,
R₁₁, R₁₂, R₁₃, p et t ayant les significations données ci-après,
- R₂ représente un atome d'hydrogène, un atome d'halogène, un radical alkyle ou le radical -OR₁₁,
R₁₁ ayant la signification donnée ci-après,
- R₃ représente un atome ou un radical choisi parmi:
(i) un atome ou un radical choisi parmi l'atome d'hydrogène, un radical alkyle, un radical alkényle, un radical alkynyle, un radical aryle, un radical monohydroxyalkyle ou polyhydroxyalkyle, un radical polyéther, un radical cyano ou un radical -O-R₁₁,
R₁₁ ayant la signification donnée ci-après,
(ii) un radical 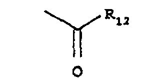 R₁₂ ayant la signification donnée ci-après,
(iii) un radical 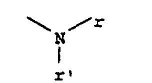 r et r' ayant la signification donnée ci-après
- Z1 représente O,
- m est un nombre entier compris entre 0 et 10,
étant entendu que dans tout ce qui précède :
R₄, R₅, R₆, R₇, identiques ou différents, sont choisis parmi:
(i) un atome d'hydrogène,
(ii) un radical alkyle présentant au moins 4 atomes de carbone, parmi lesquels le carbone attaché au radical phényl est au moins substitué par deux atomes de carbone,
(iii) un radical alcane cyclique ou polycyclique contenant de 1 à 10 atomes de carbone, éventuellement substitué par un ou des atomes d'halogène ou un ou des radicaux hydroxylealcane cyclique ou polycyclique contenant de 1 à 10 atomes de carbone, éventuellement substitué par un ou des atomes d'halogène ou un ou des radicaux hydroxyle,
(iv) un radical -(Z₂)ₙ-(CH₂)_{q}-CO-R₁₂,
(v) un radical -Z₃-R₁₁,
avec au moins un des radicaux R₄, R₅, R₆ et R₇ étant un radical alkyle tel que défini en (ii) ou un radical alcane cyclique ou polycyclique contenant de 1 à 10 atomes de carbone, éventuellement substitué par un ou des atomes d'halogène ou un ou des radicaux hydroxylealcane cyclique ou polycyclique contenant de 1 à 10 atomes de carbone, éventuellement substitué par un ou des atomes d'halogène ou un ou des radicaux hydroxyle (iii), Z₂, Z₃, R₁₁,R₁₂, n et q ayant la signification donnée ci-après,
R₈ et R₉ représentent des radicaux alkyles inférieur,
R₁₀ représente un radical alkyleayant de 1 à 12 atomes de carbone, un radical - OR₁₁ ou un radical polyéther,
R₁₁, identique ou différent, représente un atome d'hydrogène, un radical alkyleayant de 1 à 12 atomes de carbone, un radical aryle, un radical benzyle ou phénéthyle éventuellement substitué par au moins un atome d'halogène, un radical hydroxyle, une fonction nitro ou un groupe méthoxy, un radical monohydroxyalkyle ou polyhydroxyalkyle ou un radical polyéther, un radical acyleayant de 1 à 6 atomes de carbone,
R₁₂, identique ou différent, représente:
(a) un atome d' hydrogène, un radical alkynyle, un radical alkényle, un radical alkyle, un radical pipéridino, morpholino, pyrrolidino ou pipérazino, éventuellement substitué en position 4 par un radical alkyle en C₁-C₆ ou polyhydroxyalkyle,
(b) un radical 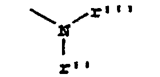 r" et r''' ayant la signification donnée ci-après
(c) un radical -OR₁₃
R₁₃, identique ou différent, représente un atome d'hydrogène, un radical alkyle, un radical monohydroxyalkyle ou polyhydroxyalkyle, un radical aryle ou benzyle ou phénéthyle éventuellement substitué par au moins un atome d'halogène, un radical hydroxyle, une fonction nitro ou un groupe méthoxy éventuellement substitué(s) ou un un reste dérivant de glucose, de galactose ou de mannose, ou bien encore de l'acide glucuronique d'aminoacide ou de reste de dipeptide ou de tripeptide résultant de la combinaison d'acides aminés.
R', identique ou différent, représente un groupement protecteur de fonctions amines, un atome d'hydrogène, un radical alkyleayant de 1 à 12 atomes de carbone, un radical polyéther ou un radical phényle éventuellement substitué par au moins un atome d'halogène, un radical hydroxyle, un radical alkyle, une fonction nitro, un groupe methoxy ou une fonction amine éventuellement substituée ou un reste dérivant de lysine, glycine ou d'acide aspartique , un reste de dipeptide ou de tripeptide résultant de la combinaison d'acides aminés ou un reste dérivant de glucose, de galactose ou de mannose, ou bien encore de l'acide glucuronique,
r et r', identiques ou différents, représentent des groupements protecteurs de fonctions amines, un atome d'hydrogène, un radical alkyleayant de 1 à 12 atomes de carbone, un radical polyéther ou un radical phényle éventuellement substitué par au moins un atome d'halogène, un radical hydroxyle, un radical alkyle, une fonction nitro, un groupe methoxy ou une fonction amine éventuellement substituée ou un reste dérivant de lysine, glycine ou d'acide aspartique, un reste de dipeptide ou de tripeptide résultant de la combinaison d'acides aminés ou un reste dérivant de glucose, de galactose ou de mannose, ou bien encore de l'acide glucuronique, ou encore pris ensemble, forment un radical pipéridino, morpholino, pyrrolidino ou pipérazino, éventuellement substitué en position 4 par un radical alkyle en C1-C6 ou polyhydroxyalkyle,
r" et r''', Identiques ou différents, un atome d' hydrogène, un radical alkyleayant de 1 à 12 atomes de carbone, un radical polyéther ou un radical phényle éventuellement substitué par au moins un atome d'halogène, un radical hydroxyle, un radical alkyle, une fonction nitro, un groupe methoxy ou une fonction amine éventuellement substituée ou un reste dérivant de lysine, glycine ou d'acide aspartique, un reste de dipeptide ou de tripeptide résultant de la combinaison d'acides aminés ou un reste dérivant de glucose, de galactose ou de mannose, ou bien encore de l'acide glucuronique, ou encore pris ensemble, forment un radical pipéridino, morpholino, pyrrolidino ou pipérazino, éventuellement substitué en position 4 par un radical alkyle en C1-C6 ou polyhydroxyalkyle,
Y représente C(R₉)₂, O, S, Nr', CHOH, CO, SO, SO₂,
Z₂ représente O, S, NR',
Z₃ représente O ou S,
n, identique ou différent, est égal à 0 ou 1; p, identique ou différent, est égal à 0,1,2 ou 3; t est égal à 0,1, 2 ou 3; q est un entier compris entre 0 et 10,
ainsi que leurs sels et leurs Isomères optiques et géométriques.

2. Composés selon la revendication 1, caractérisés par le fait qu'ils se présentent sous forme de sels d'un métal alcalin ou alcalino-terreux, de zinc, d'une amine organique, d'un acide minéral ou organique.

3. Composés selon l'une des revendications 1 ou 2, caractérisés par le fait que les radicaux alkyles sont choisis parmi le groupe constitué par les radicaux méthyle, éthyle, isopropyle, butyle, tertiobutyle, hexyle, nonyle et dodécyle.

4. Composés selon l'une des revendications précédentes, caractérisés en ce que les radicaux polyhydroxyalkyles sont choisis parmi le groupe constitué par les radicaux 2,3-dihydroxypropyle, 2,3,4-trihydroxybutyle, 2,3,4,5-tétrahydroxypentyle ou le reste du pentaérythritol.

5. Composés selon l'une des revendications précédentes, caractérisés en ce que les radicaux alkényles sont choisis dans le groupe constitué par les radicaux contenant de 1 à 5 atomes de carbone et présentant une ou plusieurs insaturations éthyléniques, et en particulier le radical allyle.

6. Composés selon l'une quelconque des revendications précédentes, caractérisés en ce que les atomes d'halogène sont choisis dans le groupe constitué par le fluor et le chlore.

7. Composés selon l'une quelconque des revendications précédentes, caractérisés en ce que le radical aminoalkyle est choisi parmi les radicaux aminométhyle, 3-aminopropyle, 6-aminohexyle.

8. Composés selon l'une quelconque des revendications précédentes, caractérisés en ce que le radical alkynyle présente de 2 à 6 atomes de carbone.

9. Composés selon l'une quelconque des revendications précédentes, caractérisés en ce que les radicaux cycloaliphatiques de C3 à C6 atomes de carbone sont choisis parmi le radical cyclopropyle et le radical cyclohexyle.

10. Composés selon l'une quelconque des revendications précédentes, caractérisés en ce que les radicaux acyleayant de 1 à 6 atomes de carbone sont choisis parmi les radicaux acétyle, propionyle ou pivaloyle.

11. Composés selon l'une quelconque des revendications précédentes, caractérisés en ce que les radicaux alcane cyclique ou polycyclique contenant de 1 à 10 atomes de carbone, éventuellement substitué par un ou des atomes d'halogène ou un ou des radicaux hydroxylealcane cyclique ou polycyclique contenant de 1 à 10 atomes de carbone, éventuellement substitué par un ou des atomes d'halogène ou un ou des radicaux hydroxyle sont choisis parmi les radicaux adamantyle ou 1-méthylcyclohéxyle.

12. Composés selon l'une quelconque des revendications précédentes, caractérisés en ce que les radicaux polyéther sont choisis parmi les radicaux méthoxyméthyl éther, méthoxyéthoxyméthyl éther ou méthylthyométhyl éther.

13. Composés selon la revendication 1, caractérisés par le fait qu'ils sont pris, seuls ou en mélanges, dans le groupe constitué par :
- Acide 3-[3-(1-adamantyl)-4-méthoxyphényl]-3-méthyl-2H-1-benzofuran- 6-carboxylique,
- 3-[3-(1-adamantyl)-4-méthoxyphényl]-3-méthyl-2H-1-benzofuran-6-carboxylate de méthyle,
- Acide 3-[3-(1- adamantyl)-4-méthoxyphényl)]-3-méthyl-2H-1-benzofuran-5-carboxylique,
- 3-[3-(1- adamantyl)-4-méthoxyphényl)]-3-méthyl-2H-1-benzofuran-5-carboxylate de méthyle,
- Acide 3-méthyl-3-(5,6,7,8-tétrahydro-5,5,8,8-tétraméthyl-2-naphtyl)-2H-1-benzofuran-6-carboxylique,
- 3-méthyl-3-(5,6,7,8-tétrahydro-5,5,8,8-tétraméthyl-2-naphtyl)-2H-1-benzofuran-6-carboxylate de méthyle,
- Acide 3-(propen-2-yl)-3-(5,6,7,8-tétrahydro-5,5,8,8-tétraméthyl-2-naphtyl)-2H-1-benzofuran-6-carboxylique,
- 3-(propen-2-yl)-3-(5,6,7,8-tétrahydro-5,5,8,8-tétraméthyl-2-naphtyl)-2H-1-benzofuran-6-carboxylate de méthyle,
- Acide 3-(propen-2-yl)-3-(5,6,7,8-tétrahydro-5,5,8,8-tétraméthyl-2-naphtyl)-2H-1-benzofuran-5-carboxylique,
- 3-(propen-2-yl)-3-(5,6,7,8-tétrahydro-5,5,8,8-tétraméthyl-2-naphtyl)-2H-1-benzofuran-5-carboxylate de méthyle,
- Acide 3-méthyl-3-(5,6,7,8-tétrahydro-5,5,8,8-tétraméthyl-2-naphtyl)-2H-1-benzofuran-5-carboxylique,
- 3-méthyl-3-(5,6,7,8-tétrahydro-5,5,8,8-tétraméthyl-2-naphtyl)-2H-1-benzofuran-5-carboxylate de méthyle,
- Acide 3-méthyl-3-(1,2,3,4-tétrahydro-1,4a,9b-triméthyl-1,4-méthanodibenzofuran-8-yl)-2H-1-benzofuran-6-carboxylique,
- 3-méthyl-3-(1,2,3,4-tétrahydro-1,4a,9b-triméthyl-1,4-méthanodibenzofuran-8-yl)-2H-1-benzofuran-6-carboxylate de méthyle,
- Acide 3-méthyl-3-(1,2,3,4-tétrahydro-1,4a,9b-triméthyl-1,4-méthanodibenzofuran-8-yl)-2H-1-benzofuran-5-carboxylique,
- 3-méthyl-3-(1,2,3,4-tétrahydro-1,4a,9b-triméthyl-1,4-méthanodibenzofuran-8-yl)-2H-1-benzofuran-5-carboxylate de méthyle,
- Acide 3-allyl-3-(5,6,7,8-tétrahydro-5,5,8,8-tétraméthyl-2-naphtyl)-2H-1-benzofuran-6-carboxylique,
- 3-allyl-3-(5,6,7,8-tétrahydro-5,5,8,8-tétraméthyl-2-naphtyl)-2H-1-benzofuran-6-carboxylate de méthyle,
- [3-(5,6,7,8-tétrahydro-5,5,8,8-tétraméthyl-2-naphtyl)-3-méthyl-2*H*-1-benzofuran-5-oyl]-morpholine,
- N-4-hydroxyphényl-3-(5,6,7,8-tétrahydro-5,5,8,8-tétraméthyl-2-naphtyl)3-méthyl-*2H*-1-benzofuran-5-carboxamide,
- N-butyl-3-méthyl-3-(5,5,8,8-tetramethyl-5,6,7,8-tetrahydro-naphthalen-2-yl)-*2H*-1-benzofuran-5-carboxamide,
- 3-[4-(1-adamantyl)-3-méthoxyphényl-3-méthyl-*2H*-1-benzofuran]-6-carboxylate de méthyle,
- acide 3-[4-(1-adamantyl)-3-méthoxyphényl-3-méthyl-2H-1-benzofuran]-6-carboxylique,
- 3-(3-méthyl-5,6,7,8-tétrahydro-5,5,8,8-tétraméthyl-2-naphtyl)-3-méthyl-2H-1-benzofuran-6-carboxylate de méthyle,
- acide 3-(3-méthyl-5,6,7,8-tétrahydro-5,5,8,8-tétraméthyl-2-naphtyl)-3-méthyl-*2H*-1-benzofuran-6-carboxylique,
- 3-(5,6,7,8-tétrahydro-5,5,8,8-tétraméthyl-2-naphtyl)-3-(3,5-di-*tert*-butyl-4-hydroxybenzyl)-*2H*-1-benzofuran-6-carboxylate de méthyle,
- acide 3-(5,8,7,8-tétrahydro-5,5,8,8-tétraméthyl-2-naphtyl)-3-(3,5-di-*tert*-butyl-4-hydroxybenzyl)-*2H*-1-benzofuran-6-carboxylique,
- 3-(5,6,7,8-tétrahydro-5,5,8,8-tétraméthyl-2-naphtyl)-3-méthyl-*2H*-1-benzofuran-5-méthanol,
- 3-(5,6,7,8-tétrahydro-5,5,8,8-tétraméthyl-2-naphtyl)-3-méthyl-*2H*-1-benzofuran-5-carbaldehyde,
- (-)-3-(5,6,7,8-tetrahydro-5,5,8,8-tetramethyl-2-naphtyl)-3-méthyl-2*H*-1-benzofuran-5-carboxylate de méthyle,
- (+)-3-(5,6,7,8-tetrahydro-5,5,8,8-tetramethyl-2-naphtyl)-3-méthyl-2*H*-1-benzofuran-5-carboxylate de méthyle,
- (-)-3-(5,6,7,8-tetrahydro-5,5,8,8-tetramethyl-2-naphtyl)-3-méthyl-2*H*-1-benzofuran-6-carboxylate de méthyle,
- (+)-3-(5,6,7,8-tetrahydro-5,5,8,8-tetramethyl-2-naphtyl)-3-méthyl-2H-1-benzofuran-6-carboxylate de méthyle,
- 3-(5,6,7,8-tétrahydro-5,5,8,8-tétraméthyl-2-naphtyl)-3-(2-hexényl)-2*H*-1-benzofuran-6-carboxylate de méthyle,
- acide 3-(5,6,7,8-tétrahydro-5,5,8,8-tétraméthyl-2-naphtyl)-3-(2-hexényl)-2H-1-benzofuran-6-carboxylique,
- acide 3-(5,6,7,8-tétrahydro-5,5,8,8-tétraméthyl-2-naphtyl)-3-hexyl-*2H*-1-benzofuran-6-carboxylique,
- 3-Méthoxycarbonylmethyl-3-(5,5,8,8-tetramethyl-5,6,7,8-tétrahydronaphtalen-2-yl)-2H-1-benzofuran-6-carboxylate de méthyle,
- acide 3-carboxyméthyl-3-(5,5,8,8-tetraméthyl-5,6,7,8-tétrahydronaphtalen-2-yl)-2H-1-benzofuran-6-carboxylique.

14. Composés selon la revendication 1, caractérisés par le fait qu'ils présentent l'une au moins des caractéristiques suivantes:
- R₁ est un radical -(CH₂)ₚ-CO-O-R₁₃
- R₂ est un hydrogène
- R₃ est un hydrogène ou un radical alkényle
- R₅ ou R₆ est un radical -OR₁₁
- R₇ est un radical alcane cyclique ou polycyclique contenant de 1 à 10 atomes de carbone, éventuellement substitué par un ou des atomes d'halogène ou un ou des radicaux hydroxylealcane cyclique ou polycyclique contenant de 1 à 10 atomes de carbone, éventuellement substitué par un ou des atomes d'halogène ou un ou des radicaux hydroxyle
- Z₁ est un atome d' oxygène
- Y est un radical C(R₉)₂
- m est égal à 1.

15. Composés selon l'une quelconque des revendications précédentes pour une utilisation comme médicament.

16. Utilisation d'un composé selon l'une quelconque des revendications 1 à 14 dans la fabrication d'un médicament destiné au traitement:
- des affections dermatologiques liées à un désordre de la kératinisation portant sur la différenciation et sur la prolifération notamment les acnés vulgaires, comédoniennes, polymorphes, rosacées, les acnés nodulokystiques, conglobata, les acnés séniles, les acnés secondaires telles que l'acné solaire, médicamenteuse ou professionnelle;
- des ichtyoses, des états ichtyosiformes, de la maladie de Darier, des kératodermies palmoplantaires, des leucoplasies et des états leucoplasiformes, du lichen cutané ou muqueux(buccal);
- d'affections dermatologiques liées à un trouble de la kératinisation avec une composante inflammatoire et/ou immuno-allergique et, notamment, toutes les formes de psoriasis qu'il soit cutané, muqueux ou unguéal, et même le rhumatisme psoriatique, ou encore l'atopie cutanée, telle que l'eczéma ou l'atopie respiratoire ou encore l'hypertrophie gingivale;des affections inflammatoires ne présentant pas de trouble de la kératinisation;
- des proliférations dermiques ou épidermiques qu'elles soient bénignes ou malignes, qu'elles soient ou non d'origine virale telles que les verrues vulgaires, les verrues planes et l'épidermodysplasie verruciforme, les papillomatoses orales ou florides et les proliférations pouvant être induites par les ultra-violets notamment dans le cas des épithélioma baso et spinocellulaires;
- des désordres dermatologiques tels que les dermatoses bulleuses et les maladies du collagène;
- des troubles ophtalmologiques, notamment les cornéopathies;
- du vieillissement de la peau, qu'il soit photoinduit ou chronologique ou des pigmentations et des kératoses actiniques, ou toutes pathologies associées au vieillissement chronologique ou actinique;
- des stigmates de l'atrophie épidermique et/ou dermique induite par les corticostéroïdes locaux ou systémiques, ou tout autre forme d'atrophie cutanée,
- des troubles de la cicatrisation ou des vergetures;
- des troubles de la fonction sébacée tels que l'hyperséborrhée de l'acné ou la séborrhée simple;
- des états cancéreux ou précancéreux, plus particuliérement les leucémies promyélocytaires;
- d'affections inflammatoires telles que l'arthrite,
- de toute affection d'origine virale au niveau cutané ou général;
- de l'alopécie;
- d'affections dermatologiques à composante immunitaire;
- d'affections du système cardiovasculaire telles que l'artériosclérose ou l'hypertension ainsi que le diabète non-insulino dépendant,
- de désordres cutanés dus à une exposition aux rayonnements U.V..

17. Composition pharmaceutique, caractérisée par le fait qu'elle comprend, dans un support pharmaceutiquement acceptable, au moins l'un des composés tels que définis à l'une quelconque des revendications 1 à 14.

18. Composition selon la revendication 17, caractérisée en ce que la concentration en composé(s) selon l'une des revendication 1 à 14 est comprise entre 0,001 % et 5 % en poids par rapport à l'ensemble de la composition.

19. Composition cosmétique, caractérisée par le fait qu'elle comprend, dans un support cosmétiquement acceptable, au moins l'un des composés tels que définis à l'une quelconque des revendications 1 à 14.

20. Composition selon la revendication 19, caractérisée en ce que la concentration en composé(s) selon l'une des revendications 1 à 14 est comprise entre 0,001 % et 3 % en poids par rapport à l'ensemble de la composition.

21. Utilisation d'une composition cosmétique telle que définie à l'une des revendications 19 ou 20 pour l'hygiène corporelle ou capillaire.

## Patentansprüche

1. Heterocyclische, diaromatische Verbindungen, dadurch gekennzeichnet, daß sie der folgenden allgemeinen Formel (1) 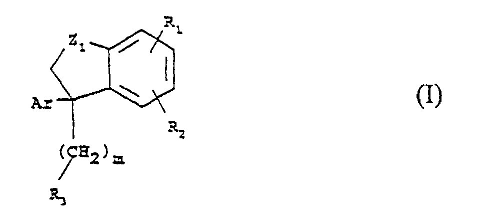 entsprechen, in der bedeuten:
- Ar
• die Gruppe der folgenden Formel (II), 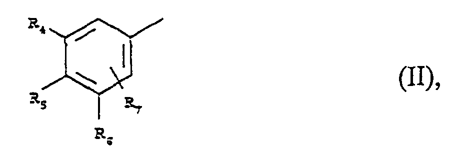
• die Gruppe der folgenden Formel (III), 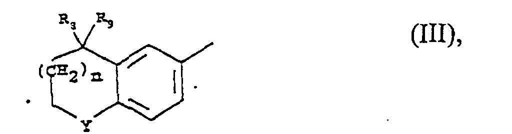
• die Gruppe der folgenden Formel (IV) 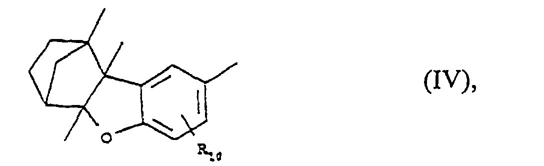
- R₁ ein Atom oder eine Gruppe, das/die ausgewählt ist unter
(i) der Gruppe -CH₃,
(ii) der Gruppe -(CH₂)ₚ-O-R₁₁,
(iii) der Gruppe -OR₁₁,
(iv) der Gruppe 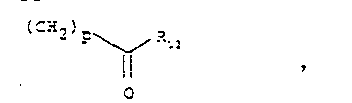
(v) der Gruppe -S(O)ₜR₁₃,
worin R₁₁, R₁₂, R₁₃, p und t die weiter unten angegebene Bedeutung haben
- R₂ ein Wasserstoffatom, ein Halogenatom, eine Alkylgruppe oder eine Gruppe -OR₁₁, worin R₁₁ die weiter unten angegebenen Bedeutung hat,
- R₃ ein Atom oder eine Gruppe, das/die ausgewählt ist unter
(i) Atomen und Gruppen, die ausgewählt sind unter Wasserstoff, Alkylgruppen, Alkenylgruppen, Alkinylgruppen, Arylgruppen, Monohydroxyalkyl- oder Polyhydroxyalkylgruppen, Polyethergruppen, Cyano und der Gruppe -O-R₁₁, worin
R₁₁ die weiter unten angegebene Bedeutung hat,
(ii) den Gruppen der Formel 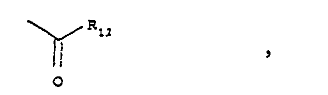 worin R₁₂ die weiter unten angegebene Bedeutung hat, oder
(iii) den Gruppen der Formel 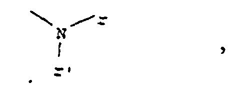 worin r und r' die weiter unten angegebene Bedeutung haben,
- Z₁ Sauerstoff,
- m eine ganze Zahl im Bereich von 0 bis 10,
mit der Maßgabe, daß im folgenden durchweg bedeuten:
- R₄, R₅, R₆, R₇, gleich oder verschieden, Reste, die ausgewählt sind unter
(i) Wasserstoff,
(ii) Alkylgruppen mit mindestens 4 Kohlenstoffatomen, von denen das Kohlenstoffatom, das mit dem Phenylrest verbunden ist, mindestens mit zwei Kohlenstoffatomen substituiert ist,
(iii) cyclischen oder polycyclischen Alkanresten, die 1 bis 10 Kohlenstoffatome enthalten, die gegebenenfalls mit einem oder mehreren Halogenatomen oder einer oder mehreren cyclischen oder polycyclischen Hydroxyalkanresten, die 1 bis 10 Kohlenstoffatome enthalten, substituiert sind, die gegebenenfalls mit einem oder mehreren Halogenatomen oder einer oder mehreren Hydroxygruppen substituiert sind,
(iv) der Gruppe -(Z₂)ₙ-(CH₂)_{q}-CO-R₁₂,
(v) der Gruppe -Z₃-R₁₁,
wobei mindestens einer der Reste R₄, R₅, R₆ und R₇ eine wie in (ii) definierte Alkylgruppe oder gemäß (iii) einen cyclischen oder polycyclischen Alkanrest, der 1 bis 10 Kohlenstoffatome enthält, der gegebenenfalls mit einem oder mehreren Halogenatomen oder einer oder mehreren cyclischen oder polycyclischen Hydroxyalkanresten, die 1 bis 10 Kohlenstoffatome enthalten, substituiert ist, die gegebenenfalls mit einem oder mehreren Halogenatomen oder einer oder mehreren Hydroxygruppen substituiert sind, bedeutet und Z₂, Z₃, R₁₁, R₁₂, n und q die weiter unten angegebene Bedeutung haben,
- R₈ und R₉ niedere Alkylgruppen,
- R₁₀ eine Alkylgruppe, die 1 bis 12 Kohlenstoffatome enthält, eine Gruppe -OR₁₁ oder eine Polyethergruppe,
- R₁₁, gleich oder verschieden, ein Wasserstoffatom, eine Alkylgruppe, die 1 bis 12 Kohlenstoffatome enthält, eine Arylgruppe, eine Benzylgruppe oder Phenethylgruppe, die gegebenenfalls mit mindestens einem Halogenatom, mindestens einer Hydroxygruppe, Nitrogruppe oder Methoxygruppe substituiert ist, eine Monohydroxyalkyl- oder Polyhydroxyalkylgruppe oder eine Polyethergruppe, eine Acylgruppe, die 1 bis 6 Kohlenstoffatome enthält,
- R₁₂, gleich oder verschieden,
(a) ein Wasserstoffatom, eine Alkinylgruppe, eine Alkenylgruppe, eine Alkylgruppe, eine Piperidino-, Morpholino-, Pyrrolidino- oder Piperazinogruppe, die gegebenenfalls in 4-Stellung mit einer C₁-C₆-Alkylgruppe oder Polyhydroxyalkyl substituiert ist,
(b) eine Gruppe 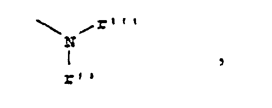 worin r" und r"' die weiter unten angegebene Bedeutung haben,
(c) eine Gruppe -OR₁₃,
- R₁₃, gleich oder verschieden, ein Wasserstoffatom, eine Alkylgruppe, eine Monohydroxyalkylgruppe oder Polyhydroxyalkylgruppe, eine Arylgruppe oder Benzylgruppe oder Phenethylgruppe, die gegebenenfalls mit mindestens einem Halogenatom, mindestens einer Hydroxygruppe, Nitrogruppe oder Methoxygruppe substituiert ist, die gegebenenfalls substituiert ist/sind, oder eine Gruppe, die abgeleitet ist von Glucose, Galactose Mannose oder Glucuronsäure, einer Aminosäure, oder ein Dipeptid- oder Tripeptidrest, der durch die Kombination von Aminosäuren gebildet ist,
- R', gleich oder verschieden, eine Schutzgruppe für Aminfunktionen, ein Wasserstoffatom, eine Alkylgruppe, die 1 bis 12 Kohlenstoffatome aufweist, eine Polyethergruppe oder eine Phenylgruppe, die gegebenenfalls mit mindestens einem Halogenatom, mindestens einer Hydroxygruppe, Alkylgruppe, Nitrogruppe, Methoxygruppe oder mindestens einer gegebenenfalls substituierten Amingruppe substituiert ist, oder ein Rest, der von Lysin, Glycin oder Asparaginsäure abgeleitet ist, ein Dipeptid- oder Tripeptidrest, der durch die Kombination von Aminosäuren gebildet ist, oder eine Gruppe, die von Glucose, Galactose, Mannose oder Glucuronsäure abgeleitet ist,
- r und r', gleich oder verschieden, Schutzgruppen für Aminfunktionen, ein Wasserstoffatom, eine Alkylgruppe, die 1 bis 12 Kohlenstoffatome aufweist, eine Polyethergruppe oder eine Phenylgruppe, die gegebenenfalls mit mindestens einem Halogenatom, mindestens einer Hydroxygruppe, Alkylgruppe, Nitrogruppe, Methoxygruppe oder mindestens einer gegebenenfalls substituierten Amingruppe substituiert ist, oder ein Rest, der von Lysin, Glycin oder Asparaginsäure abgeleitet ist, ein Dipeptid- oder Tripeptidrest, der durch die Kombination von Aminosäuren gebildet ist, oder eine Gruppe, die von Glucose, Galactose, Mannose oder Glucuronsäure abgeleitet ist, oder Reste, die zusammen einen Piperidino-, Morpholino-, Pyrrolidino- oder Piperazinorest bilden, der gegebenenfalls in 4-Stellung mit C₁-C₆-Alkyl oder Polyhydroxyalkyl substituiert ist,
- r" und r"', gleich oder verschieden, ein Wasserstoffatom, eine Alkylgruppe, die 1 bis 12 Kohlenstoffatome enthält, eine Polyethergruppe oder eine Phenylgruppe, die gegebenenfalls mit mindestens einem Halogenatom, mindestens einer Hydroxygruppe, Alkylgruppe, Nitrogruppe, Methoxygruppe oder mindestens einer gegebenenfalls substituierten Amingruppe substituiert ist, oder ein Rest, der von Lysin, Glycin oder Asparaginsäure abgeleitet ist, ein Dipeptid- oder Tripeptidrest, der durch die Kombination von Aminosäuren gebildet ist, oder eine Gruppe, die von Glucose, Galactose, Mannose oder Glucuronsäure abgeleitet ist, oder Reste, die zusammen einen Piperidino-, Morpholino-, Pyrrolidino- oder Piperazinorest bilden, der gegebenenfalls in 4-Stellung mit C₁-C₆-Alkyl oder Polyhydroxyalkyl substituiert ist
- Y C(R₉)₂, O, S, Nr', CHOH, CO, SO, SO₂,
- Z₂ O, S, NR',
- Z₃ O oder S,
- n, gleich oder verschieden, die Zahl 0 oder 1, p, gleich oder verschieden, die Zahl 0, 1, 2 oder 3, T die Zahl 0, 1, 2 oder 3, q eine ganze Zahl im Bereich von 0 bis 10,
und die optischen und geometrischen Isomere dieser Verbindungen der Formel (I) sowie ihre Salze.

2. Verbindungen nach Anspruch 1, dadurch gekennzeichnet, daß sie in Form eines Alkalimetall- oder Erdalkalimetallsalzes, des Zinksalze, eines Salzes eines organischen Amins, einer anorganischen Säure oder einer organischen Säure vorliegen.

3. Verbindungen nach einem der Ansprüche 1 und 2, dadurch gekennzeichnet, daß die Alkylreste unter Methyl, Ethyl, Isopropyl, Butyl, tert.-Butyl, Hexyl, Nonyl und Dodecyl ausgewählt sind.

4. Verbindungen nach einem der vorhergehenden Ansprüche, dadurch gekennzeichnet, daß die Polyhydroxyalkylreste unter 2,3-Dihydroxypropyl, 2,3,4-Trihydroxybutyl, 2,3,4,5-Tetrahydroxypentyl und dem Pentaerythrit-Rest ausgewählt sind.

5. Verbindungen nach einem der vorhergehenden Ansprüche, dadurch gekennzeichnet, daß die Alkenylreste unter Resten, die 1 bis 5 Kohlenstoffatome enthalten und eine oder mehrere ethylenisch ungesättigte Gruppen aufweisen, und insbesondere dem Allylrest ausgewählt sind.

6. Verbindungen nach einem der vorhergehenden Ansprüche, dadurch gekennzeichnet, daß die Halogenatome unter Fluor und Chlor ausgewählt sind.

7. Verbindungen nach einem der vorhergehenden Ansprüche, dadurch gekennzeichnet, daß der Aminoalkylrest unter Aminomethyl, 3-Aminopropyl, 6-Aminohexyl ausgewählt ist.

8. Verbindungen nach einem der vorhergehenden Ansprüche, dadurch gekennzeichnet, daß der Alkinylrest 2 bis 6 Kohlenstoffatome aufweist.

9. Verbindungen nach einem der vorhergehenden Ansprüche, dadurch gekennzeichnet, daß die cycloaliphatischen Reste mit 3 bis 6 Kohlenstoffatomen unter Cyclopropyl und Cyclohexyl ausgewählt sind.

10. Verbindungen nach einem der vorhergehenden Ansprüche, dadurch gekennzeichnet, daß die Acylreste mit 1 bis 6 Kohlenstoffatomen unter Acetyl, Propionyl und Pivaloyl ausgewählt sind.

11. Verbindungen nach einem der vorhergehenden Ansprüche, dadurch gekennzeichnet, daß die cyclischen oder polycyclischen Alkanreste, die 1 bis 10 Kohlenstoffatome enthalten, die gegebenenfalls mit einem oder mehreren Halogenatomen oder einem oder mehreren cyclischen cyclischen oder polycyclischen Hydroxyalkanresten, die 1 bis 10 Kohlenstoffatome enthalten, substituiert sind, die gegebenenfalls mit einem oder mehreren Halogenatomen oder einer oder mehreren Hydroxygruppen substituiert sind, unter Adamantyl und 1-Methylcyclohexyl ausgewählt sind.

12. Verbindungen nach einem der vorhergehenden Ansprüche, dadurch gekennzeichnet, daß die Polyethergruppen unter der Methoxymethylether-, der Methoxyethoxymethylether- und der Methylthiomethylether-Gruppe ausgewählt sind.

13. Verbindungen nach Anspruch 1, dadurch gekennzeichnet, daß sie einzeln oder im Gemisch aus der folgenden Gruppe entnommen werden, die aus den folgenden Verbindungen besteht:
- 3-[3-(1-Adamantyl)-4-methoxyphenyl]-3-methyl-2H-1-benzofuran-6-carbonsäure,
- 3-[3-(1-Adamantyl)-4-methoxyphenyl]-3-methyl-2H-1-benzofuran-6-carbonsäuremethylester,
- 3-[3-(1-Adamantyl)-4-methoxyphenyl]-3-methyl-2H-1-benzofuran-5-carbonsäure,
- 3-[3-(1-Adamantyl)-4-methoxyphenyl]-3-methyl-2H-1-benzofuran-5-carbonsäuremethylester,
- 3-Methyl-3-(5,6,7,8-tetrahydro-5,5,8,8-tetramethyl-2-naphthyl)-2H-1-benzofuran-6-carbonsäure,
- 3-Methyl-3-(5,6,7,8-tetrahydro-5,5,8,8-tetramethyl-2-naphthyl)-2H-1-benzofuran-6-carbonsäuremethylester,
- 3-(Propen-2-yl)-3-(5,6,7,8-tetrahydro-5,5,8,8-tetramethyl-2-naphthyl)-2H-1-benzofuran-6-carbonsäure,
- 3-(Propen-2-yl)-3-(5,6,7,8-tetrahydro-5,5,8,8-tetramethyl-2-naphthyl)-2H-1-benzofuran-6-carbonsäuremethylester,
- 3-(Propen-2-yl)-3-(5,6,7,8-tetrahydro-5,5,8,8-tetramethyl-2-naphthyl)-2H-1-benzofuran-5-carbonsäure,
- 3-(Propen-2-yl)-3-(5,6,7,8-tetrahydro-5,5,8,8-tetramethyl-2-naphthyl)-2H-1-benzofuran-5-carbonsäuremethylester,
- 3-Methyl-3-(5,6,7,8-tetrahydro-5,5,8,8-tetramethyl-2-naphthyl)-2H-1-benzofuran-5-carbonsäure,
- 3-Methyl-3-(5,6,7,8-tetrahydro-5,5,8,8-tetramethyl-2-naphthyl)-2H-1-benzofuran-5-carbonsäuremethylester,
- 3-Methyl-3-(1,2,3,4-tetrahydro-1,4a,9b-trimethyl-1,4-methanodibenzofuran-8-yl)-2H-1-benzofuran-6-carbonsäure,
- 3-Methyl-3-(1,2,3,4-tetrahydro-1,4a,9b-trimethyl-1,4-methanodibenzofuran-8-yl)-2H-1-benzofuran-6-carbonsäuremethylester,
- 3-Methyl-3-(1,2,3,4-tetrahydro-1,4a,9b-trimethyl-1,4-methanodibenzofuran-8-yl)-2H-1-benzofuran-5-carbonsäure,
- 3-Methyl-3-(1,2,3,4-tetrahydro-1,4a,9b-trimethyl-1,4-methanodibenzofuran-8-yl)-2H-1-benzofuran-5-carbonsäuremethylester,
- 3-Allyl-3-(5,6,7,8-tetrahydro-5,5,8,8-tetramethyl-2-naphthyl)-2H-1-benzofuran-6-carbonsäure,
- 3-Allyl-3-(5,6,7,8-tetrahydro-5,5,8,8-tetramethyl-2-naphthyl)-2H-1-benzofuran-6-carbonsäuremethylester,
- [3-(5,6,7,8-Tetrahydro-5,5,8,8-tetramethyl-2-naphthyl)-3-methyl-*2H-*1-benzofuran-5-oyl]-morpholin,
- N-4-Hydroxyphenyl-3-(5,6,7,8-tetrahydro-5,5,8,8-tetramethyl-2-naphthyl)-3-methyl-*2H*-1-benzofuran-5-carboxamid,
- N-Butyl-3-methyl-3-(5,5,8,8-tetramethyl-5,6,7,8-tetrahydronapthalin-2-yl)-*2H*-1-benzofuran-5-carboxamid,
- 3-[4-(1-Adamantyl)-3-methoxyphenyl-3-methyl-*2H*-1-benzofuran]-6-carbonsäuremethylester,
- 3-[4-(1-Adamantyl)-3-methoxyphenyl-3-methyl-2H-1-benzofuran]-6-carbonsäure,
- 3-(3-Methyl-5,6,7,8-tetrahydro-5,5,8,8-tetramethyl-2-naphthyl)-3-methyl-*2H*-1-benzofuran-6-carbonsäuremethylester,
- 3-(3-Methyl-5,6,7,8-tetrahydro-5,5,8,8-tetramethyl-2-naphthyl)-3-methyl-*2H*-1-benzofuran-6-carbonsäure,
- 3-(5,6,7,8-Tetrahydro-5,5,8,8-tetramethyl-2-naphthyl)-3-(3,5-di-*tert*.-butyl-4-hydroxybenzyl)-*2H*-1-benzofuran-6-carbonsäuremethylester,
- 3-(5,6,7,8-Tetrahydro-5,5,8,8-tetramethyl-2-naphthyl)-3-(3,5-di-*tert*.-butyl-4hydroxybenzyl)-*2H*-1-benzofuran-6-carbonsäure,
- 3-(5,6,7,8-Tetrahydro-5,5,8,8-tetramethyl-2-naphthyl)-3-methyl-*2H*-1-benzo-furan-5-methanol,
- 3-(5,6,7,8-Tetrahydro-5,5,8,8-tetramethyl-2-naphthyl)-3-methyl-*2H*-1-benzofuran-5-carbaldehyd,
- (-)-3-(5,6,7,8-Tetrahydro-5,5,8,8-tetramethyl-2-naphthyl)-3-methyl-*2H*-1-benzofuran-5-carbonsäuremethylester,
- (+)-3-(5,6,7,8-Tetrahydro-5,5,8,8-tetramethyl-2-naphthyl)-3-methyl-*2H*-1-benzofuran-5-carbonsäuremethylester,
- (-)-3-(5,6,7,8-Tetrahydro-5,5,8,8-tetramethyl-2-naphthyl)-3-methyl-*2H*-1-benzofuran-6-carbonsäuremethylester,
- (+)-3-(5,6,7,8-Tetrahydro-5,5,8,8-tetramethyl-2-naphthyl)-3-methyl-*2H*-1-benzofuran-6-carbonsäuremethylester,
- 3-(5,6,7,8-Tetrahydro-5,5,8,8-tetramethyl-2-naphthyl)-3-(2-hexenyl)-*2H*-1-benzofuran-6-carbonsäuremethylester,
- 3-(5,6,7,8-Tetrahydro-5,5,8,8-tetramethyl-2-naphthyl)-3-(2-hexenyl)-*2H*-1-benzofuran-6-carbonsäure,
- 3-(5,6,7,8-Tetrahydro-5,5,8,8-tetramethyl-2-naphthyl)-3-hexyl-*2H*-1-benzofuran-6-carbonsäure,
- 3-Methoxycarbonylmethyl-3-(5,5,8,8-tetramethyl-5,6,7,8-tetrahydronaphthalin-2-yl)-*2H*-1-benzofuran-6-carbonsäuremethylester,
- 3-Carboxymethyl-3-(5,5,8,8-tetramethyi-5,6,7,8-tetrahydronaphthalin-2-yl)-*2H*-1-benzofuran-6-carbonsäure,

14. Verbindungen nach Anspruch 1, dadurch gekennzeichnet, daß sie mindestens eine der folgenden Bedingungen erfüllen:
- R₁ ist ein Rest -(CH)ₚ-CO-O-R₁₃,
- R₂ ist ein Wasserstoffatom,
- R₃ ist ein Wasserstoffatom oder ein Alkenylrest,
- R₅ oder R₆ ist ein Rest -OR₁₁,
- R₇ ist ein cyclischer oder polycyclischer Alkanrest, der 1 bis 10 Kohlenstoffatome enthält, die gegebenenfalls mit einem oder mehreren Halogenatomen oder einem oder mehreren cyclischen cyclischen oder polycyclischen Hydroxyalkanresten, die 1 bis 10 Kohlenstoffatome enthalten, substituiert sind, die gegebenenfalls mit einem oder mehreren Halogenatomen oder einer oder mehreren Hydroxygruppen substituiert sind, ausgewählt ist.
- Z₁ stellt ein Sauerstoffatom dar,
- Y ist ein Rest C(R₉)₂.
- m ist die Zahl 1.

15. Verbindungen nach einem der vorhergehenden Ansprüche für eine Verwendung als Arzneimittel.

16. Verwendung einer Verbindung nach einem der Ansprüche 1 bis 14 zur Herstellung eines Arzneimittels, das vorgesehen ist zur Behandlung
- von Hautkrankheiten, die mit einer Störung der Keratinisierung verbunden sind, die die Differenzierung und Zellproliferation betrifft, insbesondere zur Behandlung der Akne vulgaris, Akne comedonica, der polymorphen Akne, der Akne rosaceae, der nodulären Akne, der Akne conglobata, der altersbedingten Aknen, der als Nebenwirkung auftretenden Aknen, wie der Akne solaris, der medikamentenbedingten Akne oder der Akne professionalis,
- anderer Störungen der Keratinisierung, insbesondere der Ichtyosen, der ichtyosiformen Zustände, der Darrier-Krankheit, der Keratosis palmoplantaris, der Leukoplasien, der leukoplasiformen Zustände, der Haut- und Schleimhautflechten (buccal) (Lichen),
- anderer Hauterkrankungen, die mit einer Störung der Keratinisierung zusammenhängen und eine entzündliche und/oder immunoallergische Komponente haben und insbesondere aller Formen der Psoriasis, die die Haut, die Schleimhäute und die Finger- und Zehennägel betreffen, und des psoriatischen Rheumas und der Hautatopien, wie Ekzemen, oder der respiratorischen Atopie oder auch der Hypertrophie des Zahnfleischs,
- von Entzündungen, die nicht mit einer Störung der Keratinisierung zusammenhängen,
- aller gutartigen oder bösartigen Wucherungen der Dermis oder Epidermis, die gegebenenfalls viralen Ursprungs sind, wie Verruca vulgaris, Verruca plana, Epidermodysplasia verruciformis, orale Papillomatose, Papillomatosis florida, und der Wucherungen, die durch UV-Strahlung hervorgerufen werden können, insbesondere des Epithelioma basocellulare und Epithelioma spinocellulare,
- anderer Hautkrankheiten, wie der Dermatitis bullosa und der das Kollagen betreffenden Krankheiten,
- bestimmter Augenkrankheiten, insbesondere der Hornhauterkrankungen,
- der lichtbedingten und der altersbedingten Hautalterung, der Pigmentierungen und der Keratosis actinica und aller Krankheiten, die mit der normalen Alterung oder der lichtbedingten Alterung zusammenhängen,
- von Wunden/Narben der Atrophien der Epidermis und/oder Dermis, die durch lokal oder systemisch angewendete Corticosteroide hervorgerufen werden und aller sonstigen Arten der Hautatrophie,
- von Störungen der Wundheilung und Schwangerschaftsstreifen,
- von Störungen der Talgproduktion, wie Hyperseborrhö bei Akne oder der einfachen Seborrhö,
- von krebsartigen Zuständen oder präkanzerogenen Zuständen, insbesondere der promyelozytären Leukämien,
- von Entzündungserkrankungen, wie Arthritis,
- aller virusbedingten Erkrankungen der Haut oder anderer Bereiche des Körpers,
- der Alopecie,
- von Hautkrankheiten mit einer immunologischen Komponente,
- von Herz-Kreislauf-Erkrankungen, wie Arteriosklerose oder Bluthochdruck, sowie des Insulin-unabhängigen Diabetes,
- von Hautproblemen, die durch UV-Strahlung hervorgerufen werden.

17. Pharmazeutische Zusammensetzung, dadurch gekennzeichnet, daß sie in einem pharmazeutisch akzeptablen Träger mindestens eine der Verbindungen, die wie in einem der Ansprüche 1 bis 14 definiert sind, enthält.

18. Zusammensetzung nach Anspruch 17, dadurch gekennzeichnet, daß die Konzentration an Verbindung(en) nach einem der Ansprüche 1 bis 14 im Bereich von 0,001 bis 5 Gew.-%, bezogen auf das Gesamtgewicht der Zusammensetzung, liegt.

19. Kosmetische Zusammensetzung, dadurch gekennzeichnet, daß sie in einem kosmetisch akzeptablen Träger mindestens eine der wie in einem der Ansprüche 1 bis 14 definierten Verbindungen enthält.

20. Zusammensetzung nach Anspruch 19, dadurch gekennzeichnet, daß die Konzentration an Verbindung(en) nach einem der Ansprüche 1 bis 14 im Bereich von 0,001 bis 3 Gew.-%, bezogen auf das Gesamtgewicht der Zusammensetzung, liegt.

21. Verwendung einer wie in einem der Ansprüche 19 und 20 definierten kosmetischen Zusammensetzung für die Körperreinigung oder die Reinigung der Haare.

## Claims

1. Heterocyclic biaryl compounds, characterized in that they correspond to the general formula (I) below: 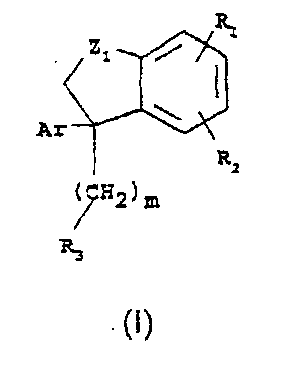 in which:
- Ar represents
. either the radical of formula (II) below: 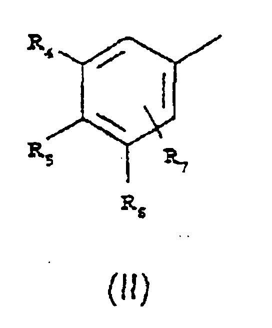
. or the radical of formula (III) below: 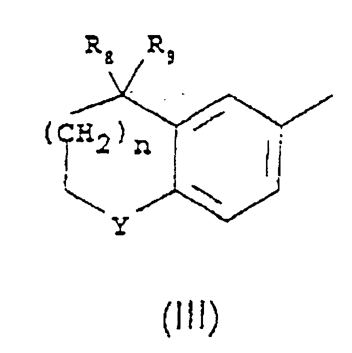
. or the radical of formula (IV) below: 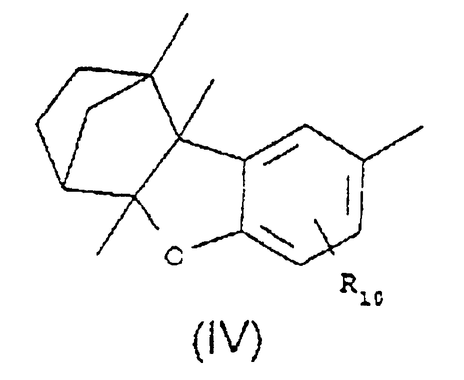
- R₁ represents an atom or a radical chosen from
(i) the -CH₃ radical,
(ii) the radical -(CH₂)ₚ-O-R₁₁,
(iii) a radical -OR₁₁,
(iv) a radical 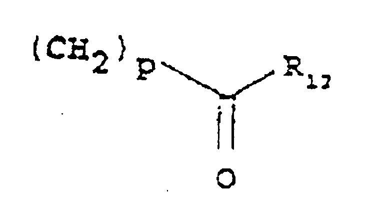
(v) a radical -S(O)ₜR₁₃,
R₁₁, R₁₂, R₁₃, p and t having the meanings given below,
- R₂ represents a hydrogen atom, a halogen atom, an alkyl radical or the radical -OR₁₁,
R₁₁ having the meaning given below,
- R₃ represents an atom or a radical chosen from:
(i) an atom or a radical chosen from a hydrogen atom, an alkyl radical, an alkenyl radical, an alkynyl radical, an aryl radical, a monohydroxyalkyl or polyhydroxyalkyl radical, a polyether radical, a cyano radical or a radical -O-R₁₁,
R₁₁ having the meaning given below,
(ii) a radical 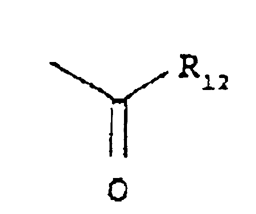 R₁₂ having the meaning given below,
(iii) a radical 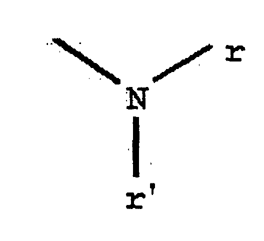 r and r' having the meaning given below,
- Z₁ represents O,
- m is an integer between 0 and 10,
it being understood in all of the preceding text that:
R₄, R₅, R₆ and R₇, which may be identical or different,. are chosen from:
(i) a hydrogen atom,
(ii) an alkyl radical having at least 4 carbon atoms, among which the carbon attached to the phenyl radical is substituted with at least two carbon atoms,
(iii) a cyclic or polycyclic alkane radical containing from 1 to 10 carbon atoms, optionally substituted with one or more halogen atoms or one or more cyclic or polycyclic hydroxyalkane radicals containing from 1 to 10 carbon atoms, optionally substituted with one or more halogen atoms or one or more hydroxyl radicals,
(iv) a radical -(Z₂)ₙ- (CH₂)_{q}-CO-R₁₂,
(v) a radical -Z₃-R₁₁,
with at least one of the radicals R₄, R₅, R₆ and R₇ being an alkyl radical as defined in (ii) or a cyclic or polycyclic alkane radical containing from 1 to 10 carbon atoms, optionally substituted with one or more halogen atoms or one or more cyclic or polycyclic hydroxyalkane radicals containing from 1 to 10 carbon atoms, optionally substituted with one or more halogen atoms or one or more hydroxyl radicals (iii),
Z₂, Z₃, R₁₁, R₁₂, n and q having the meanings given below,
R₈ and R₉ represent lower alkyl radicals,
R₁₀ represents an alkyl radical containing from 1 to 12 carbon atoms, a radical -OR₁₁ or a polyether radical,
R₁₁, which may be identical or different, represents a hydrogen atom, an alkyl radical containing from 1 to 12 carbon atoms, an aryl radical, a benzyl or phenethyl radical optionally substituted with at least one halogen atom, a hydroxyl radical, a nitrofunction or a methoxy group, a monohydroxyalkyl or polyhydroxyalkyl radical or a polyether radical, or an acyl radical containing from 1 to 6 carbon atoms,
R₁₂, which may be identical or different, represents:
(a) a hydrogen atom, an alkynyl radical, an alkenyl. radical, an alkyl radical or a piperidino, morpholino, pyrrolidino or piperazino radical optionally substituted in position 4 with a C₁-C₆ alkyl or polyhydroxyalkyl radical,
(b) a radical 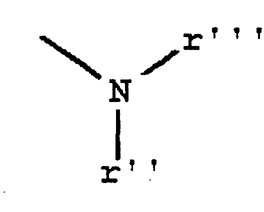 r" and r''' having the meaning given below
(c) a radical -OR₁₃
R₁₃, which may be identical or different, represents a hydrogen atom, an alkyl radical, a monohydroxyalkyl or polyhydroxyalkyl radical, an aryl or benzyl or phenethyl radical optionally substituted with at least one halogen atom, a hydroxyl radical, a nitro function or a methoxy group which is (are) optionally substituted, or a residue derived from glucose, from galactose or from mannose, or alternatively from glucuronic acid residue derived from glucose, from galactose or from mannose, or alternatively from glucuronic acid or from amino acid or from dipeptide or tripeptide residue resulting from a combination of amino acids,
R', which may be identical or different, represents a protecting group for amine functions, a hydrogen atom, an alkyl radical containing from 1 to 12 carbon atoms, a polyether radical or a phenyl radical optionally substituted with at least one halogen atom, a hydroxyl radical, an alkyl radical, a nitro function, a methoxy group or an optionally substituted amine function or a residue derived from lysine, glycine or aspartic acid, a dipeptide or tripeptide residue resulting from a combination of amino acids or a residue derived from glucose, from galactose or from mannose, or alternatively from glucuronic acid,
r and r', which may be identical or different, represent protecting groups for amine functions, a hydrogen atom, an alkyl radical containing from 1 to 12 carbon atoms, a polyether radical or a phenyl radical optionally substituted with at least one halogen atom, a hydroxyl radical, an alkyl radical, a nitro function, a methoxy group or an optionally substituted -amine function or a residue derived from lysine, glycine or aspartic acid, a dipeptide or tripeptide residue resulting from a combination of amino acids or a residue derived from glucose, from galactose or from mannose, or alternatively from glucuronic acid, or else, taken together, form a piperidino, morpholino, pyrrolidino or piperazino radical, optionally substituted in position 4 with a C₁-C₆ alkyl or polyhydroxyalkyl radical,
r" and r"', which may be identical or different, a hydrogen atom, an alkyl radical containing from 1 to 12 carbon atoms, a polyether radical or a phenyl radical optionally substituted with at least one halogen atom, a hydroxyl radical, an alkyl radical, a nitro function, a methoxy group or an optionally substituted amine function or a residue derived from lysine, glycine or aspartic acid, a dipeptide or tripeptide residue resulting from a combination of amino acids or a residue derived from glucose, from galactose or from mannose or alternatively from glucuronic acid, or else, taken together, form a piperidino, morpholino, pyrrolidino or piperazino radical, optionally substituted in position 4 with a C₁-C₆ alkyl or polyhydroxyalkyl radical,
Y represents C(R₉)₂, O, S, Nr', CHOH, CO, SO or SO₂,
Z₂ represents O, S or NR',
Z₃ represents O or S,
n, which may be identical or different, is equal to 0 or 1; p, which may be identical or different, is equal to 0, 1, 2 or 3; t is equal to 0, 1, 2 or 3; q is an integer between 0 and 10, as well as the salts thereof and the optical and geometrical isomers thereof.

2. Compounds according to Claim 1, characterized in that they are in the form of salts of an alkali metal or alkaline-earth metal, of zinc, of an organic amine or of an inorganic or organic acid.

3. Compounds according to either of Claims 1 and 2, characterized in that the alkyl radicals are chosen from the group consisting of the methyl, ethyl, isopropyl, butyl, tert-butyl, hexyl, nonyl and dodecyl radicals.

4. Compounds according to one of the preceding claims, characterized in that polyhydroxyalkyl radicals are chosen from the group consisting of the 2,3-dihydroxypropyl, 2,3,4-trihydroxybutyl and 2,3,4,5-tetrahydroxypentyl radicals or the pentaerythritol residue.

5. Compounds according to one of the preceding claims, characterized in that the alkenyl radicals are chosen from the group consisting of radicals containing from 1 to 5 carbon atoms and having one or more ethylenical unsaturations, and in particular the allyl radical.

6. Compounds according to any one of the preceding claims, characterized in that the halogen atoms are chosen from the group consisting of fluorine and chlorine.

7. Compounds according to any one of the preceding claims, characterized in that the aminoalkyl radical is chosen from the aminomethyl, 3-aminopropyl and 6-aminohexyl radicals.

8. Compounds according to any one of the preceding claims, characterized in that the alkynyl radical has from 2 to 6 carbon atoms.

9. Compounds according to any one of the preceding claims, characterized in that the cycloaliphatic radicals of C3 to C6 carbon atoms are chosen from the cyclopropyl radical and the cyclohexyl radical.

10. Compounds according to any one of the preceding claims, characterized in that the acyl radicals having from 1 to 6 carbon atoms are chosen from the acetyl, propionyl and pivaloyl radicals.

11. Compounds according to any one of the preceding claims, characterized in that the cyclic or polycyclic alkane radicals containing from 1 to 10 carbon atoms, optionally substituted with one or more halogen atoms or one or more cyclic or polycyclic hydroxyalkane radicals containing from 1 to 10 carbon atoms, optionally substituted with one or more halogen atoms or one or more hydroxyl radicals, are chosen from adamantyl and 1-methylcyclohexyl radicals.

12. Compounds according to any one of the preceding claims, characterized in that the polyether radicals are chosen from the methoxymethyl ether, methoxyethoxymethyl ether and methylthiomethyl ether radicals.

13. Compounds according to Claim 1, characterized in that they are taken, alone or as mixtures, from the group consisting of:
- 3-[3-(1-adamantyl)-4-methoxyphenyl]-3-methyl-2H-1-benzofuran-6-carboxylic acid,
- methyl 3-[3-(1-adamantyl)-4-methoxyphenyl]-3-methyl-2H-1-benzofuran-6-carboxylate,
- 3-[3-(1-adamantyl)-4-methoxyphenyl)]-3-methyl-2H-1-benzofuran-5-carboxylic acid,
- methyl 3-[3-(1-adamantyl)-4-methoxyphenyl)]-3-methyl-2H-1-benzofuran-5-carboxylate,
- 3-methyl-3-(5,6,7,8-tetrahydro-5,5,8,8-tetramethyl-2-naphthyl)-2H-1-benzofuran-6-carboxylic acid,
- methyl 3-methyl-3-(5,6,7,8-tetrahydro-5,5,8,8-tetramethyl-2-naphthyl)-2H-1-benzofuran-6-carboxylate,
- 3-(propen-2-yl)-3-(5,6,7,8-tetrahydro-5,5,8,8-tetramethyl-2-naphthyl)-2H-1-benzofuran-6-carboxylic acid,
- methyl 3-(propen-2-yl)-3-(5,6,7,8-tetrahydro-5,5,8,8-tetramethyl-2-naphthyl)-2H-1-benzofuran-6-carboxylate,
- 3-(propen-2-yl)-3-(5,6,7,8-tetrahydro-5,5,8,8-tetramethyl-2-naphthyl)-2H-1-benzofuran-5-carboxylic acid,
- methyl 3-(propen-2-yl)-3-(5,6,7,8-tetrahydro-5,5,8,8-tetramethyl-2-naphthyl)-2H-1-benzofuran-5-carboxylate,
- 3-methyl-3-(5,6,7,8-tetrahydro-5,5,8,8-tetramethyl-2-naphthyl)-2H-1-benzofuran-5-carboxylic acid,
- methyl 3-methyl-3-(5,6,7,8-tetrahydro-5,5,8,8-tetramethyl-2-naphthyl)-2H-1-benzofuran-5-carboxylate,
- 3-methyl-3-(1,2,3,4-tetrahydro-1,4a,9b-trimethyl-1,4-methanodibenzofur-8-yl)-2H-1-benzofuran-6-carboxylic acid,
- methyl 3-methyl-3-(1,2,3,4-tetrahydro-1,4a,9b-trimethyl-1,4-methanodibenzofur-8-yl)-2H-1-benzofuran-6-carboxylate,
- 3-methyl-3-(1,2,3,4-tetrahydro-1,4a,9b-trimethyl-1,4-methanodibenzofur-8-yl)-2H-1-benzofuran-5-carboxylic acid,
- methyl 3-methyl-3-(1,2,3,4-tetrahydro-1,4a, 9b-trimethyl-1,4-methanodibenzofur-8-yl) -2H-1-benzofuran-5-carboxylate,
- 3-allyl-3-(5,6,7,8-tetrahydro-5,5,8,8-tetramethyl-2-naphthyl)-2H-1-benzofuran-6-carboxylic acid,
- methyl 3-allyl-3-(5,6,7,8-tetrahydro-5,5,8,8-tetramethyl-2-naphthyl)-2H-1-benzofuran-6-carboxylate,
- [3-(5,6,7,8-tetrahydro-5,5,8,8-tetramethyl-2-naphthyl)-3-methyl-2*H*-1-benzofur-5-oyl]morpholine,
- N-4-hydroxyphenyl-3-(5,6,7,8-tetrahydro-5,5,8,8-tetramethyl-2-naphthyl)-3-methyl-2*H*-1-benzofuran-5-carboxamide,
- N-butyl-3-methyl-3-(5,5,8,8-tetramethyl-5,6,7,8-tetrahydro-2-naphthyl)-*2H*-1-benzofuran-5-carboxamide,
- methyl 3-[4-(1-adamantyl)-3-methoxyphenyl-3-methyl-*2H*-1-benzofuran]-6-carboxylate,
- 3-[4-(1-adamantyl)-3-methoxyphenyl-3-methyl-2H-1-benzofuran]-6-carboxylic acid,
- methyl 3-(3-methyl-5,6,7,8-tetrahydro-5,5,8,8-tetramethyl-2-naphthyl)-3-methyl-2*H*-1-benzofuran-6-carboxylate,
- 3-(3-methyl-5,6,7,8-tetrahydro-5,5,8,8-tetramethyl-2-naphthyl)-3-methyl-2H-1-benzofuran-6-carboxylic acid,
- methyl 3-(5,6,7,8-tetrahydro-5,5,8,8-tetramethyl-2-naphthyl)-3-(3,5-di-*tert*-butyl-4-hydroxybenzyl)-2*H*-1-benzofuran-6-carboxylate,
- 3-(5,6,7,8-tetrahydro-5,5,8,8-tetramethyl-2-naphthyl)-3-(3,5-di-*tert*-butyl-4-hydroxybenzyl)-*2H*-1-benzofuran-6-carboxylic acid,
- 3-(5,6,7,8-tetrahydro-5,5,8,8-tetramethyl-2-naphthyl)-3-methyl-2*H*-1-benzofuran-5-methanol,
- 3-(5,6,7,8-tetrahydro-5,5,8,8-tetramethyl-2-naphthyl) -3-methyl-2*H*-1-benzofuran-5-carbaldehyde,
- methyl (-)-3-(5,6,7,8-tetrahydro-5,5,8,8-tetramethyl-2-naphthyl) -3-methyl-2*H*-1-benzofuran-5-carboxylate,
- methyl (+)-3-(5,6,7,8-tetrahydro-5,5,8,8-tetramethyl-2-naphthyl)-3-methyl-2*H*-1-benzofuran-5-carboxylate,
- methyl (-)-3-(5,6,7,8-tetrahydro-5,5,8,8-tetramethyl-2-naphthyl) -3-methyl-2*H*-1-benzofuran-6-carboxylate,
- methyl (+) -3-(5,6,7,8-tetrahydro-5,5,8,8-tetramethyl-2-naphthyl)-3-methyl-2H-1-benzofuran-6-carboxylate,
- methyl 3-(5,6,7,8-tetrahydro-5,5,8,8-tetramethyl-2-naphthyl)-3-(2-hexenyl)-*2H*-1-benzofuran-6-carboxylate,
- 3-(5,6,7,8-tetrahydro-5,5,8,8-tetramethyl-2-naphthyl)-3-(2-hexenyl)-2H-1-benzofuran-6-carboxylic acid,
- 3-(5,6,7,8-tetrahydro-5,5,8,8-tetramethyl-2-naphthyl)-3-hexyl-2*H*-1-benzofuran-6-carboxylic acid,
- methyl 3-methoxycarbonylmethyl-3- (5,5,8,8-tetramethyl-5,6,7,8-tetrahydro-2-naphthyl)-2*H*-1-benzofuran-6-carboxylate,
- 3-carboxymethyl-3-(5,5,8,8-tetramethyl-5,6,7,8-tetrahydro-2-naphthyl)-2H-1-benzofuran-6-carboxylic acid.

14. Compounds according to Claim 1, characterized in that they have at least one of the following characteristics:
- R₁ is a radical -(CH₂)ₚ-CO-O-R₁₃
- R₂ is a hydrogen
- R₃ is a hydrogen or an alkenyl radical
- R₅ or R₆ is a radical -OR₁₁
- R₇ is a cyclic or polycyclic alkane radical containing from 1 to 10 carbon atoms, optionally substituted with one or more halogen atoms or one or more cyclic or polycyclic hydroxyalkane radicals containing from 1 to 10 carbon atoms, optionally substituted with one or more halogen atoms or one or more hydroxyl radicals,
- Z₁ is an oxygen atom
- Y is a radical C(R₉)₂
- m is equal to 1.

15. Compounds according to any one of the preceding claims, for use as medication.

16. Use of a compound according to any one of Claims 1 to 14, in the manufacture of a medicinal product intended for the treatment:
- of dermatological complaints associated with a keratinization disorder which has a bearing on differentiation and on proliferation, in particular for treating common acne, comedones, polymorphonuclear leukocytes, acne rosacea, nodulocystic acne, acne conglobata, senile acne and secondary acnes such as solar acne, medication-induced acne or occupational acne,
- of ichthyosis, ichthyosiform states, Darrier's disease, palmoplantar keratoderma, leucoplasias and leucoplasiform states, and cutaneous or mucous (buccal) lichen,
- of dermatological complaints associated with a keratinization disorder having an inflammatory and/or immunoallergic component and, in particular, all forms of psoriasis, whether it is cutaneous, mucous or ungual psoriasis, and even psoriatic rheumatism, or alternatively cutaneous atopy, such as eczema, or respiratory atopy or alternatively gingival hypertrophy; of inflammatory complaints which do not exhibit a keratinization disorder,
- of dermal or epidermal proliferations, whether benign or malignant and whether or not they are of viral origin, such as common warts, flat warts and verruciform epidermodysplasia, oral or florid papillomatoses and proliferations which may be induced by ultraviolet radiation, in particular in the case of basocellular and spinocellular epithelioma,
- of dermatological disorders such as bullosis and collagen diseases,
- of ophthalmological disorders, in particular corneopathies,
- of light-induced and chronological ageing of the skin or of actinic keratoses and pigmentations, or any pathology associated with chronological or
- actinic ageing,
- of the stigmata of epidermal and/or dermal atrophy induced by local or systemic corticosteroids, or any other form of skin atrophy,
- of cicatrization disorders or stretchmarks,
- of disorders of sebaceous functioning such as the hyperseborrhoea of acne or simple seborrhoea,
- of cancerous or precancerous states, more particularly promyelocytic leukaemia;
- of inflammatory complaints such as arthritis,
- of any complaint of viral origin on the skin or generally,
- of alopecia,
- of dermatological complaints having an immunological component,
- of complaints of the cardiovascular system such as arteriosclerosis or hypertension and non-insulin-dependent diabetes,
- of skin disorders due to exposure to UV radiation.

17. Pharmaceutical composition, characterized in that it comprises, in a pharmaceutically acceptable support, at least one of the compounds as defined in any one of Claims 1 to 14.

18. Composition according to Claim 17, characterized in that the concentration of compound(s) according to one of Claims 1 to 14 is between 0.001% and 5% by weight relative to the composition as a whole.

19. Cosmetic composition, characterized in that it comprises, in a cosmetically acceptable support, at least one of the compounds as defined in any one of Claims 1 to 14.

20. Composition according to Claim 19, characterized in that the concentration of compound(s) according to one of Claims 1 to 14 is between 0.001% and 3% by weight relative to the composition as a whole.

21. Use of a cosmetic composition as defined in either of Claims 19 and 20, for body or hair hygiene.
